# EUROPEAN PATENT APPLICATION

(11) **EP 2 985 294 A1**
(43) Date of publication of application: **17.02.2016**
(21) Application number: 14181060.6
(22) Date of filing: 14.08.2014
(51) Int. Cl.: C07K 16/30, C07K 16/28, C07K 16/46

(54) **Recombinant antibody molecule and its use for target cell restricted T cell activation**

(71) Applicant: Deutsches Krebsforschungszentrum, 69120 Heidelberg (DE); Julius-Maximilians-Universität Würzburg, 97070 Würzburg (DE); Eberhard Karls Universität Tübingen, 72074 Tübingen (DE)
(72) Inventor: Jung, Gundram, 72108 Rottenburg (DE); Salih, Helmut R., 72072 Tübingen (DE); Stuhler, Gernot, 72070 Tübingen (DE); Grosse-Hovest, Ludger, 72070 Tübingen (DE); Lochmann, Berit, 72072 Tübingen (DE)
(74) Representative: Schiweck, Weinzierl & Koch

(57) **Abstract**

The present disclosure relates to a recombinant antibody molecule, bispecific as well as tri-specific hetero-dimeric antibody molecules, as well as a method for producing the same, its use and a nucleic acid molecule encoding the recombinant antibody molecules. The disclosure in particular provides an antibody molecule that is capable of mediating target cell restricted activation of immune cells.

## Description

### FIELD OF THE INVENTION

The present invention relates to new recombinant non aggregating and "combinatorial" antibody molecules, bispecific as well as tri-specific heterodimeric antibody molecules, as well as a method for producing the same, uses thereof and nucleic acid molecules encoding the recombinant antibody molecules. The invention in particular provides an antibody molecule that is capable of mediating target cell restricted activation of immune cells.

### BACKGROUND

Scientific work starting in the mid eighties has established that bispecific antibodies directed to a tumor associated antigen (TAA) and the T cell receptor (TCR)/CD3-complex are capable of activating T cells and mediate the lysis of TAA expressing tumor cells by the activated T cells [1-3]. Since CD3-antibodies, bound to Fc receptors (FcRs) via their Fc-part, are exceedingly efficient in inducing T cell activation and cytokine release, it is of importance to construct Fc-depleted or -attenuated bispecific CD3-antibodies in order to prevent FcR binding and to allow for a target cell restricted- rather than FcR-mediated activation of T cells [4,5].

The production of bispecific antibodies meeting this critical prerequisite in industrial quality and quantity remains a formidable challenge. Recently, a recombinant, bispecific single chain (bssc) antibody with CD19 X CD3-specificity, termed Blinatumomab, has demonstrated considerable efficiency in the treatment of patients with ALL [6] and is currently tested in phase III trials. Notably, the drug is applied as continuous 24hr infusion due to its low serum halflife and safely applicable doses are approx. 50 µg per patient and day and thus 10.000 times lower than that of established monospecific antitumor antibodies. The resulting serum concentrations of the drug are below 1 ng/ml [7]. This dose limitation, also observed in earlier clinical trials [8,9], is due to off-target T cell activation resulting in systemic cytokine release. Obviously, this phenomenon prevents an optimal therapeutic activity of bispecific antibodies stimulating the TCR/CD3 complex.

In principle, dose limiting off-target T cell activation and the resulting toxicity problem may be caused by the following phenomena (P1-P3):

(P1) The TAA targeted by the bispecific antibody is not entirely tumor specific resulting in antibody mediated T cell activation by normal, tumor associated antigen expressing cells.

(P2) It is widely held that T cell activation requires a multivalent CD3 stimulus formed after binding of a bispecific TAA X CD3 antibody to TAA expressed by a target cell, e.g. a tumor cell. A monovalent stimulus, as provided by most current bispecific molecules in solution, does not activate T cells. This is the basis of the concept of target cell restricted T cell activation with bispecific antibodies as outlined above in pargraph [002]. However more recent data from our laboratory suggest that a monovalent TCR/CD3 stimulus in the absence of target cells may lead to some T cell activation and even target cell killing. This type of target cell independent T cell activation by the CD3 part of a bispecific antibody is exaggerated in the presence of endothelial cells. The latter finding is in line with a publication of Molema et al. demonstrating increased endothelial cell interaction by PBMC coated with bispecific TAA X CD3 antibodies in the Fab₂-format [10]. From these data it can be concluded that this type of of-target T cell activation contributes significantly to the dosing problem encounterd by bispecific antibodies stimulating the TCR/CD3 complex.

(P3) Single chain antibodies that are often used as recombinant bispecific antibody format have the tendency to aggregate. Thus, even small amounts of aggregated CD3 antibody may lead to T cell activation in the absence of the tumor associated antigen.

A first approach to address these problems is described in International Patent Application WO 2013/104804. This patent application discloses a set of of two polypeptides each of which comprises a targeting moiety "T" binding to an antigen "A" and a fragment of "F" of a functional domain, wherein said two polypeptides are not associated with each other in absence of a substrate that has "A" at (on) its surface and wherein, upon dimerization of "F", the resulting dimer becomes functional. In the concrete embodiments described in WO 2013/104804, the targeting moiety "T" is a single chain Fv fragment that binds, for example, to a tumor associated antigen and the fragment "F" of the functional domain is a variable domain of the light chain or the heavy chain of an antibody molecule such as a CD3 binding antibody. These molecules comprising a single chain Fv fragment and an unpaired variable domain (of either the light or the heavy chain of an antibody, here referred to as "triple domain single chain (tdsc)-molecules". However, also this approach makes essentially use of the single chain antibody format.

Generally acknowledged shortcomings of single chain antibodies in general, and thus also of tdsc-molecules, are (1) their low molecular weight resulting in a low serum half life (2) low production rates during fermentation, (3) loss of affinity as compared to parental antibodies in a physiological format and (4) a general tendency to form multimers or aggregates. While the first three shortcomings are technical in nature even tiny amounts of aggregated or multimeric material may lead to off target T cell activation and increased toxicity if TCR/CD3 antibodies are used (P3 in the list above). Taken together, these phenomena have considerably hampered the development of bispecific antibodies. The aim of this invention is to improve these critical properties for bifunctional combinatorial antibodies.

It is therefore an object of the present invention to provide a recombinant antibody molecule that overcomes at least some of the above difficulties and that can be generally used in therapy, amongst others for strictly target cell restricted activation of immune cells as described above.

### SUMMARY OF THE INVENTION

In a first aspect the present invention provides a recombinant antibody molecule (which is also referred herein as "Fabv"). The recombinant antibody molecule consists of a Fab fragment comprising a first binding site for a first antigen, a variable domain of either the light chain or the heavy chain of a second binding site for a second antigen and an immunoglobulin CH2 domain, wherein the Fab fragment and the variable domain are linked via the CH2 domain, wherein in the immunoglobulin CH2 domain at least one cysteine residue that is able to form a disulfide bridge for dimerisation is lacking or mutated.

In a second aspect the invention provides a bispecific hetero-dimeric antibody molecule. This bispecific hetero-dimeric antibody molecule consists of recombinant antibody molecules (monomers),
wherein the first monomer of the hetero-dimer consists of a Fab fragment comprising a first binding site for a first antigen, a variable domain of the light chain of a second binding site for a second antigen and an immunoglobulin CH2 domain, wherein the Fab fragment and the variable domain of the light chain are linked via the CH2 domain, and
wherein the second monomer of the hetero-dimer consists of a Fab fragment comprising a first binding site for the first antigen, a variable domain of the heavy chain of the second binding site for the second antigen and an immunoglobulin CH2 domain, wherein the Fab fragment and the variable domain of the heavy chain are linked via the CH2 domain,
wherein the variable domain of the light chain of the second binding site of the first monomer and the variable domain of the heavy chain of the second binding site of the second monomer associate thereby forming, the second binding site and dimerizing the hetero-dimer, and
wherein in at least one of the immunoglobulin CH2 domain of the first monomer or the second monomer at least one cysteine residue that is able to form a disulfide bridge for dimerisation is lacking or mutated, thereby preventing that a CH2 domain mediated disulfide bridge is formed between the two monomers.

In a third aspect the invention provides a tri-specific hetero-dimeric antibody molecule comprising a hetero-dimer of recombinant antibody molecules (monomers, wherein the first monomer of the hetero-dimer consists of a Fab fragment comprising a first binding site for a first antigen, a variable domain of the light chain of a second binding site for a second antigen and an immunoglobulin CH2 domain, wherein the Fab fragment and the variable domain of the light chain are linked via the CH2 domain, and wherein the second monomer of the hetero-dimer consists of a Fab fragment comprising a third binding site for a third antigen, wherein the third antigen is different from the first antigen, a variable domain of the heavy chain of the second binding site for the second antigen and an immunoglobulin CH2 domain, wherein the Fab fragment and the variable domain of the heavy chain are linked via the CH2 domain,
wherein the variable domain of the light chain of the second binding site of the first monomer and the variable domain of the heavy chain of the second binding site of the second monomer associate thereby forming, the second binding site and dimerizing the hetero-dimer, and
wherein in at least one of the immunoglobulin CH2 domain of the first monomer or the second monomer at least one cysteine residue that is able to form a disulfide bridge for dimerisation is lacking or mutated, thereby preventing that a CH2 domain mediated disulfide bridge is formed between the two monomers.

In an fourth aspect the invention provides a pharmaceutical composition comprising an antibody molecule of the present invention.

In a fifth aspect the invention provides an antibody molecule of the present invention for use in the treatment or diagnosis of a disease.

In a sixth aspect the invention provides the use of an antibody molecule of the present invention for the treatment of a disease, wherein the antibody molecule forms a hetero-dimer only in vivo on a target cell, thereby reducing "off target activation".

In a seventh aspect the invention provides a nucleic acid molecule encoding an antibody molecule of the present invention.

In a eigth aspect the invention provides a nucleic acid molecule of the present invention comprised in a vector.

In a ninth aspect the invention provides host cell comprising the nucleic acid molecule or the vector of the present invention.

In a tenth aspect the invention provides for a method of producing an antibody molecule of the present invention, comprising expressing a nucleic acid encoding the antibody molecule under conditions allowing expression of the nucleic acid.

These aspects of the invention will be more fully understood in view of the following description, drawings and non-limiting examples.

### BRIEF DESCRIPTION OF THE DRAWINGS

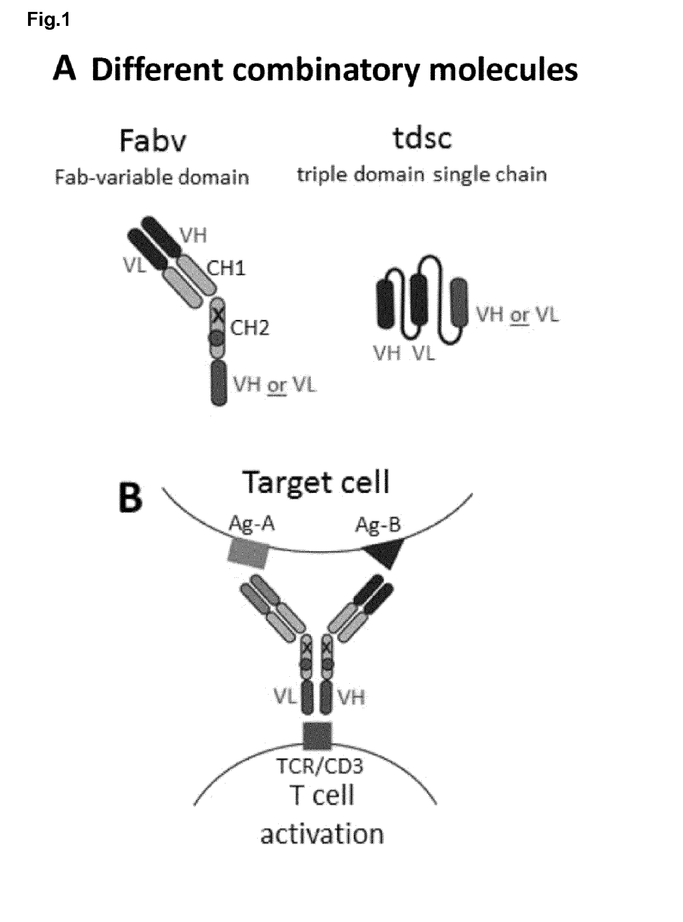

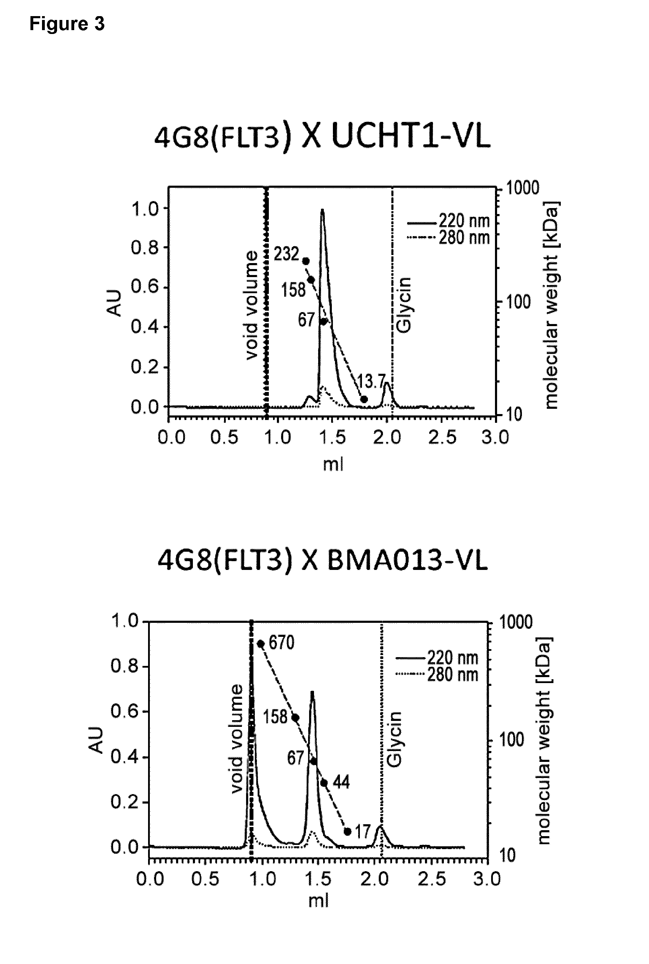

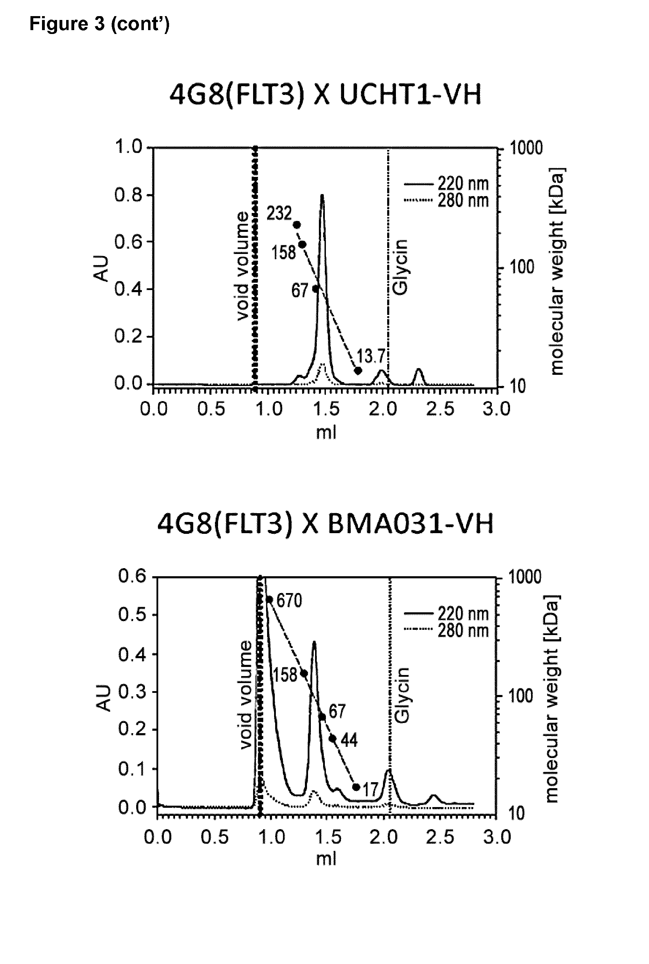

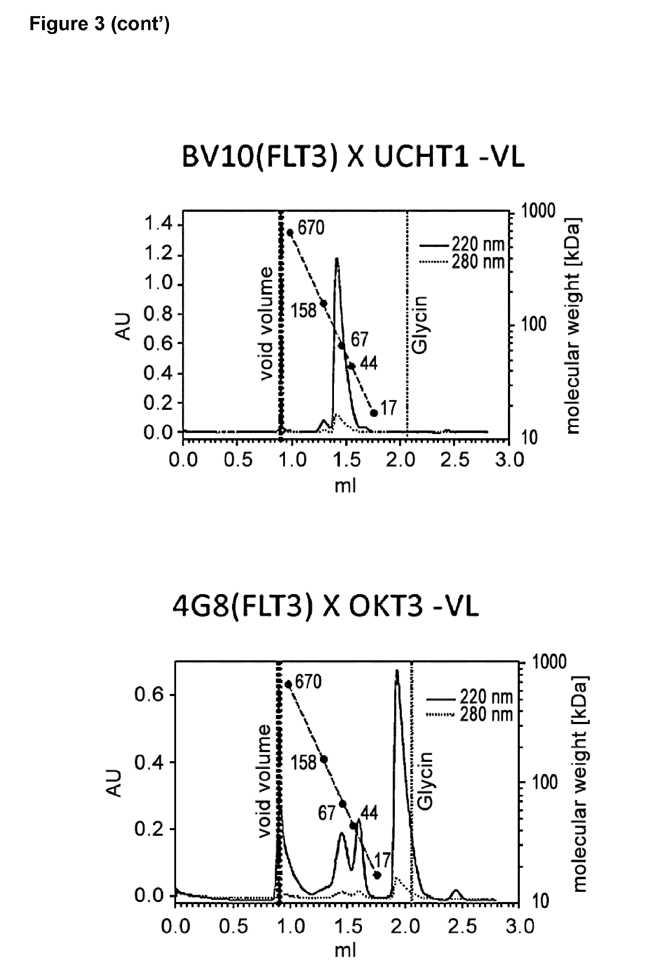

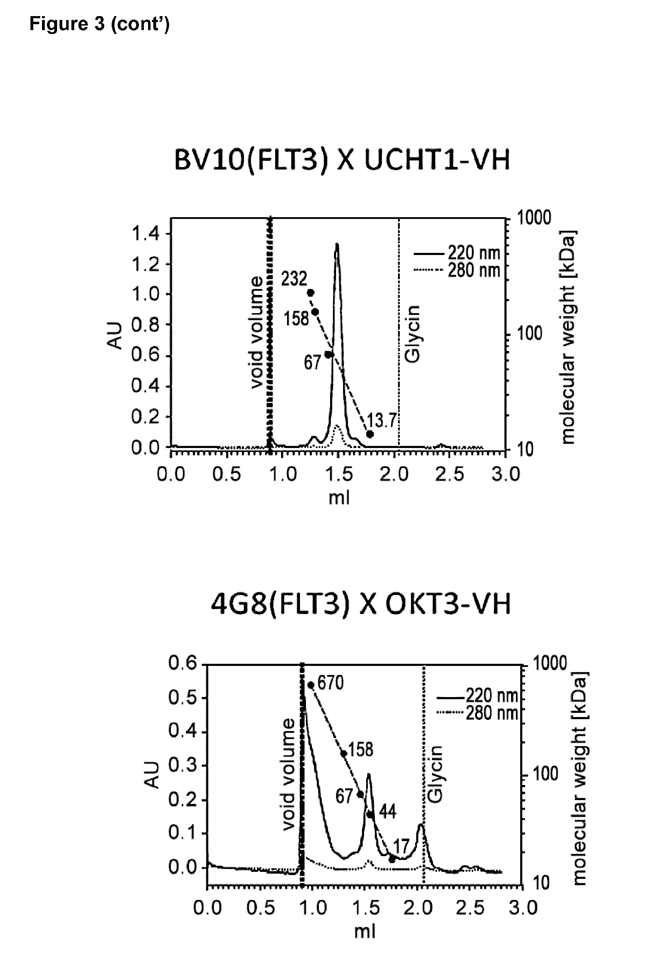

**Fig. 1A****: Different types of combinatorial Fabv-molecules.** The CH2-domain of the Fabv-antibody molecule of the present invention is modified to prevent homodimerization and may further be modified to prevent binding to Fc-receptors. To prevent binding to Fc receptors and homodimerization via disulfide bonds several amino acid modifications can be introduced into this domain (o, x). The other "one and a half molecule" depicted in Fig. 1, is the known so-called "tdsc" that is described in International Patent Application WO 2013/104804 (supra). In the "tdsc"-molecule a single chain Fv fragment forms a first binding site that binds to a first antigen, and the single chain Fv fragment is linked to an unpaired variable domain (either the VH or the VL domain) of a second antibody binding site that binds a second antigen.
**Fig. 1B****: The principle of a target cell restricted T cell activation realized with combinatorial Fabv antibodies.** In the illustrative embodiment of Fig. 1 B one antibody molecule directed to Antigen A (Ag-A) on the target cells carries the VH-domain of an anti CD3-antibody, a second antibody molecule directed to Antigen B (Ag-B) carries the corresponding VL-domain of the CD3 binding antibody. Preferably, and both (Ag-A and Ag-B) are TAAs. Furthermore, when Ag-A is the same as Ag-B, then these antigens can be targeted with a bispecific hetero-dimeric antibody molecule of the invention. If Ag-A and Ag-B are different from each other, they can be targeted with a tri-specific hetero-dimeric antibody molecule of the invention. If both antibody molecules bind to the target cell a functional VH-VL binding site is formed by association of the unpaired VL-domain with the unpaired VH-domain ("on-site complementation") that binds to CD3, thereby activating T cells.
   A preferred CD3 antibody for this approach is UCHT1 because the tendency of the VH/VL-regions of this antibody to form aggregates when present in the Fabv-format of the invention is minimal (Fig. 3). Moreover, judging from the binding behavior (the affinity binding) of the single chain Fv fragment of the antibody UCHT-1, it is deduced that the affinity of the UCHT1 within the Fabv^{ko}-format is almost completely preserved as compared to that of the parental, bivalent UCHT1 antibody (data now shown).

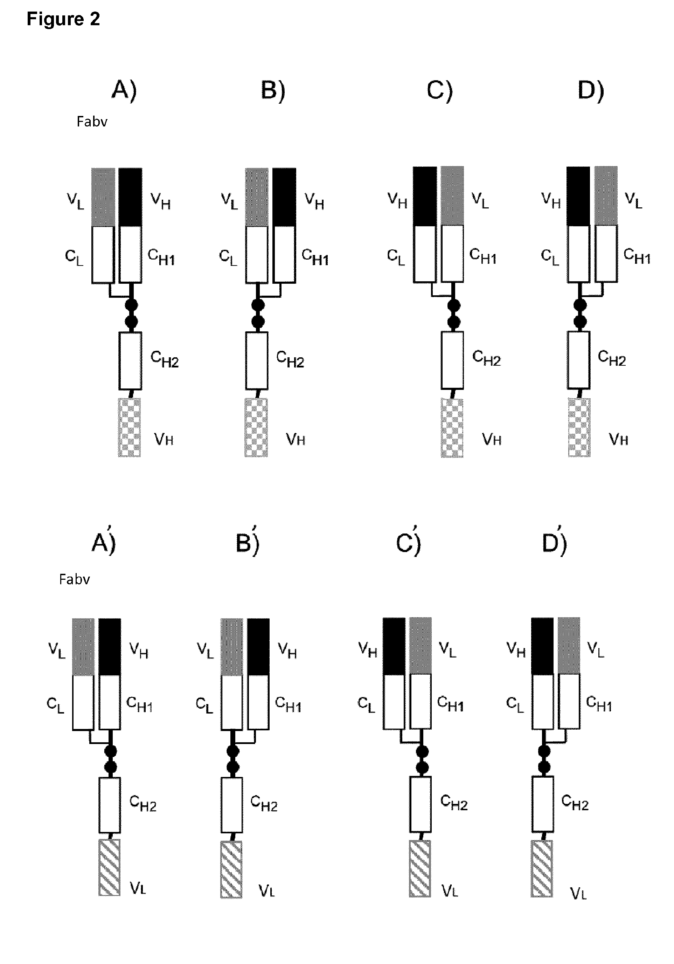

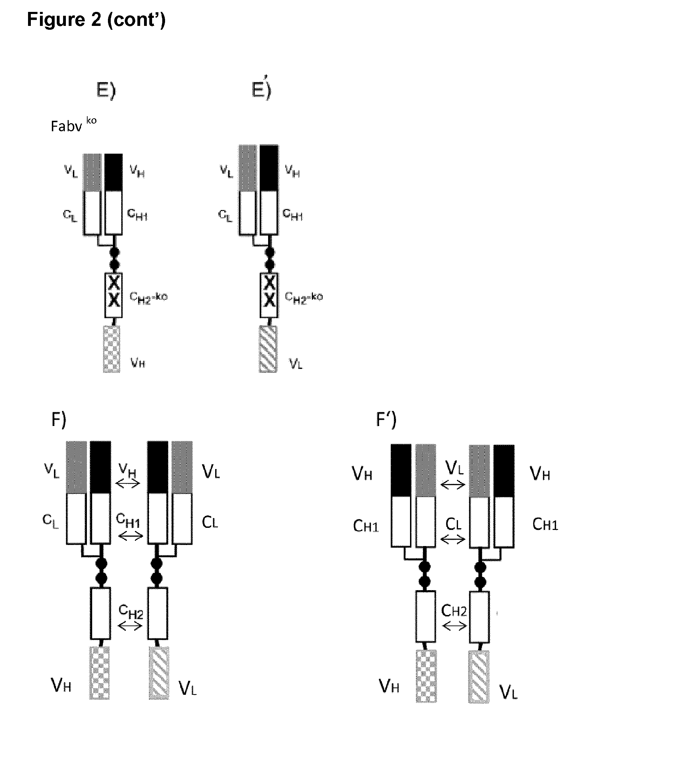

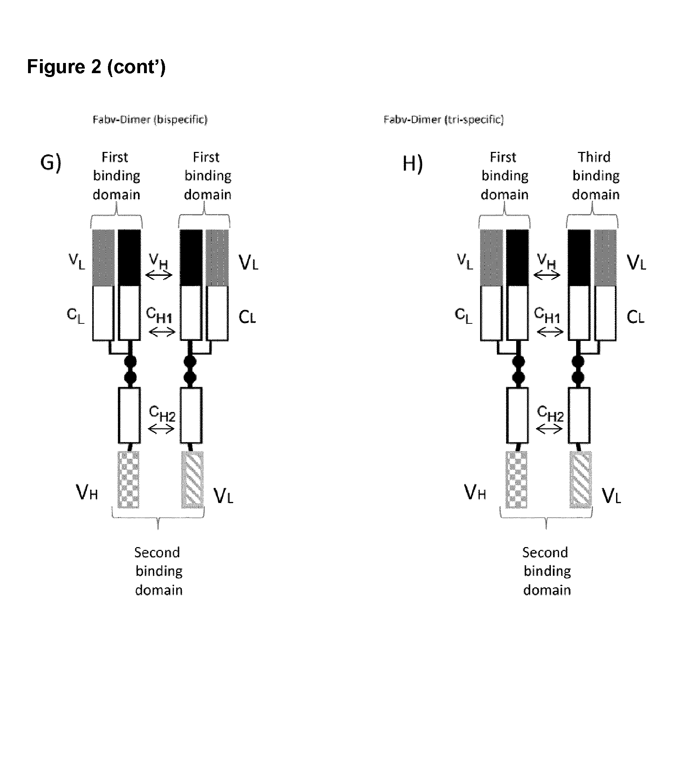

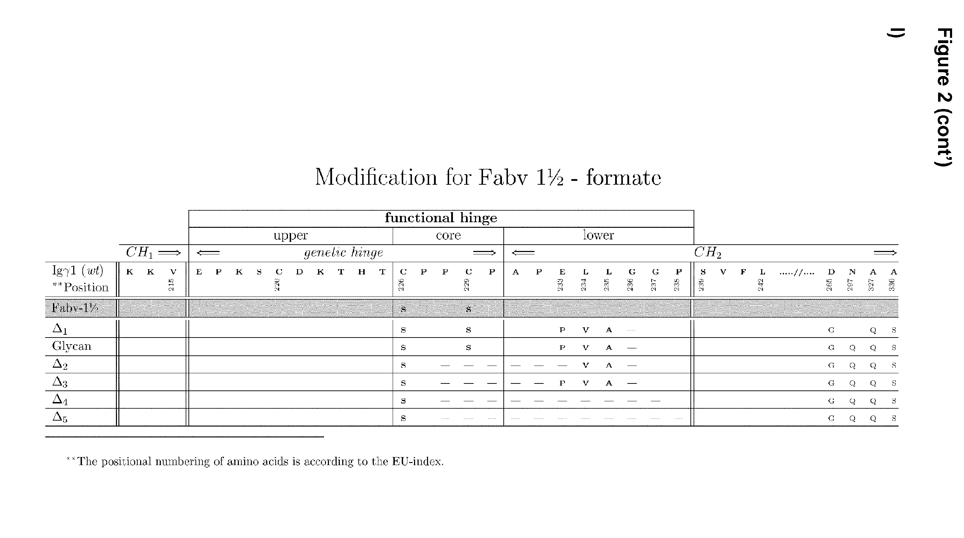

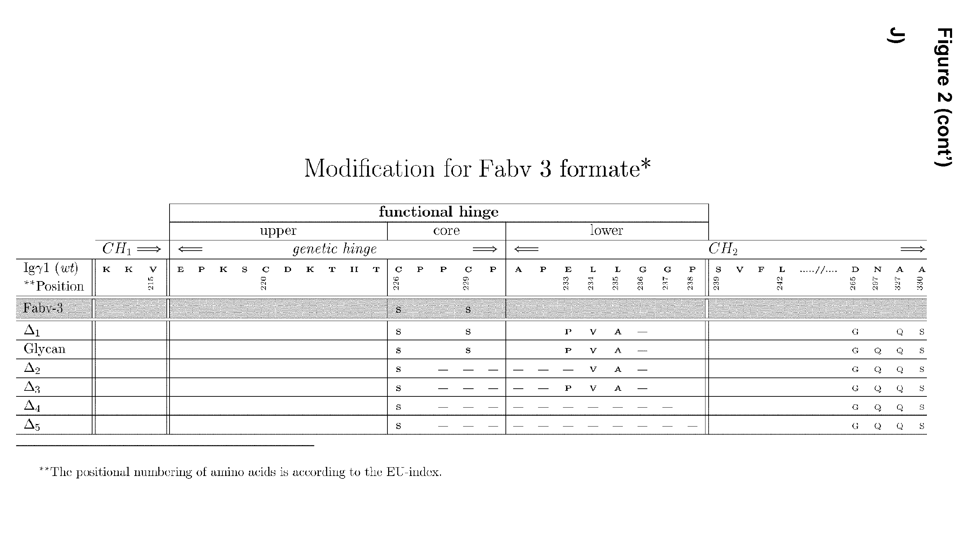

**Fig. 2** schematically depicts embodiments of antibody molecules according to the invention.
**Fig. 2A** depicts a recombinant antibody molecule with a Fab fragment, a CH2 domain and a single VH domain. The antibody molecule has a main chain in which the CH2 domain is coupled via its N-terminus to the heavy chain CH1 and VH domains of a Fab fragment and via its C-terminus to a single VH domain (A) or a single VL domain (A') (Fabv format).
**Fig. 2B** depicts a recombinant antibody molecule with a main chain in which the CH2 domain is linked to the light chain of a Fab fragment, i.e. in which the main chain includes a VL and a CL domain, a hinge region, a CH2 domain and a single VH domain (B) or a single VL domain (B').
**Fig. 2C** shows a recombinant antibody molecule in which the main chain includes a VL and a CH1 domain, a hinge region, a CH2 domain and a single VH domain (C) or a single VL domain (C'). A second chain of lower weight includes a VH and a CL domain. In the antibody molecule of Fig. 2C or 2C' the Fab fragment is thus not a "classical (naturally occurring)" Fab fragment in which the variable domain of the light and the heavy chain are fused to its respective constant domain (CL or CH1, respectively) but a "hybrid" Fab fragment in which the variable domain is fused to the constant domain of the "opposite chain, i.e. the VH domain is fused to the CL domain and the VL domain is fused to the CH1 domain.
**Fig. 2D** depicts a recombinant antibody molecule with a main chain in which the CH2 domain is linked to a CL and a VH domain. A second chain of lower weight includes a VL and a CH1 domain. The antibody molecule of Fig. 1 D or 1 D' thus includes a "hybrid Fab fragment" (that includes the first binding site) as it is also present in the molecule of Fig.1C or 1C'.
**Fig. 2E** and **E'** depict a recombinant antibody molecule with a build-up as in Fig. 2A or 2A', in which amino acids in the CH2 domain and/or the hinge region that have been modified are indicated (indicated by "X" as depicted in Fig. 2I, Fabv^{ko}--format). Likewise, such modifications can be inserted into the molecules depicted in 2A-2D and 2A'-2D'. In the molecules depicted in Figs. 2A-2E and 2A'-2E' the cystein residues forming inter-chain disulfide bonds (C226 and C229 in human IgG-antibodies) are exchanged to prevent formation of dimers (•).
**Fig. 2F****, F'** depict illustrative embodiments a hetero-dimeric antibody molecule being a dimer of the unit depicted in Fig. 2A and 2A' and 2B and 2B'. Such a molecule may also be constructed in the Fabv-configurations depicted in Figs. 2B-2D and 2B'-2D' with and without the Fc modifications depicted in Fig. 2E and 2E'. These modifications are listed in Figure 2I.
**Fig. 2G and 2H** illustrates bispecific or tri-specific hetero-dimeric antibody molecules, which can self assemble in the Fabv-configurations depicted in Figs. 2A-2D and 2A'-2D' with and without the Fc modifications depicted in Fig. 2E and 2E'. These modifications are listed in Figure 2J. Equally, it is possbile to combine a Fabv antibody molecule with a Fabv^{ko} antibody molecule.
**Fig. 2I** lists illustrative modifications that can be introduced into the antibody molecules of the invention depicted in Figs.2A-D, Figs. 2A'-2D' to obtain Fc deficient and cysteine free derivatives as exemplified in Figs. 2E, and 2E'. Modifications are identical to those shown in Fig. 2I with the exception of the preserved cysteins (C226 and C229 in human IgG-antibodies). The numbering of amino acids is in line with the Kabat numbering [EU-Index]. *wt =* IgG1 human wild type sequence; Δ1 = knock-out; Glycan = Δ1-knock-out with deletion of saccharide moieties ≅297; Δ2-5 further knock-out variants in continuation of Δ1; - = the amino acid has been deleted.
**Fig. 2J** lists illustrative modifications that can be present in a heterodimeric antibody molecule as depicted in Fig. 2F- H, 2F'-H'-. The numbering of amino acids is in line with the Kabat numbering [EU-Index]. *wt =* IgG1 humane wild type sequence; Δ1 = knock-out; Glycan = Δ1-knock-out with deletion of saccharide moieties ≅297; Δ2-5 further knock-out variants in continuation of Δ1;-= the amino acid has been deleted.
**Fig 3** **Size exclusion chromatography of various combinatorial molecules in the Fabv-format.** Recombinant antibody molecules, constructed as depicted in Fig. 2E and 2E', were purified from the supernatant of Sp2/0 cells transfected with the respective recombinant gene constructs by affinity chromatography using a kappa-select column. After elution at pH 2.5 the recombinant antibody molecules were dialyzed against PBS and subjected to gel filtration analysis on a Superdex 200 column (PC3.2/30). 4G8 and BV10 are two FLT3 antibodies directed to domain 4 and domain 2 of the molecule, respectively. UCHT1 and OKT3 are different antibodies directed to the CD3 ∈ chain. BMA031 binds to a constant epitope of the T cell receptor (TCR). Conclusion: Whereas all antibody molecules containing the VH/VL regions of the CD3 antibody UCHT1 were largely monomeric, VH/VL domains of the OKT3 and BMA031 single chain antibody molecules caused some aggregation of the Fabv^{ko} -antibody molecules containing them.

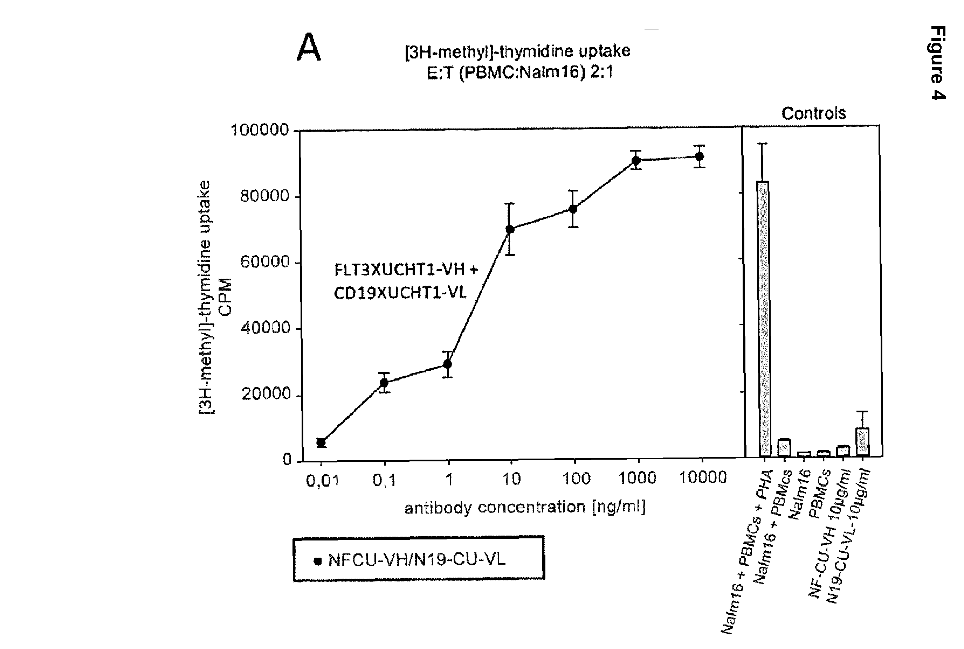

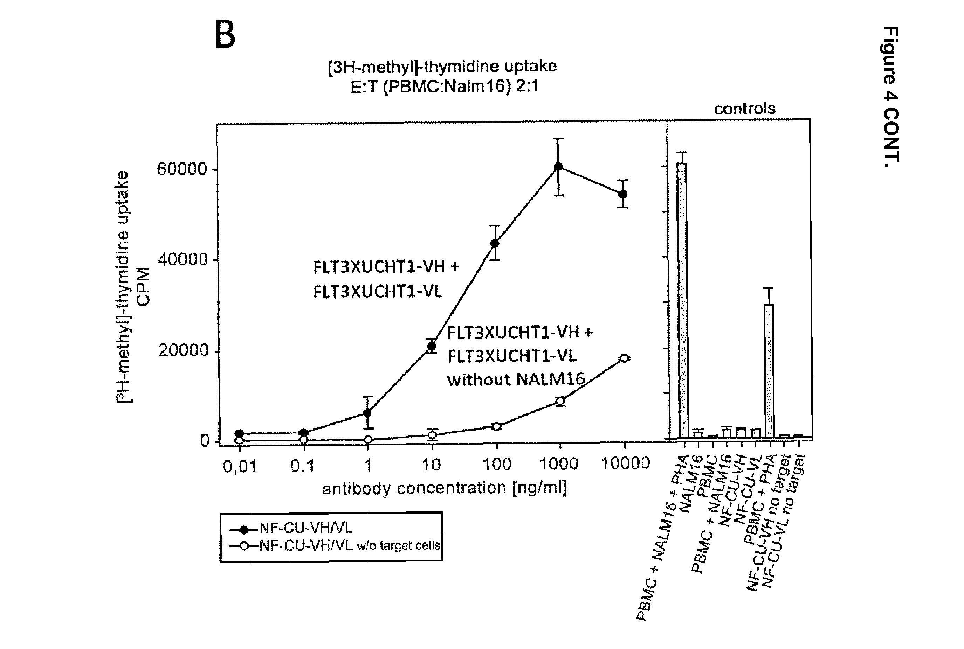

**Fig. 4** **Proliferation of T cells in the presence of FLT3+/CD19+ NALM16 cells induced by a combination of Fabv-antibodies targeting FLT3 and CD19.** PBMC expressing the CD3 antigen were incubated with NALM16 cells, expressing the FLT3- and the CD19 antigen, and the indicated antibody molecules in the format depicted in Figs 2E and 2E'. Cells were pulsed with 3H-Thymidine after 2 days. Incorporated radioactivity was measured at day 3. NFCU=FLT3XUCHT, N19CU=CD19XUCHT1. CD19-antibody=4G7, FLT3-antibody=4G8.
In Fig. 4A) in the left part of the graph FLT3XUCHT-VH was combined with CD19XUCHT1-VL. Thus, this example shows the formation of a hetero-dimeric trispecific antibody molecule targeting NALM16 cells (first and third binding site) and on the other hand PBMC cells (second binding site). As shown in the graph in 4A, the higher the antibody molecule concentration of each single antibody molecule was, the more cells incorporated thymidine. On the right hand side of the graph in A, different controls are depicted. It becomes clear that neither the single FLT3XUCHT-VH nor the single CD19XUCHT1-VL could trigger thymidine uptake in NALM16 cells. This demonstrates that a functional CD3 binding site is generated by the spontaneous formation of the two Fabv-molecules targeting FLT3 and CD19. The combination of NALM16 + PBMCs + PHA served as a positive control, while the presence of only NALM16, NALM16/PBMCs or only PBMCs served as a negative control.
In Figure 4B the FLT3xUCHT1-VH antibody molecule was utilized at the same time as the FLT3xUCHT1-VL, thus exemplifying a bispecific hetero-dimer. On the one hand, the FLT3 binding site may bind NALM16 cells (2 first binding sites), while the UCHT1 binding site may bind the PBMC cells (second binding site). Again, on the left hand side of the depicted graph, it is shown that the higher the concentration of the single antibody molecules was, the more cells incorporated thymidine. When removing only the NALM16 cells, almost no thymidine could be detected (FLT3XUCHT-VH + CD19XUCHT1-VL without NALM 16). On the right hand side of the graph in B, different controls are depicted. It becomes clear that neither the single FLT3XUCHT-VH nor the single CD19XUCHT1-VL could trigger thymidine uptake in NALM16 cells. Again this demonstrates that a functional CD3 binding site is formed by a spontaneous assembly of the two Fabv-molecules targeting FLT3. The combination of NALM16 + PBMCs + PHA or PMBC + PHA served as a positive control, while the presence of only NALM16, NF-CU-VH no target, NF-CU-VL no target or PBMCs served as a negative control, Conclusion: A combination of Fabv-antibody molecules (Fabv-3) forms a functional CD3 binding site and induces T cell activation in the presence of double positive target cells (Fig 4A) or single positive target cells (Fig. 4B). Single Fabv^{ko}-molecules comprising either the VL or the VH domain were ineffective.

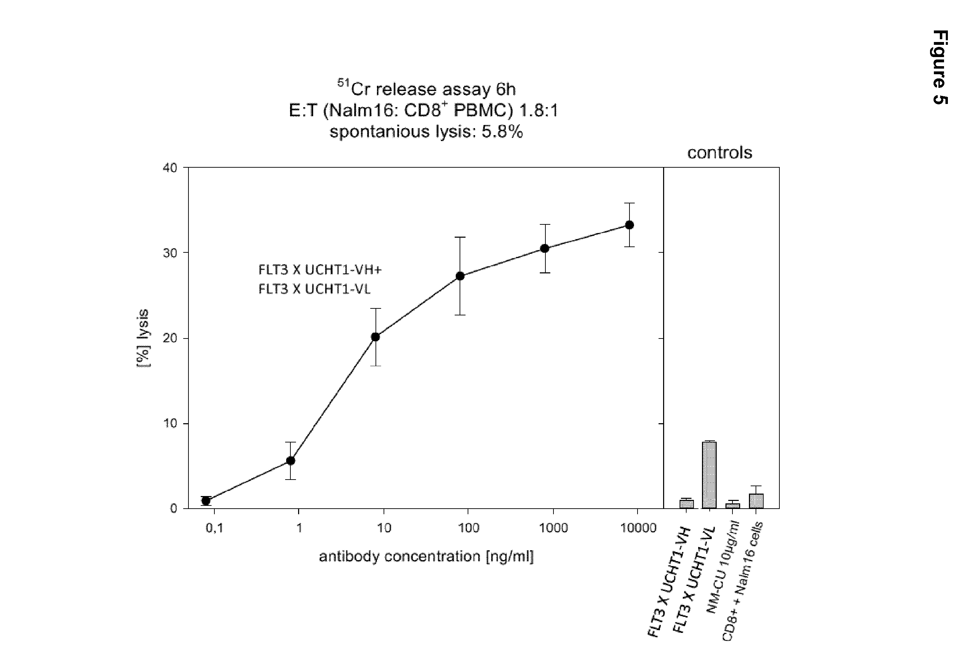

**Fig. 5** **Lysis of FLT3 expressing target cells by a combination of Fabv antibody molecules.** 51Cr-labeled Nalm16 cells were incubated with a mixture of recombinant Fabv^{ko}-antibody molecules at the indicated concentrations together with activated CD8+ cytolytic T cells purified from anti-CD3 stimulated PBMC using magnetic beads coated with CD8 antibodies. After 6 hours 51 Cr release was measured. On the right hand side of the graph, different controls are depicted. NMCU is a bispecific Fabsc control antibody, which comprises a Fab domain comprising the CSPG4 antigen specificty linked via a CH2 domain to both VH/VL chains comprising a CD3 specificity (CSPG4XCD3 (9.2.27XUCHT1)). CSPG4 is a melanoma associated antigen that is not expressed on Nalm16 cells.
   Conclusion: A combination of FLT3 targeting recombinant Fabv^{ko}-antibody molecules but not single molecules- induces lysis of FLT3 positive target cells.

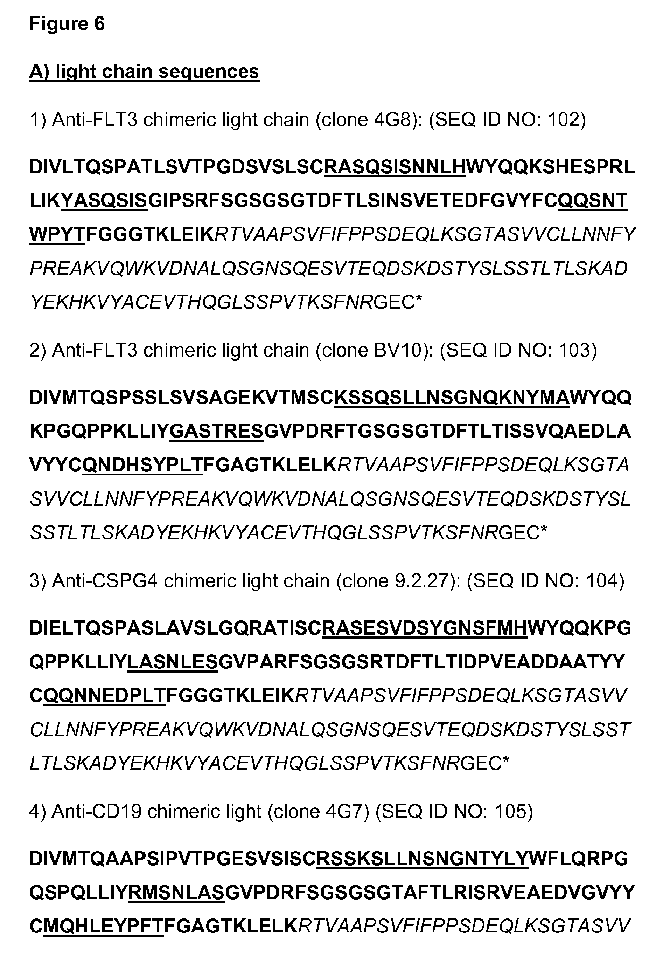

**Fig. 6A** shows the sequences of illustrative light chains that may be included in a recombinant antibody molecule of the invention. The respective peptide chains correspond to the mature protein without the corresponding leader peptide sequence. The sequences contain an N-terminal variable domain represented in bold and a C-terminal constant domain depicted in italic. The complementarity determining regions (CDRs) of the variable domain are underlined.

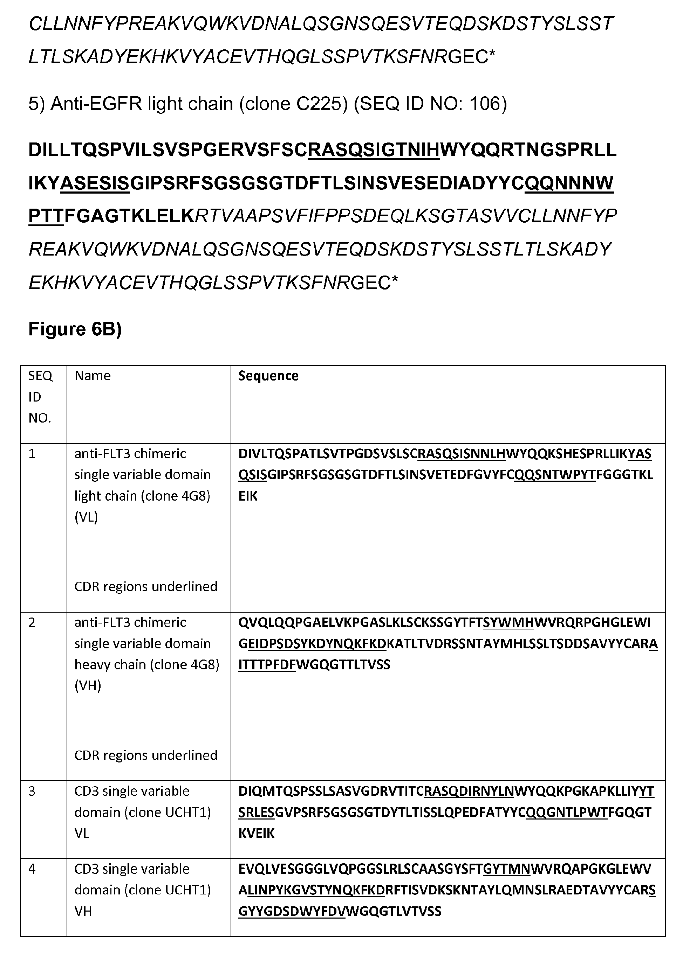

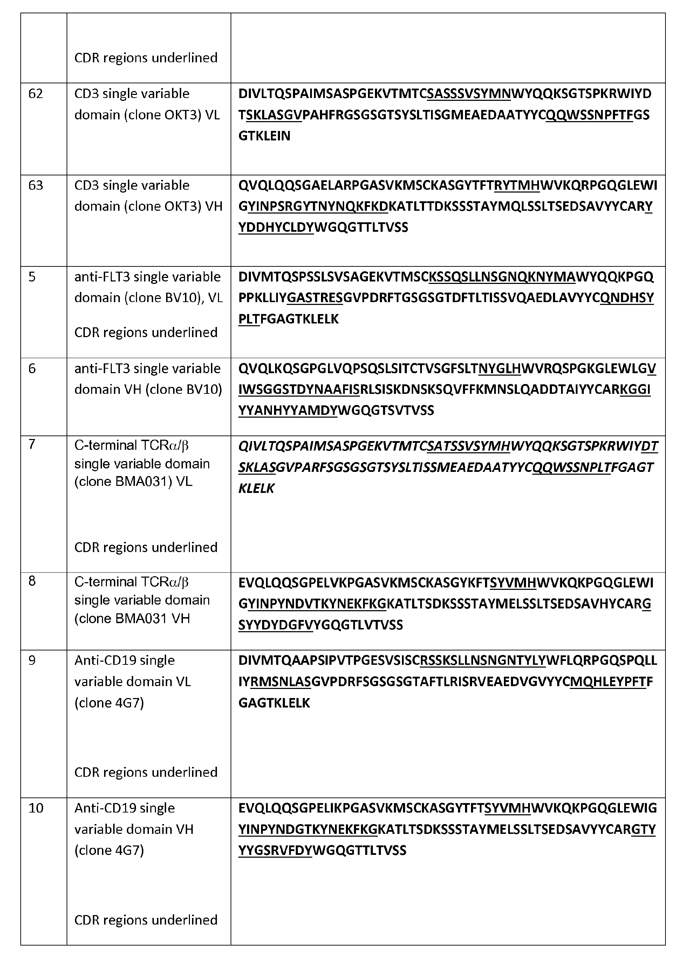

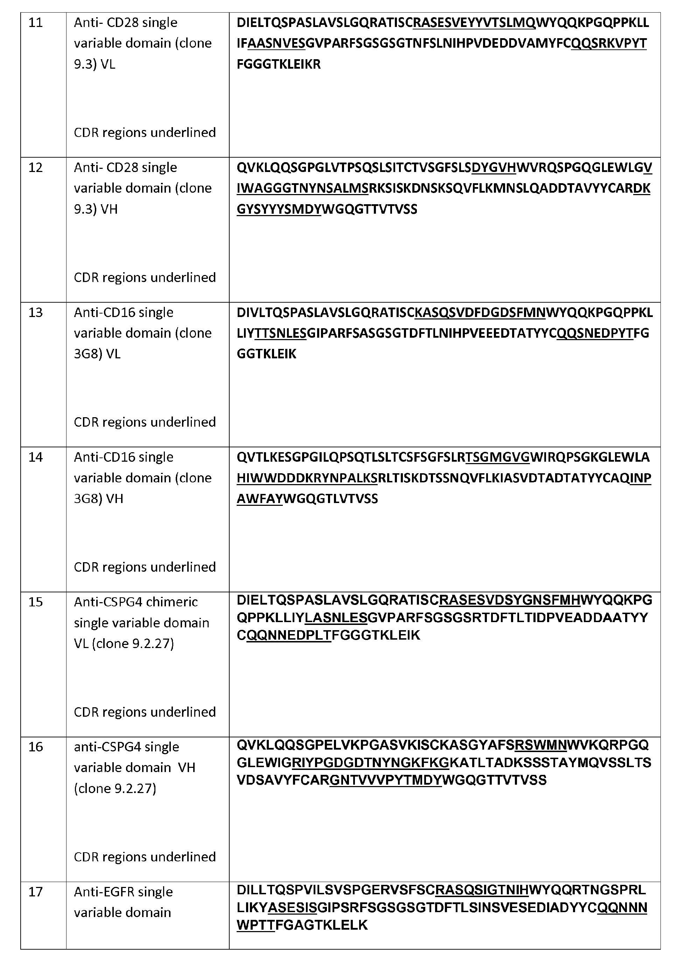

**Figure 6B** lists sequences that may be included in a recombinant antibody molecule of the invention. In SEQ ID NO: 20 parts of the hinge region and the CH2 region are depicted. The sequence SPPSPAPPVAG (SEQ ID NO: 98; also shaded in grey in SEQ ID NO: 20) represents the amino acids 226-237 (numbering of sequence positions according to the EU-index) of the CH2 domain after deletion of Gly236. Notably, the original sequence is CPPCPAPELLGG (SEQ ID NO: 99), wherein in comparison to SEQ ID NO: 98 the cysteines at positions 226 and 229 (numbering of sequence positions according to the EU-index) (in the hinge region) and the amino acid residues at positions 233-235 (numbering of sequence positions according to the EU-index) in the CH2 domain were exchanged (the exchanged amino acids were shaded in grey; also compare to Fig. 2I). The replacement of the cysteines at positions 226 and 229 (of the hinge region) between the CH1 and the CH2 domain results in these Fabv molecules of the invention not being able to form a (CH2 domain-mediated) disulfide-bridge between two of these monomers. The mutations in the CH2 domain at positions 233-236 (Glu233 was substituted by Pro, Leu234 was substutited by Val, Leu235 was substituted by Ala, and Gly236 was deleted) lead to an attenuation of the Fc function. In addition replacements at positions 265, 297, 327, 330 were indicated by shading them in grey. At position 265 the amino acid replacement is from D to G, at position 297 the amino acid replacement is from N to Q, at position 327 the amino acid replacement is from A to Q and at position 330 the amino acid replacement is from A to S. Also these introduced mutations into the CH2 domain attenuate the Fc function (numbering of sequence positions according to the EU-index).

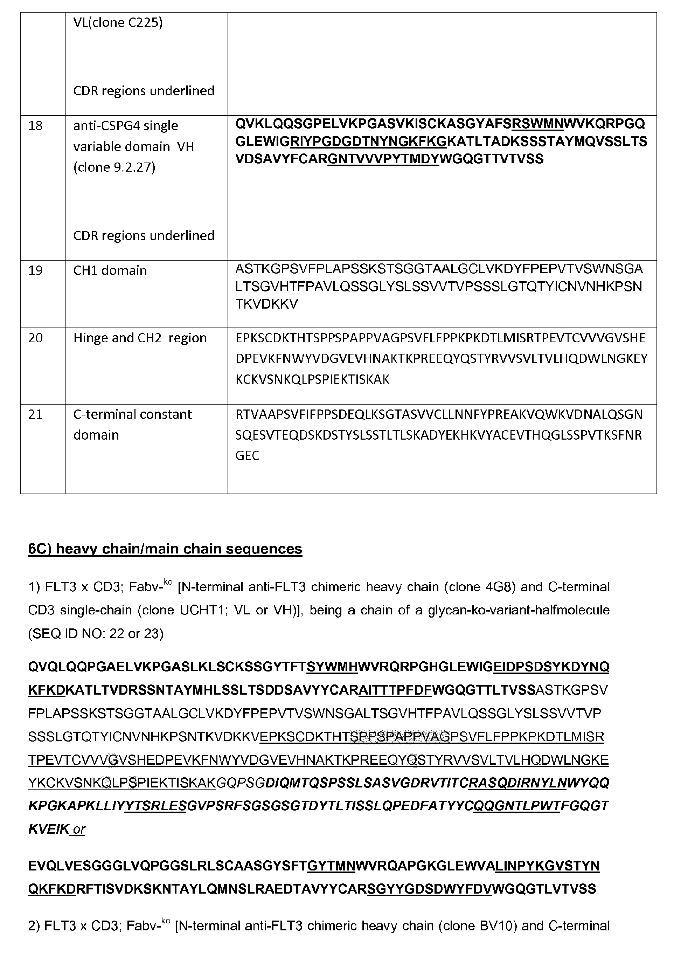

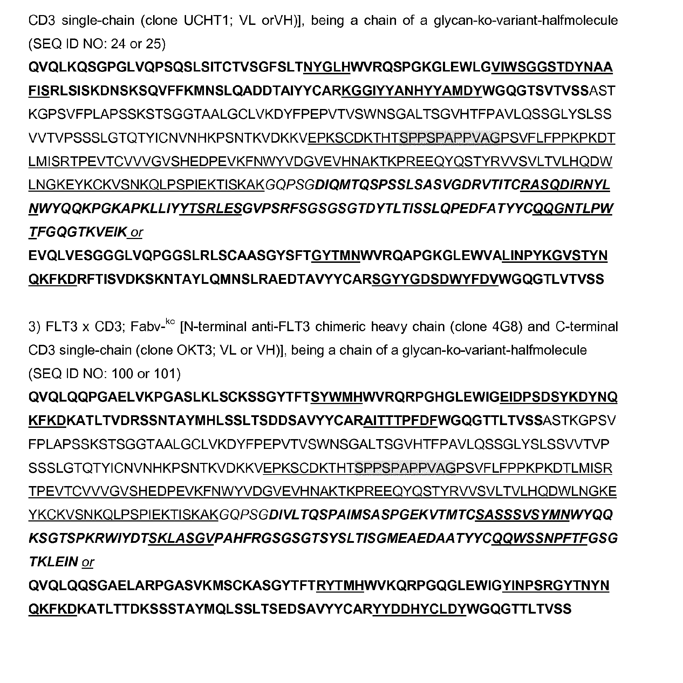

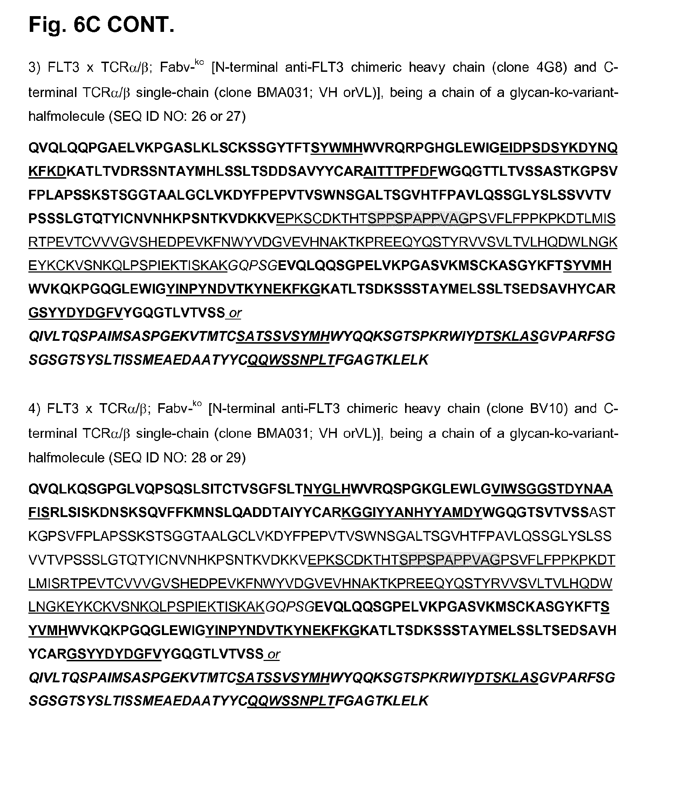

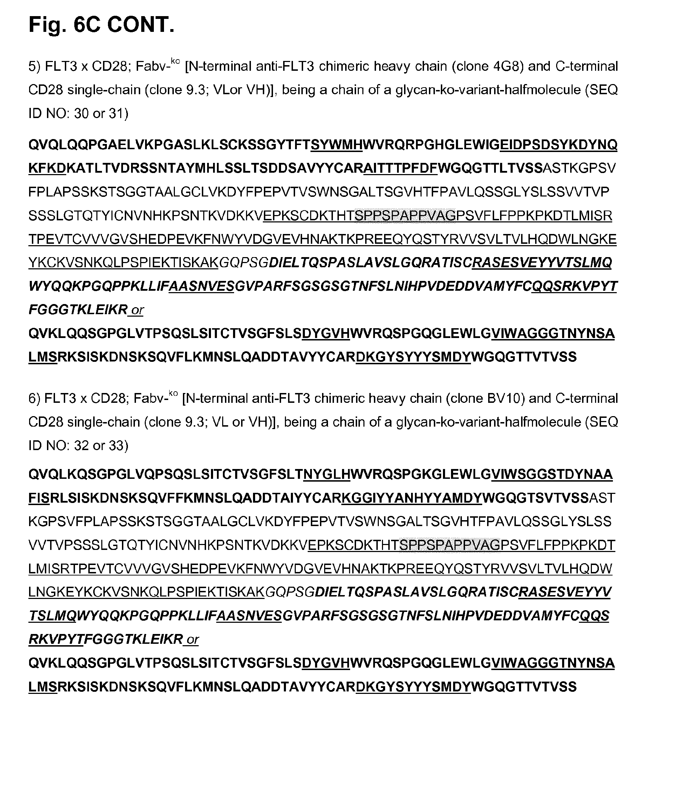

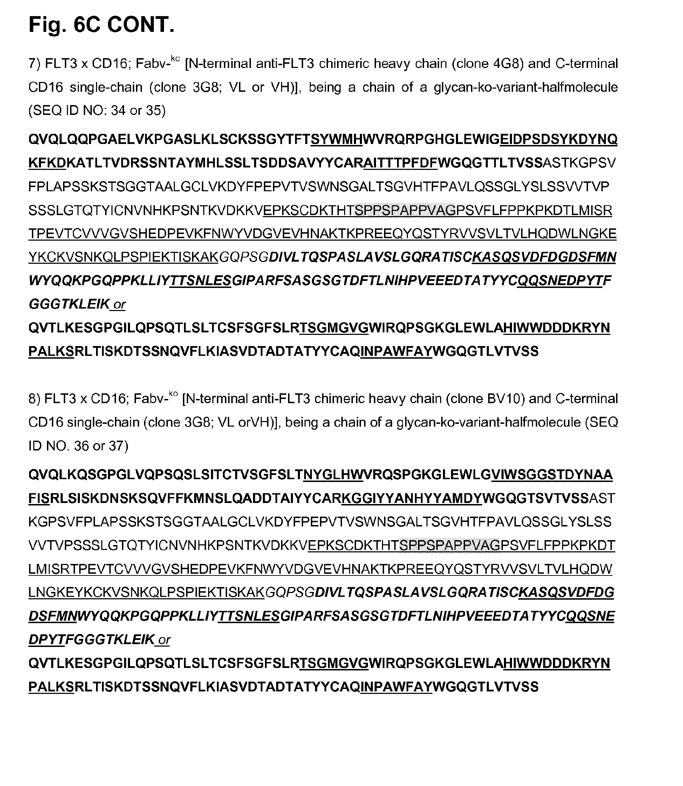

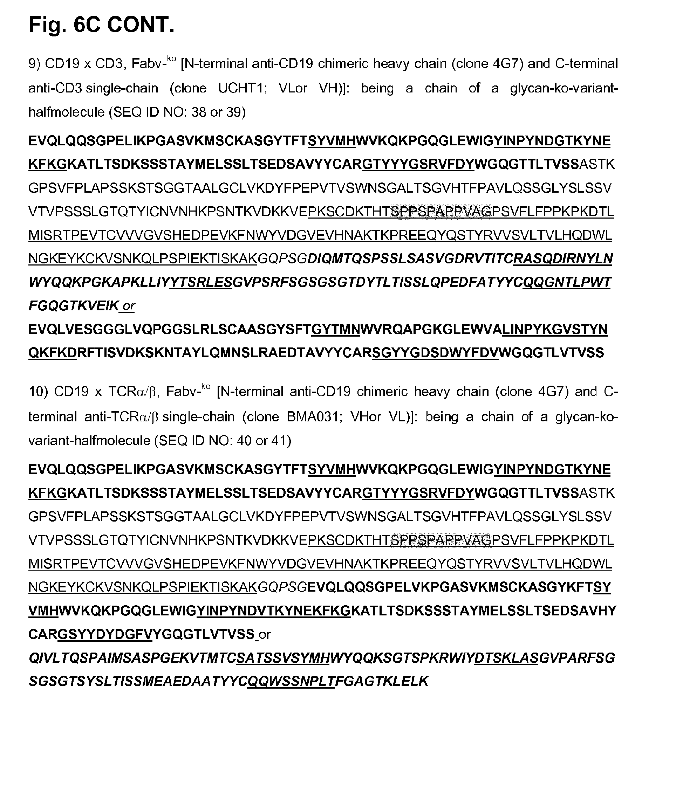

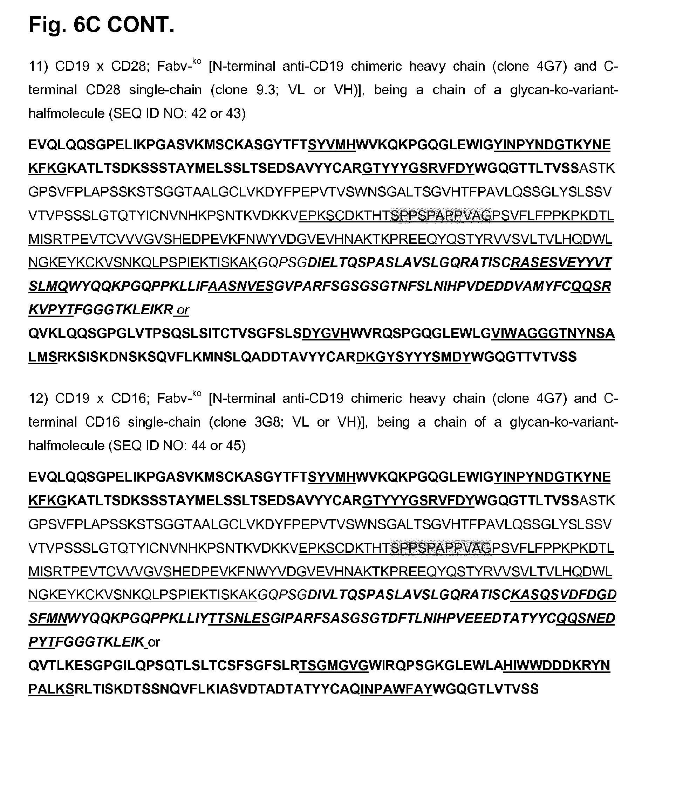

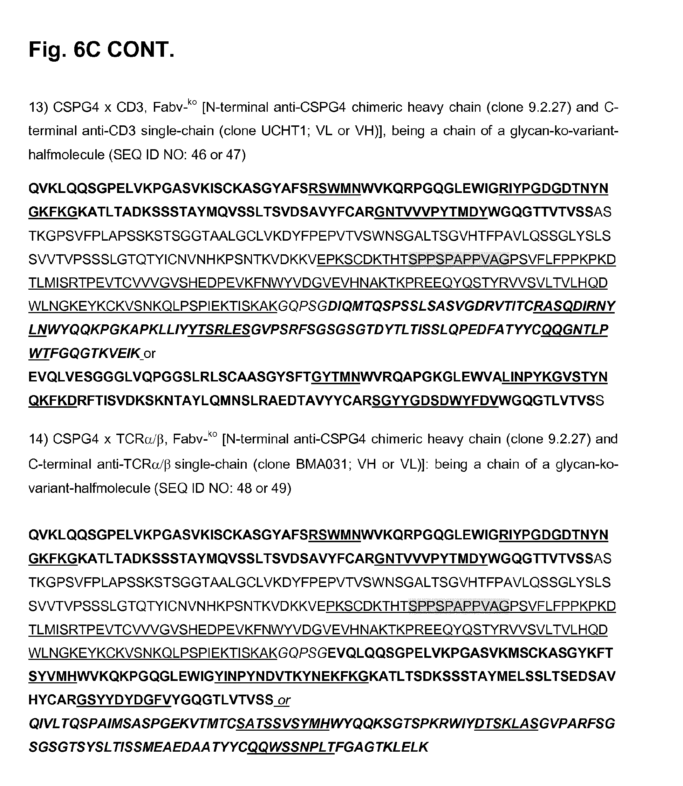

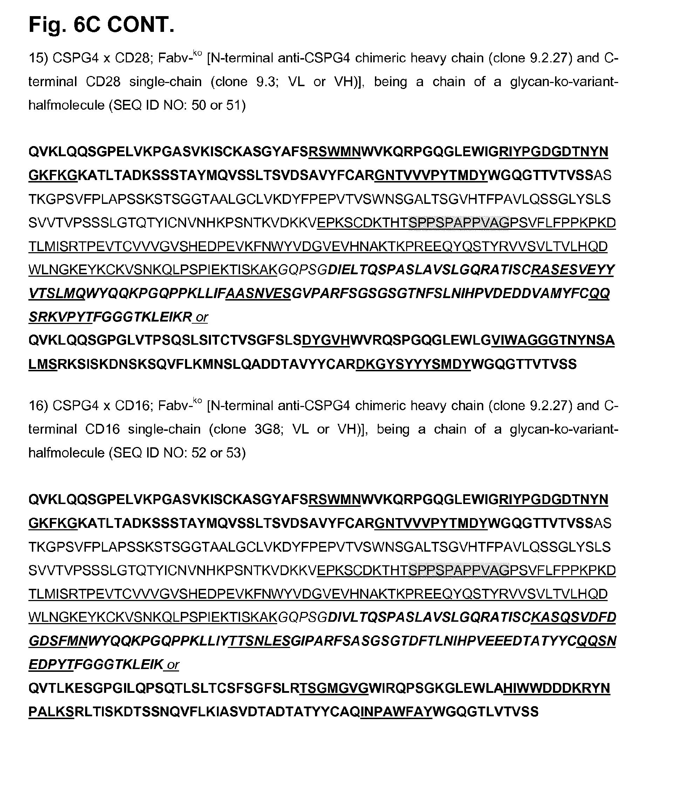

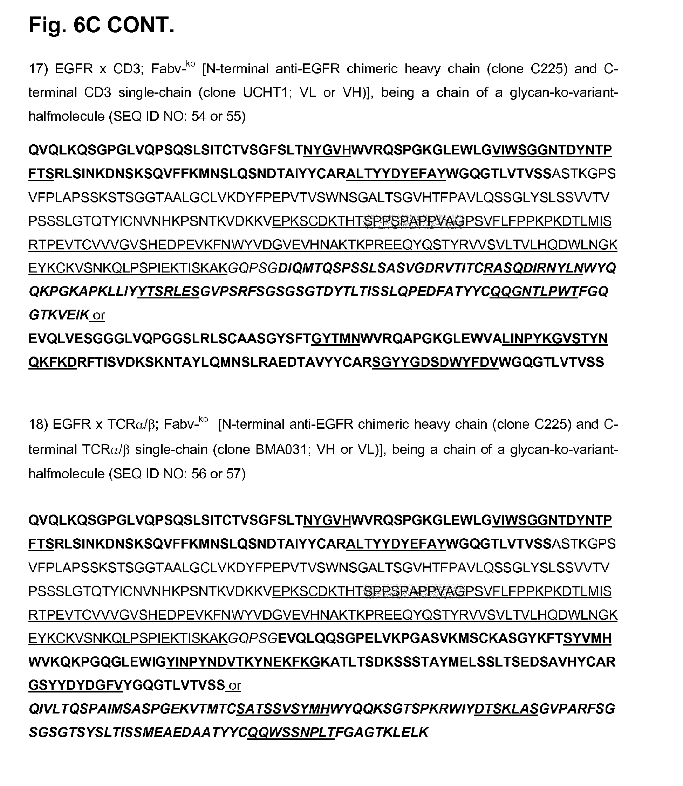

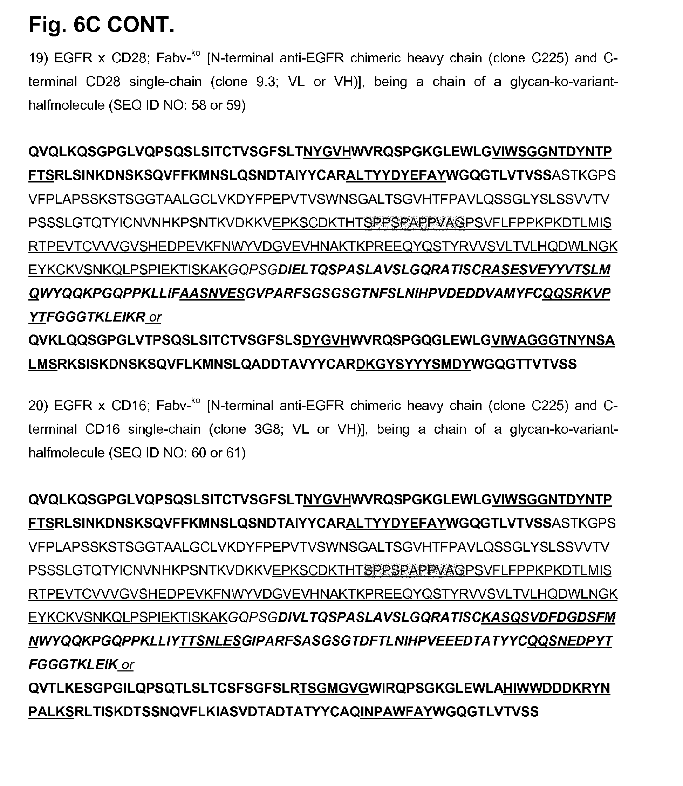

**Fig. 6C** depicts the sequences of illustrative main chains, which can in the present case also be addressed as heavy chains that may be included in an antibody of the invention. This particular main chain for the Fabv-^{ko} format (Figs 1A, 1A') includes a VH domain, a CH1 domain, a hinge region, a modified CH2 domain, a single VL domain or a VH domain.
   The VH domains are depicted in bold the CH1 domain in regular, and the hinge, CH2 regions in regular, underlined text. The main chain further includes a VL domain, which is depicted in bold, italic text, or a VH domain (bold)The complementarity determining residues (CDRs) of the respective VL or VH regions are underlined. The CH2 domain and the VH or VL domain are coupled to each other via a small linker (GQPSG), which is represented in italic. Furthermore, the position of SEQ ID NO: 98 was indicated by shading it in grey in the respective sequences.

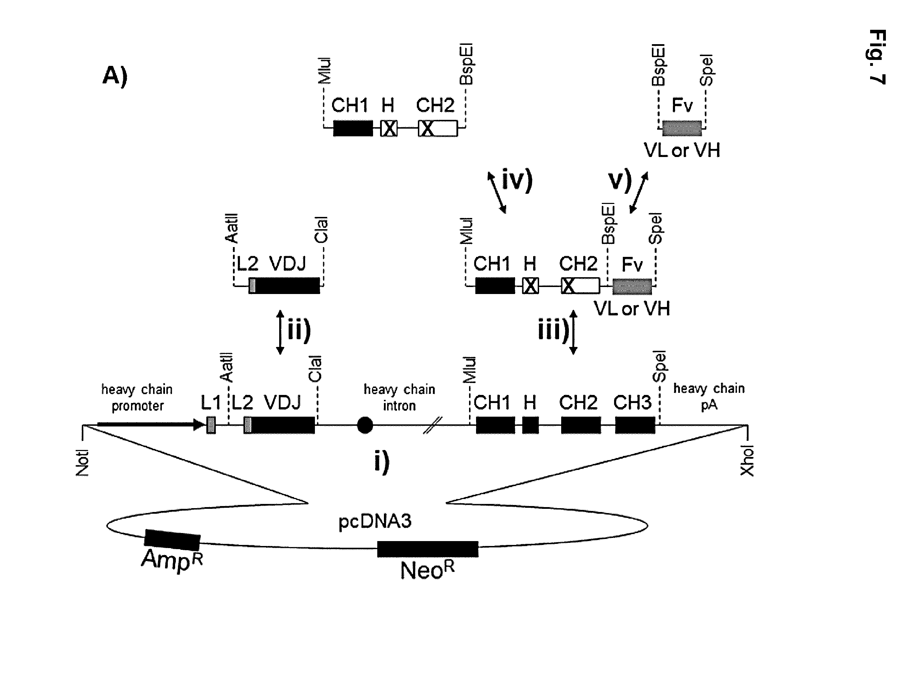

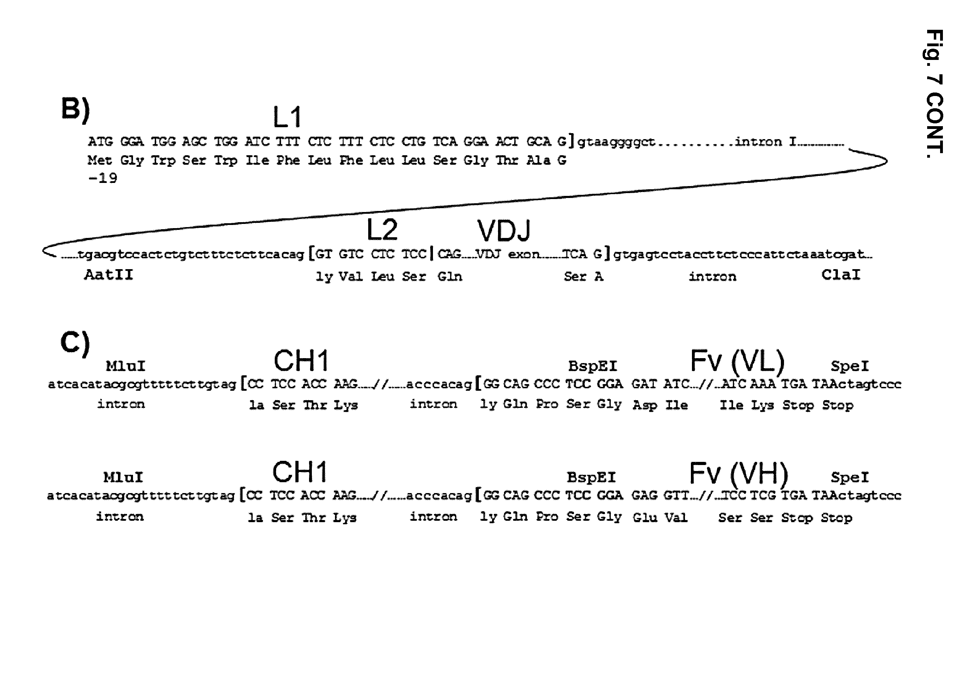

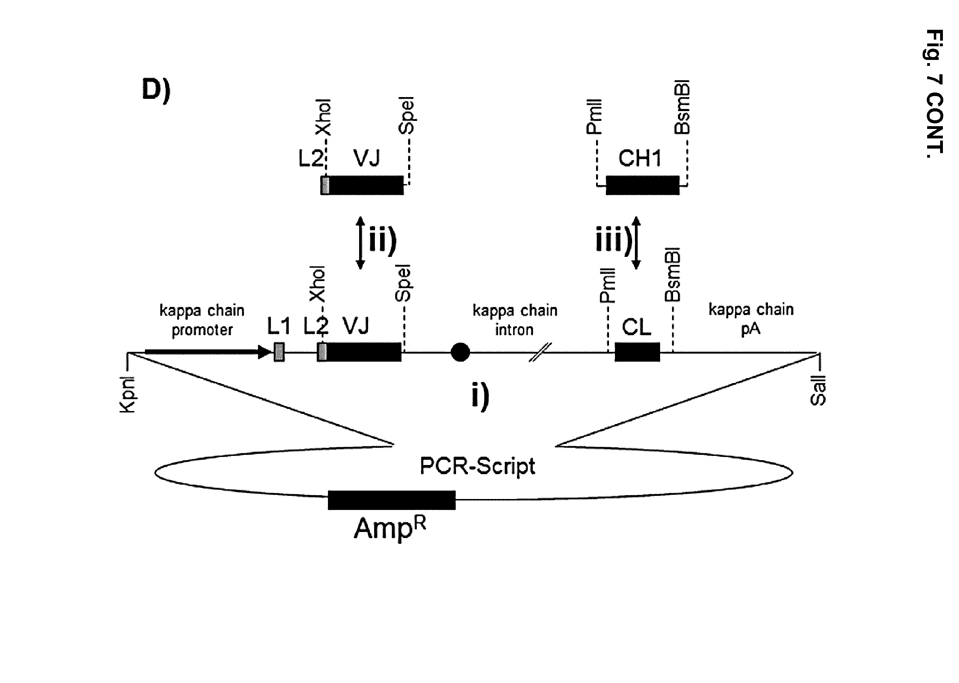

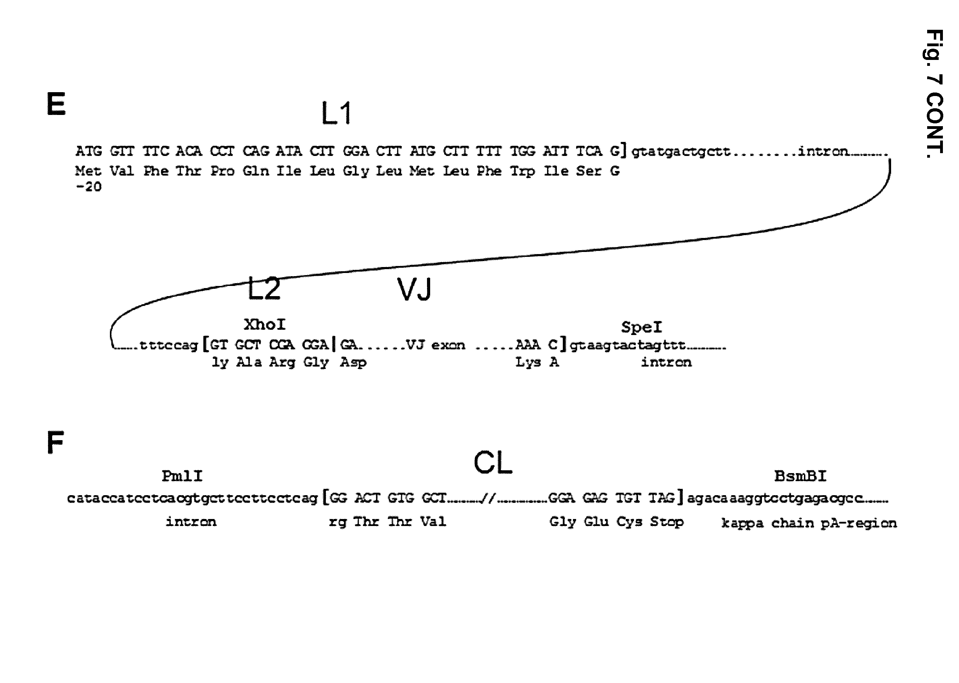

**Fig. 7** depicts how antibody molecules of the present invention can be obtained. Fig. 7A shows the original vector for the heavy chain, which contains the human γ1 isotype Ig heavy chain, which can be exchanged by a VJ region of a heavy chain of interest. The region relevant for cloning of a single variable domain fragment VL or VH is shown enlarged in Figure 7B. The Mlul-Spel fragment to be exchanged is shown enlarged in Figure 7C.

The original vector for the light chain contains the VJ region of the light chain and the C region of human κ gene are shown in Figure 7D. This VJ region of the light chain can be exchanged by a VJ region of a light chain of interest. The region adjacent to the fragment to be exchanged is shown in Figure 7E. The region adjacent to the fragment of the constant domain of the light chain (CL) to be exchanged is shown enlarged in Figure 7F.

### DETAILED DESCRIPTION

The combinatorial approach of the present invention uses two bifunctional antibody molecules, Ab1 and Ab2, targeting two different TAAs or the same TAA and each equipped with "one half" of an effector binding site, e.g. the VH or VL domain of an antibody, directed to the TCR/CD3 complex. Only if both antibodies bind to a target cell, a functional VH-VL effector-specificity, is generated by on site-complementation. This combinatorial principle enhances the specificity of the targeting- as well as of the effector-process and therefore solves the central problem of T cell activating bispecific antibodies, that is off-target T cell activation as outlined above in P1 and P2:
(i) increasing the specificity of the targeting process by a combination of two antibodies directed to different surface antigens limits T cell activation by normal cells carrying the respective target antigens (solves P1).
(ii) On site complementation of the effector specificity will prevent off-target T cell activation induced by binding of bispecific antibodies to T cells in the absence of target cells (solves P2).

As mentioned above, such a combinatorial approach was initially realized by coupling the VH-VL-single chain antibodies containing the variable regions of Ab1 and Ab2 to the VH and VL domains of the CD3-antibody OKT3, resulting in the triple domain single chain (tdsc)-molecules described in the International patent application WO 2013/104804 (see also Fig. 1).

However, the principal advantages of the antibody molecule format of the present invention, compared to an antibody in the tdsc-format consisting solely of three variable domains are (1) superior production rates, (2) improved serum half life, (3) preserved binding affinity of the targeting part and (4) decreased multimerization/aggregation tendency. Decreased multimerization/aggregation is particularly important, if the C-terminal single chain antibody is directed to the TCR/CD3 complex to induce target cell restricted T cell activation. In this case even small amounts of aggregates may lead to off-target T cell activation. It is therefore of importance to avoid aggregation by the selection of suitable antibodies and antibody formats.

The term "antibody" generally refers to a proteinaceous binding molecule with immunoglobulin-like functions. Typical examples of an antibody are immunoglobulins, as well as derivatives or functional fragments thereof which still retain the binding specificity. Techniques for the production of antibodies are well known in the art. The term "antibody" also includes immunoglobulins (Ig's) of different classes (i.e. IgA, IgG, IgM, IgD and IgE) and subclasses (such as IgG1, IgG2 etc.). Illustrative examples of an antibody are F_{ab} fragments, F(ab')₂, Fᵥ fragments, single-chain Fᵥ fragments (scFᵥ), diabodies or domain antibodies (Holt LJ et al., Trends Biotechnol. 21(11), 2003, 484-490). Domain antibodies may be single domain antibodies, single variable domain antibodies or immunoglobulin single variable domain having only one variable domain, which may be VH or VL, that specifically bind an antigen or epitope independently of other V regions or domains. Such an immunoglobulin single variable domain may not only encompass an isolated antibody single variable domain polypeptide, but also a larger polypeptide that includes or consists of one or more monomers of an antibody single variable domain polypeptide sequence. The definition of the term "antibody" thus also includes embodiments such as chimeric, single chain and humanized antibodies.

An antibody molecule according to the invention may carry one or more domains that have a sequence with at least about 60 %, at least about 70 %, at least about 75 %, at least about 80 %, at least about 85 %, at least about 90 %, at least about 92 %, at least about 95 %, at least about 96 %, at least about 97 %, at least about 98 % or at least about 99 % sequence identity with a corresponding naturally occuring domain of an immunoglobulin M, an immunoglobulin G, an immunoglobulin A, an immunoglobulin D or an immunoglobulin E. It is noted in this regard, the term "about" or "approximately" as used herein means within a deviation of 20%, such as within a deviation of 10% or within 5% of a given value or range.

Accordingly, the main chain (longer polypeptide chain) of an antibody molecule of the invention may include, including consist of, domains with the above sequence identity with a corresponding domain of an immunoglobulin mu heavy chain, of an immunoglobulin gamma heavy chain, of an immunoglobulin alpha heavy chain, of an immunoglobulin delta heavy chain or of an immunoglobulin epsilon heavy chain. Further, an antibody molecule of the invention may include, including consist of, domains with the above sequence identity with a corresponding domain of an immunoglobulin lambda light chain or of an immunoglobulin kappa light chain. In some embodiments the entire heavy chain domains of an antibody molecule according to the invention have at least about 60 %, at least about 70 %, at least about 75 %, at least about 80 %, at least about 85 %, at least about 90 %, at least about 92 %, at least about 95 %, at least about 97 %, at least about 98 % or at least about 99 % sequence identity with the corresponding regions of an immunoglobulin mu heavy chain, of an immunoglobulin gamma heavy chain (such as gamma 1, gamma 2, gamma 3 or gamma 4 heavy chains), of an immunoglobulin alpha heavy chain (such as alpha 1 or alpha 2 heavy chains), of an immunoglobulin delta heavy chain or of an immunoglobulin epsilon heavy chain. In some embodiments all light chain domains present in an antibody molecule according to the invention have at least about 60 %, at least about 70 %, at least about 75 %, at least about 80 %, at least about 85 %, at least about 90 %, at least about 92 %, at least about 95 %, at least about 97 %, at least about 98 % or at least about 99 % sequence identity with the corresponding regions of an immunoglobulin lambda light chain (such as lambda 1, lambda 2, lambda 3 or lambda 4 light chains) or of an immunoglobulin kappa light chain.

"Percent (%) sequence identity" with respect to amino acid sequences disclosed herein is defined as the percentage of amino acid residues in a candidate sequence that are pair-wise identical with the amino acid residues in a reference sequence, i.e. an antibody molecule of the present disclosure, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publically available computer software such as BLAST, ALIGN, or Megalign (DNASTAR) software. Those skilled in the art can determine appropriate parameters for measuring alignment, including any algorithms needed to achieve maximum alignment over the full length of the sequences being compared. The same is true for nucleotide sequences disclosed herein.

The term "variable" refers to the portions of the immunoglobulin domains that exhibit variability in their sequence and that are involved in determining the specificity and binding affinity of a particular antibody (i.e., the "variable domain(s)"). Variability is not evenly distributed throughout the variable domains of antibodies; it is concentrated in sub-domains of each of the heavy and light chain variable regions. These sub-domains are called "hypervariable regions", "HVR," or "HV," or "complementarity determining regions" (CDRs). The more conserved (i.e., non-hypervariable) portions of the variable domains are called the "framework" regions (FR). The variable domains of naturally occurring heavy and light chains each include four FR regions, largely adopting a β-sheet configuration, connected by three hypervariable regions, which form loops connecting, and in some cases forming part of, the β -sheet structure. The hypervariable regions in each chain are held together in close proximity by the FR and, with the hypervariable regions from the other chain, contribute to the formation of the antigen- binding site (see Kabat et al., see below). Generally, naturally occurring immunoglobulins include six CDRs (see below); three in the VH (H1, H2, H3), and three in the VL (L1, L2, L3). In naturally occurring immunoglobulins, H3 and L3 display the most diversity of the six CDRs, and H3 in particular is believed to play a unique role in conferring fine specificity to immunoglobulins. The constant domains are not directly involved in antigen binding, but exhibit various effector functions, such as, for example, antibody-dependent, cell-mediated cytotoxicity and complement activation.

The corresponding immunoglobulin mu heavy chain, gamma heavy chain, alpha heavy chain, delta heavy chain, epsilon heavy chain, lambda light chain or kappa light chain may be of any species, such as a mammalian species, including a rodent species, an amphibian, e.g. of the subclass Lissamphibia that includes e.g. frogs, toads, salamanders or newts or an invertebrate species. Examples of mammals include, but are not limited to, a rat, a mouse, a rabbit, a guinea pig, a squirrel, a hamster, a hedgehog, a platypus, an American pika, an armadillo, a dog, a lemur, a goat, a pig, a cow, an opossum, a horse, a bat, a woodchuck, an orang-utan, a rhesus monkey, a woolly monkey, a macaque, a chimpanzee, a tamarin (saguinus oedipus), a marmoset or a human.

The term "immunoglobulin" refers to a glycoprotein that includes at least two heavy (H) chains and two light (L) chains linked by disulfide bonds, or an antigen binding portion thereof. Each heavy chain has a heavy chain variable region (abbreviated herein as V_{H}) and a heavy chain constant region. In some embodiments the heavy chain constant region includes three domains, C_{H1}, C_{H2} and C_{H3}. Each light chain has a light chain variable region (abbreviated herein as V_{L}) and a light chain constant region. The light chain constant region includes one domain, C_{L}. The V_{H} and V_{L} regions can be further subdivided into regions of hypervariability, termed complementarity determining regions (CDR), interspersed with regions that are more conserved, termed framework regions (FR). The CDRs contain most of the residues responsible for specific interactions of the antibody with the antigen. Each V_{H} and V_{L} has three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an epitope of an antigen.

Each light chain of an immunoglobulin includes an N-terminal variable (V) domain (VL) and a constant (C) domain (CL). Each heavy chain includes an N-terminal V domain (VH), three or four C domains (CHs), and a hinge region. An antibody molecule according to the invention likewise contains these domains and regions (even though the second binding site of a Fabv-molecule consists of a single VH or VL domain and the formation of a functional binding site requires the heterodimerization of two Fabv-molecules as depicted in Fig. 1).

An immunoglobulin when used herein, is typically a tetrameric glycosylated protein composed of two light (L) chains of approximately 25 kDa each and two heavy (H) chains of approximately 50 kDa each. Two types of light chain, termed lambda and kappa, may be found in immunoglobulins. Depending on the amino acid sequence of the constant domain of heavy chains, immunoglobulins can be assigned to five major classes: A, D, E, G, and M, and several of these may be further divided into subclasses (isotypes), e.g., IgG1, IgG2, IgG3, IgG4, IgA1, and IgA2. An IgM immunoglobulin consists of 5 of the basic heterotetramer unit along with an additional polypeptide called a J chain, and contains 10 antigen binding sites, while IgA immunoglobulins contain from 2-5 of the basic 4-chain units which can polymerize to form polyvalent assemblages in combination with the J chain. In the case of IgGs, the 4-chain unit is generally about 150,000 daltons.

As used herein, "recombinant" refers to the alteration of genetic material by human intervention. Typically, recombinant refers to the manipulation of DNA or RNA in a virus, cell, plasmid or vector by molecular biology (recombinant DNA technology) methods, including cloning and recombination. A recombinant cell, polypeptide, or nucleic acid can be typically described with reference to how it differs from a naturally occurring counterpart (the "wild-type"). A "recombinant antibody molecule" refers to a an antibody molecule, preferably a Fabv antibody molecule that has been genetically altered to comprise a amino acid sequence which is not found in nature

The term "amino acid" or "amino acid residue" refers to an α- or β-amino carboxylic acid.

When used in connection with a protein or peptide, the term "amino acid" or "amino acid residue" typically refers to an α-amino carboxylic acid having its art recognized definition such as an amino acid selected from the group consisting of: L-alanine (Ala or A); L-arginine (Arg or R); L-asparagine (Asn or N); L-aspartic acid (Asp or D); L-cysteine (Cys or C); L-glutamine (Gln or Q); L-glutamic acid (Glu or E); glycine (Gly or G); L-histidine (His or H); L-isoleucine (ILE or I): L-leucine (Leu or L); L-lysine (Lys or K); L-methionine (Met or M); L-phenylalanine (Phe or F); L-proline (Pro or P); L-serine (Ser or S); L-threonine (Thr or T); L-tryptophan (Trp or W); L-tyrosine (Tyr or Y); and L-valine (Val or V), although modified, synthetic, or rare amino acids such as e.g. taurine, ornithine, selenocysteine, homocystine, hydroxyproline, thioproline, iodo-tyrosine, 3-nitro-tyrosine, ornithine, citrulline, canavanine, 5-hydroxytryptophane, carnosine, cycloleucine, 3,4-dihydroxy phenylalanine, N-acetylcysteine, prolinol, allylglycine or acetidine-2-carboxylic acid may be used as desired. Generally, amino acids can be grouped as having a nonpolar side chain (e.g., Ala, Cys, ILE, Leu, Met, Phe, Pro, Val); a negatively charged side chain (e.g., Asp, Glu); a positively charged side chain (e.g., Arg, His, Lys); or an uncharged polar side chain (e.g., Asn, Cys, Gln, Gly, His, Met, Phe, Ser, Thr, Trp, and Tyr).

The term "epitope", also known as the "antigenic determinant", refers to the portion of an antigen to which an antibody or T-cell receptor specifically binds, thereby forming a complex. Thus, the term "epitope" includes any molecule or protein determinant capable of specific binding to an immunoglobulin or T-cell receptor. The binding site(s) (paratope) of an antibody molecule described herein may specifically bind to/interact with conformational or continuous epitopes, which are unique for the target structure. Epitopic determinants usually consist of chemically active surface groupings of molecules such as amino acids or sugar side chains and usually have specific three dimensional structural characteristics, as well as specific charge characteristics. In some embodiments, epitope determinants include chemically active surface groupings of molecules such as amino acids, sugar side chains, phosphoryl, or sulfonyl, and, in certain embodiments, may have specific three dimensional structural characteristics, and/or specific charge characteristics. With regard to polypeptide antigens a conformational or discontinuous epitope is characterized by the presence of two or more discrete amino acid residues, separated in the primary sequence, but assembling to a consistent structure on the surface of the molecule when the polypeptide folds into the native protein/antigen (Sela, M., Science (1969) 166, 1365-1374; Laver, W.G., et al. Cell (1990) 61, 553-556). The two or more discrete amino acid residues contributing to the epitope may be present on separate sections of one or more polypeptide chain(s). These residues come together on the surface of the molecule when the polypeptide chain(s) fold(s) into a three-dimensional structure to constitute the epitope. In contrast, a continuous or linear epitope consists of two or more discrete amino acid residues, which are present in a single linear segment of a polypeptide chain. As an illustrative example, a "context-dependent" CD3 epitope refers to the conformation of said epitope. Such a context-dependent epitope, localized on the epsilon chain of CD3, can only develop its correct conformation if it is embedded within the rest of the epsilon chain and held in the right position by heterodimerization of the epsilon chain with either CD3 gamma or delta chain. In contrast thereto, a context-independent CD3 epitope may be an N-terminal 1-27 amino acid residue polypeptide or a functional fragment thereof of CD3 epsilon. Generally, epitopes can be linear in nature or can be a discontinuous epitope. Thus, as used herein, the term "conformational epitope" refers to a discontinuous epitope formed by a spatial relationship between amino acids of an antigen other than an unbroken series of amino acids. The term "epitope" also includes an antigenic determinant of a hapten, which is known as a small molecule that can serve as an antigen by displaying one or more immunologically recognized epitopes upon binding to larger matter such as a larger molecule e.g. a protein.

An antibody or antibody molecule/fragment is said to specifically bind to an antigen when it recognizes its target antigen in a complex mixture of proteins and/or macromolecules. Antibodies are said to "bind to the same epitope" if the antibodies cross-compete so that only one antibody can bind to the epitope at a given point of time, i.e. one antibody prevents the binding or modulating effect of the other.

The term "specific" in this context, or "specifically recognizing", also used as "directed to", means in accordance with this invention that the antibody molecule is capable of specifically interacting with and/or binding to at least two, e.g. at least three or at least four amino acids of an epitope but does not essentially bind to another epitope or antigen. Such binding may be exemplified by the specificity of a "lock-and-key-principle". Specific binding is believed to be effected by specific motifs in the amino acid sequence of the binding region of the antibody, and the antibody and the epitope or the antigen bind to each other as a result of their primary, secondary or tertiary structure as well as the result of secondary modifications of said structure. The specific interaction of the epitope/antigen-interaction-site with its specific epitope/antigen may result as well in a simple binding of said site to the antigen. Moreover, the specific interaction of the antigen-interaction-site with its specific epitope/antigen may alternatively result in the initiation of a signal, such as for instance due to the induction of a change of the conformation of the antigen or an oligomerization of the antigen.

Typically, binding is considered specific when the binding affinity is higher than 10⁻⁶ M. In particular, binding is considered specific when binding affinity is about 10⁻⁸ to 10⁻¹¹ M (K_{D}), or of about 10⁻⁹ to 10⁻¹¹ M or even higher. Thus, antibody molecules with an affinity of the first binding site and/or the second binding site and/or the third binding site in the picomolar range (with a K_{D} of 10⁻¹² M) are also encompassed in the present invention. If necessary, nonspecific binding of a binding site can be reduced without substantially affecting specific binding by varying the binding conditions.

In some embodiments an antigen to which an antibody molecule according to the invention binds is an antigen that is included in the extracellular matrix or it is a cell surface antigen. In some embodiments an antigen to which an antibody according to the invention binds is a tumor associated antigen. In some embodiments, either the first binding site and/or the second binding site and/or the third binding site binds a tumor associated antigen. In other embodiments, the tumor associated antigen is located on the vasculature of a tumor. In further embodiments, the tumor associated antigen is selected from the group consisting of CD10, CD19, CD20, CD21, CD22, CD25, CD30, CD33, CD34, CD37, CD44v6, CD45, CDw52, Fms-like tyrosine kinase 3 (FLT-3, CD135), c-Kit (CD117), CSF1 R, (CD115), CD133, PDGFR-α (CD140a), PDGFR-β (CD140b), chondroitin sulfate proteoglycan 4 (CSPG4, melanoma-associated chondroitin sulfate proteoglycan), Muc-1, EGFR, de2-7-EGFR, EGFRvIII, Folate binding protein, Her2neu, Her3, PSMA, PSCA, PSA, TAG-72, HLA-DR, IGFR, CD133, IL3R, fibroblast activating protein (FAP), Carboanhydrase IX (MN/CA IX), Carcinoembryonic antigen (CEA), EpCAM, CDCP1, Derlin1, Tenascin, frizzled 1-10, the vascular antigens VEGFR2 (KDR/FLK1), VEGFR3 (FLT4, CD309), Endoglin, CLEC14, Tem1-8, the CUB-domain containing protein (CDCP)1 and Tie2.

It is understood that such a tumour associated antigen may be included in the extracellular matrix or be a cell surface antigen.

The term "extracellular matrix" refers to the tissue region of a multicellular animal, including a human that is found in the intercellular space, i.e. between the cells of the respective tissue. The extracellular matrix is largely a network of proteins such as fibrillar and non-fibrillar collagens or elastin, of glycoproteins such as laminin or fibronectin, of proteoglycans, such as chondroitin sulfate or keratan sulphate and of polysaccharides such as Hyaluronic acid. The extracellular matrix serves inter alia in segregating different tissues from each other or in regulating intercellular communication. In some embodiments a tumor associated antigen may be expressed partly or exclusively at the extracellular matrix of a tumor.

The term "cell surface antigen" as used herein refers to a molecule that is displayed on the surface of a cell. Typically such a molecule is located in or on the plasma membrane of the cell such that at least part of this molecule remains accessible from the ambience, i.e. from outside the cell. A respective molecule consists of or includes typically amino acid and/or saccharide moieties. An illustrative example of a cell surface molecule, which is located in the plasma membrane, is a transmembrane protein that, in its three-dimensional conformation, has regions of hydrophilicity and hydrophobicity. One or more hydrophobic region(s) allow(s) the cell surface molecule to be embedded, or inserted in the hydrophobic plasma membrane of the cell whereas hydrophilic regions of the protein extend on either side of the plasma membrane into the cytoplasm and extracellular space, respectively. Examples of a cell surface molecule located on the plasma membrane include, but are not limited to, a protein with a posttranslationally modified cysteine residue carrying a palmitoyl group, a protein modified at a C-terminal cysteine residue carrying a farnesyl group or a protein modified at the C-terminus carrying a glycosyl phosphatidyl inositol ("GPI") anchor. These groups allow covalent attachment of proteins to the outer surface of the plasma membrane, where they remain accessible for recognition by extracellular molecules such as antibodies. Examples of cell surface antigens include a cell surface receptor molecule such as a G protein coupled receptor (e.g. the β adrenergic receptor), a tyrosin kinase receptor (such as EGFR, EGFRvIII, Her2/neu, HER2/c-neu, PDGFRα, ILR-1, TNFR, CD30, CD33 or GMCSFR), a membrane receptor with associated tyrosin kinase activity (such as IL6R or LIFR) or a membrane receptor with Ser/Thr kinase activity (such as TGFβR), to name only a few examples.

Examples of a tumor associated antigen that is included in the extracellular matrix include, but are not limited to, a proteoglycan such as Melanoma-associated Chondroitin Sulfate Proteoglycan (CSPG4) or CD44v6, including a mucin such as Muc-1 or a membrane-bound enzyme such as Carbonic anhydrase IX (CAIX). Additional examples for such antigens are tenascin and the fibroblast activating protein (FAP).

The term "isolated antibody molecule" as used herein refers to an antibody molecule that has been identified and separated and/or recovered from a component of its natural environment. Contaminant components of its natural environment are matter that would interfere with diagnostic or therapeutic uses for the antibody molecule, and may include enzymes, hormones, and other proteinaceous or nonproteinaceous solutes. In some embodiments the antibody molecule is purified to greater than 95% by weight of antibody molecule as determined by the Lowry method, such as more than 99% by weight. In some embodiments the antibody molecule is purified to a degree sufficient to obtain at least 15 residues of N-terminal or internal amino acid sequence by use of a spinning cup sequenator. In some embodiments the antibody molecule is purified to homogeneity as judged by SDS-PAGE under reducing or nonreducing conditions using Coomassie blue or, preferably, silver stain. An isolated antibody molecule may in some embodiments be present within recombinant cells with one or more component(s) of the antibody's natural environment not being present. Typically an isolated antibody molecule is prepared by at least one purification step.

As indicated above the terms "V_{H}" (also referred to as VH) and "V_{L}" (also referred to as VL) are used herein to refer to the heavy chain variable domain and light chain variable domain respectively of an immunoglobulin. An immunoglobulin light or heavy chain variable region consists of a "framework" region interrupted by three hypervariable regions. Thus, the term "hypervariable region" refers to the amino acid residues of an antibody which are responsible for antigen binding. The hypervariable region includes amino acid residues from a "Complementarity Determining Region" or "CDR". There are three heavy chains and three light chain CDRs (or CDR regions) in the variable portion of an immunoglobulin. Thus, "CDRs" as used herein refers to all three heavy chain CDRs (CDRH1, CDRH2 and CDRH3), or all three light chain CDRs (CDRL1, CDRL2 and CDRL3) or both all heavy and all light chain CDRs, if appropriate. Three CDRs make up the binding character of a light chain variable region and three make up the binding character of a heavy chain variable region. CDRs determine the antigen specificity of an immunoglobulin molecule and are separated by amino acid sequences that include scaffolding or framework regions. The exact definitional CDR boundaries and lengths are subject to different classification and numbering systems. The structure and protein folding of the antibody may mean that other residues are considered part of the antigen binding region and would be understood to be so by a skilled person. CDRs provide the majority of contact residues for the binding of the immunoglobulin to the antigen or epitope.

CDR3 is typically the greatest source of molecular diversity within the antibody-binding site. H3, for example, can be as short as two amino acid residues or greater than 26 amino acids. The subunit structures and three-dimensional configurations of different classes of immunoglobulins are well known in the art. For a review of the antibody structure, see Antibodies: A Laboratory Manual, Cold Spring Harbor Laboratory, eds. Harlow et al., 1988. One of skill in the art will recognize that each subunit structure, e.g., a CH, VH, CL, VL, CDR, FR structure, includes active fragments, e.g., the portion of the VH, VL, or CDR subunit binds to the antigen, i.e., the antigen-binding fragment, or, e.g., the portion of the CH subunit that binds to and/or activates, e.g., an Fc receptor and/or complement. The CDRs typically refer to the Kabat CDRs, as described in Sequences of Proteins of immunological Interest, US Department of Health and Human Services (1991), eds. Kabat et al. Another standard for characterizing the antigen binding site is to refer to the hypervariable loops as described by Chothia. See, e.g., Chothia, et al. (1992; J. Mol. Biol. 227:799-817; and Tomlinson et al. (1995) EMBO J. 14:4628-4638. Still another standard is the AbM definition used by Oxford Molecular's AbM antibody modelling software. See, generally, e.g., Protein Sequence and Structure Analysis of Antibody Variable Domains. In: Antibody Engineering Lab Manual (Ed.: Duebel, S. and Kontermann, R., Springer-Verlag, Heidelberg). Embodiments described with respect to Kabat CDRs can alternatively be implemented using similar described relationships with respect to Chothia hypervariable loops or to the AbM-defined loops.

"Framework Region" or "FR" residues are those variable domain residues other than the hypervariable region. The sequences of the framework regions of different light or heavy chains are relatively conserved within a species. Thus, a "human framework region" is a framework region that is substantially identical (about 85% or more, usually 90-95% or more) to the framework region of a naturally occurring human immunoglobulin. The framework region of an antibody, that is the combined framework regions of the constituent light and heavy chains, serves to position and align the CDR's. The CDR's are primarily responsible for binding to an epitope of an antigen.

The terms "Fab", "Fab region", "Fab portion" or "Fab fragment" are understood to define a polypeptide that includes a V_{H}, a C_{H}1, a V_{L}, and a C_{L} immunoglobulin domain. Fab may refer to this region in isolation, or this region in the context of an antibody molecule according to the invention, as well as a full length immunoglobulin or immunoglobulin fragment. Typically a Fab region contains an entire light chain of an antibody. A Fab region can be taken to define "an arm" of an immunoglobulin molecule. It contains the epitope-binding portion of that Ig. The Fab region of a naturally occurring immunoglobulin can be obtained as a proteolytic fragment by a papain-digestion. A "F(ab')₂ portion" is the proteolytic fragment of a pepsin-digested immunoglobulin. A "Fab' portion" is the product resulting from reducing the disulfide bonds of an F(ab')₂ portion. As used herein the terms "Fab", "Fab region", "Fab portion" or "Fab fragment" may further include a hinge region that defines the C-terminal end of the antibody arm (cf. above). This hinge region corresponds to the hinge region found C-terminally of the C_{H}1 domain within a full length immunoglobulin at which the arms of the antibody molecule can be taken to define a Y. The term hinge region is used in the art because an immunoglobulin has some flexibility at this region.

A "Fabv 1½" or "Fabv" as used herein relate to a recombinant antibody molecule, which has one and a half binding domains (binding sites). Such a recombinant antibody molecule can consist of a Fab fragment comprising a first binding site for a first antigen, a variable domain of either the light chain or the heavy chain of a second binding site for a second antigen and an immunoglobulin CH2 domain, wherein the Fab fragment and the (single/unpaired) variable domain are linked via the CH2 domain. Of course, the herein described modifications may be included into these antibody molecules (see also Fig. 2A-E, 2A'-E' and 2I). In some embodiments, the antibody molecule of the present invention (Fabv), comprises the light chain of the variable domain of the second binding site for the second antigen. In other embdoiments, the antibody molecule of the present invention (Fabv) comprises a heavy chain of the variable domain of the second binding site for the second antigen.

On the other hand a "Fabv 3" as used herein relates to hetero-dimeric antibody molecules, which comprises two recombinant antibody molecules of the present invention. As such these molecules may be bispecific or tri-specific as described herein (see also Fig. 1 and 2) depending whether they are heterodimers in which two Fabv molecules bind to the same first (target)- antigen or heterodimers in which two Fabv molecules bindto different target antigens A and B, . In the latter case a molecule with three specificities is formed (Fig. 1 and 2).

The term "Fc region" or "Fc fragment" is used herein to define a C-terminal region of an immunoglobulin heavy chain, including native-sequence Fc regions and variant Fc regions. The Fc part mediates the effector function of antibodies, e.g. the activation of the complement system and of Fc-receptor bearing immune effector cells, such as NK cells. In human IgG molecules, the Fc region is generated by papain cleavage N-terminal to Cys226. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody molecule, or by recombinantly engineering the nucleic acid encoding a heavy chain of the antibody molecule. Accordingly, a composition of intact antibodies may include antibody populations with all K447 residues removed, antibody populations with no K447 residues removed, and antibody populations having a mixture of antibodies with and without the K447 residue. Suitable native-sequence Fc regions for use in the antibodies of the invention include mammalian, e.g. human or murine, IgG1, IgG2 (IgG2A, IgG2B), IgG3 and IgG4. The Fc region contains two or three constant domains, depending on the class of the antibody. In embodiments where the immunoglobulin is an IgG the Fc region has a C_{H}2 and a C_{H}3 domain.

An antibody molecule according to the invention has two chains, a shorter chain, which may in some embodiments be a light chain, and a main chain, which may in some embodiments also be addressed as the heavy chain. The antibody molecule is usually a dimer of these two chains. On the basis of the domains included in an antibody molecule of the invention the antibody molecule can be taken to have a Fab fragment, which generally includes a hinge region, a C_{H}2 domain and a single VH or VL domain. In some embodiments the arrangement of the domains of an antibody of the invention corresponds to the arrangement of domains in an immunoglobulin. As two examples, the shorter chain of an antibody molecule of the invention may have a VL domain at the N-terminus and a CL domain at the C-terminus of the shorter chain, and the main chain may have a VH domain at the N-terminus and a CH1 domain C-terminally thereto. In some embodiments the shorter chain may have a VL domain at the N-terminus and a CH1 domain at the C-terminus of the shorter chain. In some embodiments the shorter chain may have a VH domain at the N-terminus and a CH1 domain at the C-terminus of the shorter chain. In some embodiments the shorter chain may have a VH domain at the N-terminus and a CL domain at the C-terminus of the shorter chain. In some embodiments the main chain may have a VL domain at the N-terminus and a CH1 domain C-terminally thereto. In some embodiments the main chain may have a VH domain at the N-terminus and a CL domain C-terminally thereto. In some embodiments the main chain may have a VL domain at the N-terminus and a CL domain C-terminally thereto.

The shorter chain of the antibody may be linked to the main chain of the antibody by means of one or more, including two or three, disulphide bonds. A respective disulphide bond may define a bridge between a C-terminal cysteine residue of the smaller chain and a cysteine residue within the hinge region of the main chain of the antibody.

In an antibody molecule according to the invention the C-terminal region of the main chain may be defined by a single VH or VL domain. The C-terminus of the main chain may in some embodiments be defined by a VH domain. In some embodiments the C-terminus of the main chain may be defined by a VL domain. Accordingly, the VH or VL domain may in some embodiments be coupled to the CH2 domainof the main chain via the VH domain, e.g. the N-terminal end of the VH domain. In some embodiments the VH or VL domain may be coupled to the CH2 domainof the main chain via the VL domain, e.g. the N-terminal end of the VL domain.

The Fab fragment of an antibody molecule according to the invention is in some embodiments linked to the CH2 domain via a heavy chain domain of the Fab fragment. Accordingly, the main chain of the antibody molecule may have a heavy chain domain such as a CH1 domain (supra), which is coupled to the CH2 domain. As explained above, a respective CH1 domain may be coupled to the CH2 domain via a hinge region. The respective heavy chain domain of the Fab fragment may in some embodiments be arranged at the N-terminus of the polypeptide chain of the main chain of the antibody molecule. In some embodiments the Fab fragment of an antibody molecule according to the invention is linked to the CH2 domain via a light chain domain of the Fab fragment. Accordingly, the main chain of the antibody molecule may have a light chain domain such as a CL domain, which is coupled to the CH2 domain. Again, a respective CL domain may be coupled to the CH2 domain via a hinge region. The respective light chain domain of the Fab fragment may in some embodiments be arranged at the N-terminus of the polypeptide chain of the main chain of the antibody molecule. To prevent dimerization of the molecules the cysteine residues in the hinge region providing inter-chain disulfide bonds may be replaced, for example, by any other amino acid (e.g. a serine or alanine) or deleted (Fig. 2A-E, 2A'-E').

In hetero-dimeric embodiments (Figs. 2F, 2F") these cysteine residues can be preserved in one Fabv antibody molecule of the hetero-dimer. In these embodiments the antibody molecule can accordingly be taken to define a hetero-dimeric antibody molecule as described above and each main chain and each shorter chain can be individually selected. As an example, the first of the shorter chains may have a VL domain at the N-terminus and a CL domain at the C-terminus. The first main chain may have a VH domain at the N-terminus and a CH1 domain C-terminally thereto (see Fig. 2F, 2F'). Further, the first main chain may have a CH2, as well as a C-terminal VH domain or VL domain. The second of the shorter chains may have a VH domain at the N-terminus and a CH1 domain at the C-terminus. The second main chain may have a VL domain at the N-terminus and a CL domain C-terminally thereto. The second main chain may also have a CH2, as well as a C-terminal VH domain or VL domain. In some embodiments, the Fab fragment that comprises the first binding site for the first antigen consists of the VL domain fused to the CH1 domain and the VH domain fused to the CL domain. The CH1 domain of the Fab fragment can also be fused to the CH2 domain.

In further embodiments, the antibody molecule of the present invention, may comprise a light chain of the variable domain of the second binding site having a sequence identity of at least 80%, or at least 85 %, or at least 90 %, or at least 95 %, or at least 98 %, or at least 99 % or 100 % to SEQ ID NO. 3 (the sequence of the variable domain of the light chain of the CD3 binding antibody UCHT-1). In other embodiments, the antibody molecule of the present invention may comprise a heavy chain of the variable domain of the second binding site having a sequence identity of at least 80% or at least 85 %, or at least 90 %, or at least 95 %, or at least 98 %, or at least 99 % or 100 % to SEQ ID NO. 4 (the sequence of the variable domain of the heavy chain of UCHT-1). Fabv antibodies containing the VH-or VL-domains of the antibody UCHT1 form monomeric molecules have little tendency to form homo-aggregates (Fig. 3).

In one embodiment a disulphide bond between the hinge domain of (the CH2 domain) of the first main chain and a hinge domain of (the CH2 domain) of the second main chain is defined by at least one of a cysteine residue at sequence position 226 and a cysteine residue at sequence position 229 of one of the hinge domains, according to the Kabat numbering [EU-Index]. In other embodiments a hetero-dimeric antibody molecule may have one or more disulphide bonds linking the hinge regions of the two main chains of the antibody molecules and a disulphide bond linking the hinge regions of the two main chains of the antibody molecules. In some embodiments two antibody molecules of a hetero-dimeric antibody molecule according to the invention may be linked by means of a disulphide bond that is defined by a cysteine residue that is included in the CH2 domain of the main chain of a first antibody molecule and a cysteine residue that is included in the CH2 domain of the main chain of a second antibody molecule.

As a further example, the first of the shorter chains may have a VL domain at the N-terminus and a CH1 domain at the C-terminus. The first main chain may have a VH domain at the N-terminus and C-terminally linked thereto a CL domain. Further, the first main chain may have a CH2, as well as a C-terminal VH domain or VL domain. The second of the shorter chains may have a VL domain at the N-terminus and a CL domain at the C-terminus. The second main chain may have a VH domain at the N-terminus and a CH1 domain C-terminally thereto. The second main chain may also have a CH2, as well as a C-terminal VH domain or VL domain

In other embodiments, the hetero-dimeric antibody molecule comprises in at least one of the immunoglobulin CH2 domain of the first monomer or the second monomer at least one cysteine residue that is able to form a disulfide bridge for dimerisation is lacking or mutated, thereby preventing that a disulfide bridge is formed between the two monomers or thereby preventing that a CH2-domain mediated disulfide bridge is formed between the two monomers. The CH2 domain of the present invention connects the Fab domain with a single VH or VL domain (see Fig. 1). However, the CH2 domain can also include a hinge domain as depicted in Fig. 2A-J, 2A'-J'. Thus, by referring to a CH2-mediated disulphide bridge, equally a disulphide bridge formed by the hinge region and the CH2 region are meant to be included in this term. For example, the at least one cysteine residue can be selected from the sequence positions 226, 228 and 229 of the CH2 domain, preferably the cysteine at one or both of positions 226 and 229 (numbering of sequence positions according to the EU-index) is lacking or replaced by a different amino acid. These amino acids are depicted as being located in the hinge region in Fig. 2A-J, 2A'-J' or as being located within the CH2 domain in Fig. 1). In further embdiments, in at least one of the immunoglobulin CH2 domain of the first monomer or the second monomer an amino acid residue of the CH2 domain that is able to mediate binding to an Fc-receptor is lacking or mutated. Preferably the at least one amino acid residue is selected from the group consisting of sequence positions 228, 230, 231, 232, 233, 234, 235, 236, 237, 238, 265, 297, 327 and 330 (numbering of sequence positions according to the EU-index). The at least one mutation can be selected from the group consisting of a deletion of amino acid 228, a deletion of amino acid 230, a deletion of amino acid 231, a deletion of amino acid 232, a deletion of amino acid 233, a substitution Glu233→Pro, a deletion of amino acid 234, a substitution of amino acid Leu234→Val, a deletion of amino acid 235, a substitution Leu235→Ala, a deletion of amino acid 236, a deletion of amino acid 237, a deletion of amino acid 238, a substitution Asp265→Gly, a substitution Asn297→Gln, a substitution Ala327→Gln, and a substitution Ala330→Ser. Thus in one embodiment, the antibody molecule of the present invention has a Fabv formate, which comprises a modified hinge region. In other embodiments, the antibody molecule has a Fabv^{ko} formate, which includes modifications in the hinge region and CH2 region. Notably, the modifications as described herein for the Fabv (Fabv-formats) apply mutatis mutandis to the Fabv^{ko} formats).

A "bispecific" or "bifunctional" antibody molecule is an antibody molecule that has two different epitope/antigen binding sites, and accordingly has binding specificities for two different epitopes. These two epitopes may be epitopes of the same antigen or of different antigens. In contrast thereto a "bivalent antibody" may have two binding sites of identical antigenic specificity.

A "bispecific antibody" may be, defined by a first pair of heavy and light chain or of main and shorter/smaller chain (supra), and binds a different antigen or epitope on a second arm, defined by a second pair of heavy and light chain or of main and smaller chain. Such an embodiment of a bispecific antibody has two distinct antigen binding arms, in both specificity and CDR sequences. Typically, a bispecific antibody is monovalent for each antigen it binds to. A bispecific antibody is a hybrid antibody molecule, which may have a first binding region that is defined by a first light chain variable region and a first heavy chain variable region, and a second binding region that is defined by a second light chain variable region and a second heavy chain variable region. In some embodiments one of these binding regions may be defined by a heavy/light chain pair. As explained above, in the context of the present invention the bispecific heterodimeric antibody molecule has a first binding site, defined by variable regions of a main chain and a smaller chain, and a second, different half-binding site defined by a variable region of a VH domain or VL domain that are included in the main chain of the single antibody molecules. Two such molecules carrying the VH or VL domain, derived from an antibody with a second specificity, may form hetero-dimers in which the VH and VL domains restore the specificity of the second antibody.

In particular a bispecific heterodimeric antibody molecule comprises a hetero-dimer of the antibody molecule such as a recombinant antibody molecule (Fabv molecule) of the present invention
wherein the first monomer of the hetero-dimer consists of a Fab fragment comprising a first binding site for a first antigen, a variable domain of the light chain of a second binding site for a second antigen and an immunoglobulin CH2 domain, wherein the Fab fragment and the variable domain of the light chain are linked via the CH2 domain, and
wherein the second monomer of the hetero-dimer consists of a Fab fragment comprising a first binding site for the first antigen, a variable domain of the heavy chain of the second binding site for the second antigen and an immunoglobulin CH2 domain, wherein the Fab fragment and the variable domain of the heavy chain are linked via the CH2 domain.

Similarly, a bispecific heterodimeric antibody molecule can comprise a hetero-dimer of the antibody molecule such as a recombinant antibody molecule of the present invention (Fabv molecule), wherein the first monomer of the hetero-dimer consists of a Fab fragment comprising a first binding site for a first antigen, a variable domain of the heavy chain of a second binding site for a second antigen and an immunoglobulin CH2 domain, wherein the Fab fragment and the variable domain of the heavy chain are linked via the CH2 domain, and
wherein the second monomer of the hetero-dimer consists of a Fab fragment comprising a first binding site for the first antigen, a variable domain of the light chain of the second binding site for the second antigen and an immunoglobulin CH2 domain, wherein the Fab fragment and the variable domain of the light chain are linked via the CH2 domain,
wherein the variable domain of the light chain of the second binding site of the first monomer and the variable domain of the heavy chain of the second binding site of the second monomer associate thereby forming, the second binding site and dimerizing the hetero-dimer.

As outlined above, the second binding site is formed by the single VH and VL domains present in 2 different monomers. For the association of these two single domains it is necessary, that they come into close contact. This is the case upon binding to the specific epitope(s) they recognise. Thus, the association of the two monomers takes place on the target cell, comprising the epitope(s) to be detected. To ensure optimal association of the Fabv molecules, preferably these single domains should be obtained from only one antibody such as the UCHT-1 or the Okt3 antibody as described herein. However, it can also be possible to combine single VH and VL domains within the Fabv-formate from different antibodies. For example such VH/VL domains could be obtained form different antibodies, which epitoes are located spatially close to each other or which have similar or overlapping epitopes.

Thus, dimerization of the monomers into a hetero-dimer is mediated by the association of the single (unpaired) VH and VL domains of the two Fabv monomers. However, as outlined above, depending on the construction of the monomers, it may still be that disulphide bridges are formed by the remaining amino acid residues (cysteines), which could then also contribute to the dimerization process. However for dimerization to occur a spatial adjacency is necessary. This adjacency is primarily achieved by the binding to the targeted epitope(s).

A "tri-specific" or "trifunctional" hetero-dimeric antibody molecule is an antibody molecule that has three different epitope/antigen binding sites, and accordingly has binding specificities for three different target epitopes. These three epitopes may be epitopes of the same antigen or of different antigens. In contrast thereto a "trivalent antibody" may have binding sites of identical antigenic specificity.

A "tri-specific antibody" may be an antibody molecule that binds one antigen or epitope on one of two or more binding arms, defined by a first pair of heavy and light chain or of main and shorter/smaller chain (supra), binds a different antigen or epitope on a second arm, defined by a second pair of heavy and light chain or of main and smaller chain and it binds to a further antigen by the single VH or VL domain which, when brought into close proximity represent a further binding domain (see also Fig. 2H, 1B). Notably, the third binding domain is different from the first binding domain. Thus, the first and third binding domain sole have different specificities. Such an embodiment of a tri-specific heterodimeric antibody has three distinct antigen binding arms, in all specificity and CDR sequences. Typically, a tri-specific antibody is monovalent for each antigen it binds to. A tri-specific antibody is a hybrid antibody molecule, which may have a first binding region that is defined by a first light chain variable region and a first heavy chain variable region, and a second binding region that is defined by a second light chain variable region and a second heavy chain variable region, and a third binding region that is defined by a third light chain variable region and a third heavy chain variable region. In some embodiments one of these binding regions may be defined by a heavy/light chain pair. As explained above, in the context of the present invention the tri-specific heterodimeric antibody molecule has a first binding site, defined by variable regions of a main chain and a smaller chain, and a third, different binding site defined by a variable region of a VH domain and VL domain that are included in the main chain of the single antibody molecules and a second binding domain which is a composite of the spare single VH and VL domains of two (1½) Fabv antibody molecules of the present invention.

In particular, a tri-specific heterodimeric antibody molecule comprises a hetero-dimer of the antibody molecule such as a recombinant antibody molecule of the present invention (Fabv molecule),
wherein the first monomer of the hetero-dimer consists of a Fab fragment comprising a first binding site for a first antigen, a variable domain of the light chain of a second binding site for a second antigen and an immunoglobulin CH2 domain, wherein the Fab fragment and the variable domain of the light chain are linked via the CH2 domain, and
wherein the second monomer of the hetero-dimer consists of a Fab fragment comprising a third binding site for a third antigen, wherein the third antigen is different from the first antigen, a variable domain of the heavy chain of the second binding site for the second antigen and an immunoglobulin CH2 domain, wherein the Fab fragment and the variable domain of the heavy chain are linked via the CH2 domain. Similarly, a tri-specific heterodimeric antibody molecule comprises a hetero-dimer of a recombinant antibody molecule such as a recombinant antibody molecule of the present invention (Fabv molecule),
wherein the first monomer of the hetero-dimer consists of a Fab fragment comprising a first binding site for a first antigen, wherein the third antigen is different from the first antigen, a variable domain of the heavy chain of a second binding site for a second antigen and an immunoglobulin CH2 domain, wherein the Fab fragment and the variable domain of the heavy chain are linked via the CH2 domain, and
wherein the second monomer of the hetero-dimer consists of a Fab fragment comprising a third binding site for a third antigen, a variable domain of the light chain of the second binding site for the second antigen and an immunoglobulin CH2 domain, wherein the Fab fragment and the variable domain of the light chain are linked via the CH2 domain,
wherein the variable domain of the light chain of the second binding site of the first monomer and the variable domain of the heavy chain of the second binding site of the second monomer associate thereby forming, the second binding site and dimerizing the hetero-dimer. In some embodiments, the monomers in the hetero-dimers are in the Fabv (Fabv) format. In other embodiments, the monomers in the hetero-dimers are in the Fabv^{ko}-format. It is, however, also possible that one monomer is in the Fabv-formate, while the second monomer is in the Fabv^{ko}-format or vice versa. Similarly, one antibody molecule (monomer) may comprise any modifcations in the hinge and or CH2 domain, while the other monomer is in the Fabv-format or in the Fabv^{ko}-format.

Methods of making antibody molecules are known in the art, e.g. chemical conjugation of two different Fabs or a Fab with a VL or VH domain for example, also chemical conjugation of two antibody fragments, for example, of two Fab fragments or a Fab with a VL or VH domain. Alternatively, bispecific or tri-specific antibody molecules are made recombinantly. It is possible to insert the coding sequences for antibody molecules such as the Fabv into one expression vector. Thus, a method of producing a antibody molecule comprises expressing a nucleic acid encoding the antibody molecule under conditions allowing expression of the nucleic acid, preferably the antibody molecule is expressed in a host cell or a cell-free system.

The bispecific or tri-specific antibody molecule of the invention can act as a monoclonal antibody (MAb) with respect to each target. In some embodiments the antibody is chimeric, humanized or fully human.

A "dual-specific antibody", which may for instance be a full-length immunoglobulin or a construct with immunoglobulin like binding properties, is generally understood to have two binding arms, in particular arms defined by a pair of HC/LC, that can bind two different antigens or epitopes with each of its arms (see PCT publication WO 02/02773). Accordingly a dual-specific binding protein has two identical antigen binding arms, with identical specificity and identical CDR sequences, and is bivalent for each antigen it binds to.

The T cell receptor (TCR) is a particular receptor that is present on the cell surface of T cells, i.e. T lymphocytes. *In vivo* the T cell receptor exists as a complex of several proteins. The T cell receptor generally has two separate peptide chains, typically T cell receptor alpha and beta (TCRα and TCRβ) chains, on some T cells T cell receptor gamma and delta (TCRγ and TCRδ). The other proteins in the complex are the CD3 proteins: CD3εγ and CD3εδ heterodimers and, most important, a CD3ζ homodimer, which has a total of six ITAM motifs. The ITAM motifs on the CD3ζ can be phosphorylated by Lck and in turn recruit ZAP-70. Lck and/or ZAP-70 can also phosphorylate the tyrosines on many other molecules, not least CD28, LAT and SLP-76, which allows the aggregation of signalling complexes around these proteins.

An antibody molecule according to the invention can include a linker between the C-terminus of the CH2 region and the single VH or VL domain. The VH/VL domain is arranged at the C-terminus of the CH2 domain, either directly linked thereto or coupled via a linking peptide of typically 20 or less, including 10 or less amino acid residues. In some embodiments the sequence of a recombinant antibody molecule according to the invention can be compared against the sequence of IgG1, since the sequence of the antibody molecule according to the invention has a certain degree of similarity with the sequence of IgG1, as illustrated further below. In comparison to the amino acid sequence of IgG1 according to Kabat et al. (1991, Sequences of Proteins of Immunological Interest, 5th Ed., United States Public Health Service, National Institutes of Health, Bethesda) a main chain of an antibody molecule according to the invention in some embodiments includes a V_{H} domain at amino acid positions 1 to 117, a C_{H}1 domain at positions 118 to 215, a hinge region at positions 216 to 230 and a C_{H}2 domain at positions 231 to 340.

In accordance with the amino acid sequence of the main chain of an antibody of the invention the Fab fragment, consisting of the V_{H} domain, the C_{H}1 domain and the hinge region, in these embodiments typically spans amino acids 1 to 230. Within this Fab fragment the V_{H} domain is typically defined by amino acids 1 to 118, the C_{H}1 domain is defined by amino acids 119 to 216, and the hinge region is defined by amino acids 217 to 231, according to the Kabat numbering. The antibody chain with the sequence of SEQ ID NO: 22 or SEQ ID NO: 23 may serve as an example of a respective embodiment. In some embodiments the antibody molecule according to the invention has, at the positions 342 et sqq of the main chain, a chimeric sequence composed of a V_{H} domain or a V_{L} domain.

A bispecific or tri-specific hetero-dimeric antibody molecule according to the invention may have two or three binding sites of any desired specificity. In some embodiments one of the binding sites is capable of binding a tumour associated antigen. In some embodiments two of the binding sites are capable of binding a tumour associated antigen, which are typically named the first and third binding site. In some embodiments the binding site included in the Fab fragment is a binding site specific for a tumour associated surface antigen. The tumor associated surface antigen can be selected from the group consisting of CD10, CD19, CD20, CD21, CD22, CD25, CD30, CD33, CD34, CD37, CD44v6, CD45, CDw52, Fms-like tyrosine kinase 3 (FLT-3, CD135), c-Kit (CD117), CSF1R (CD115), CD133, PDGFR-α (CD140a), PDGFR-β (CD140b), chondroitin sulfate proteoglycan 4 (CSPG4, melanoma-associated chondroitin sulfate proteoglycan), Muc-1, EGFR, de2-7-EGFR, EGFRvIII, Folate binding protein, Her2neu, Her3, PSMA, PSCA, PSA, TAG-72, HLA-DR, IGFR, CD133, IL3R, fibroblast activating protein (FAP), Carboanhydrase IX (MN/CA IX), Carcinoembryonic antigen (CEA), EpCAM, CDCP1, Derlin1, Tenascin, frizzled 1-10, the vascular antigens VEGFR2 (KDR/FLK1), VEGFR3 (FLT4, CD309), Endoglin, CLEC14, Tem1-8, and Tie2.

In other embodiments, the second binding site binds a T-cell- or NK cell associated receptor molecule. Particularly, the T-cell- or NK cell associated receptor molecule is one of CD3, the T cell receptor (TCR), CD28, CD16, NKG2D,Ox40, 4-1 BB, CD2, CD5 and CD95. In one embodiment, the TCR is TCR (alpha/beta) or TCR (gamma/delta). With regard to the tri-specific hetero-dimeric antibody molecule also the first and/or third binding site can equally bind a tumor associated surface antigen or can bind to a T-cell- or NK cell associated receptor molecule.

The term "tumour associated surface antigen" as used herein refers to an antigen that is or can be presented on a surface that is located on or within tumour cells. These antigens can be presented on the cell surface with an extracellular part, which is often combined with a transmembrane and cytoplasmic part of the molecule. These antigens can in some embodiments be presented only by tumour cells and not by normal, i.e. non-tumour cells. Tumour antigens can be exclusively expressed on tumour cells or may represent a tumour specific mutation compared to non-tumour cells. In such an embodiment a respective antigen may be referred to as a tumour-specific antigen. Some antigens are presented by both tumour cells and non-tumour cells, which may be referred to as tumour-associated antigens. These tumour-associated antigens can be overexpressed on tumour cells when compared to non-tumour cells or are accessible for antibody binding in tumour cells due to the less compact structure of the tumour tissue compared to non-tumour tissue. In some embodiments the tumour associated surface antigen is located on the vasculature of a tumour.

Illustrative examples of a tumor associated surface antigen are CD10, CD19, CD20, CD22, CD33, Fms-like tyrosine kinase 3 (FLT-3, CD135), chondroitin sulfate proteoglycan 4 (CSPG4, melanoma-associated chondroitin sulfate proteoglycan), Epidermal growth factor receptor (EGFR), Her2neu, Her3, IGFR, CD133, IL3R, fibroblast activating protein (FAP), CDCP1, Derlin1, Tenascin, frizzled 1-10, the vascular antigens VEGFR2 (KDR/FLK1), VEGFR3 (FLT4, CD309), PDGFR-α (CD140a), PDGFR-β (CD140b) Endoglin, CLEC14, Tem1-8, and Tie2. Further examples may include A33, CAMPATH-1 (CDw52), Carcinoembryonic antigen (CEA), Carboanhydrase IX (MN/CA IX), CD21, CD25, CD30, CD34, CD37, CD44v6, CD45, CD133, de2-7 EGFR, EGFRvIII, EpCAM, Ep-CAM, Folate-binding protein, G250, Fms-like tyrosine kinase 3 (FLT-3, CD135), c-Kit (CD117), CSF1R (CD115), HLA-DR, IGFR, IL-2 receptor, IL3R, MCSP (Melanoma-associated cell surface chondroitin sulphate proteoglycane), Muc-1, Prostate-specific membrane antigen (PSMA), Prostate stem cell antigen (PSCA), Prostate specific antigen (PSA), and TAG-72. Examples of antigens expressed on the extracellular matrix of tumors are tenascin and the fibroblast activating protein (FAP).

In some embodiments one of the binding sites of an antibody molecule according to the invention is able to bind a T-cell specific receptor molecule and/or a natural killer cell (NK cell) specific receptor molecule. A T-cell specific receptor is the so called "T-cell receptor" (TCRs), which allows a T cell to bind to and, if additional signals are present, to be activated by and respond to an epitope/antigen presented by another cell called the antigen-presenting cell or APC. The T cell receptor is known to resemble a Fab fragment of a naturally occurring immunoglobulin. It is generally monovalent, encompassing α- and β-chains, in some embodiments it encompasses γ-chains and δ-chains (supra). Accordingly, in some embodiments the TCR is TCR (alpha/beta) and in some embodiments it is TCR (gamma/delta). The T cell receptor forms a complex with the CD3 T-Cell co-receptor. CD3 is a protein complex and is composed of four distinct chains. In mammals, the complex contains a CD3γ chain, a CD3δ chain, and two CD3ε chains. These chains associate with a molecule known as the T cell receptor (TCR) and the ζ-chain to generate an activation signal in T lymphocytes. Hence, in some embodiments a T-cell specific receptor is the CD3 T-Cell co-receptor. In some embodiments a T-cell specific receptor is CD28, a protein that is also expressed on T cells. CD28 can provide co-stimulatory signals, which are required for T cell activation. CD28 plays important roles in T-cell proliferation and survival, cytokine production, and T-helper type-2 development. Yet a further example of a T-cell specific receptor is CD134, also termed Ox40. CD134/OX40 is being expressed after 24 to 72 hours following activation and can be taken to define a secondary costimulatory molecule. Another example of a T-cell receptor is 4-1BB capable of binding to 4-1 BB-Ligand on antigen presenting cells (APCs), whereby a costimulatory signal for the T cell is generated. Another example of a receptor predominantly found on T-cells is CD5, which is also found on B cells at low levels. A further example of a receptor modifying T cell functions is CD95, also known as the Fas receptor, which mediates apoptotic signaling by Fas-ligand expressed on the surface of other cells. CD95 has been reported to modulate TCR/CD3-driven signaling pathways in resting T lymphocytes.

An example of a NK cell specific receptor molecule is CD16, a low affinity Fc receptor and NKG2D. An example of a receptor molecule that is present on the surface of both T cells and natural killer (NK) cells is CD2 and further members of the CD2-superfamily. CD2 is able to act as a co-stimulatory molecule on T and NK cells.

In some embodiments the first binding site of the antibody molecule binds a tumour associated surface antigen and the second binding site binds a T cell specific receptor molecule and/or a natural killer (NK) cell specific receptor molecule. In some embodiments the first and third binding site of the antibody molecule bind to different tumour associated surface antigens and the second binding site binds a T cell specific receptor molecule and/or a natural killer (NK) cell specific receptor molecule. In some embodiments the first binding site of the antibody molecule binds one of A33, CAMPATH-1 (CDw52), Carcinoembryonic antigen (CEA), Carboanhydrase IX (MN/CA IX), CD10, CD19, CD20, CD21, CD22, CD25, CD30, CD33, CD34, CD37, CD44v6, CD45, CD133, CDCP1, Her3, chondroitin sulfate proteoglycan 4 (CSPG4, melanoma-associated chondroitin sulfate proteoglycan), CLEC14, Derlin1, Epidermal growth factor receptor (EGFR), de2-7 EGFR, EGFRvIII, EpCAM, Endoglin, Ep-CAM, Fibroblast activation protein (FAP), Folate-binding protein, G250, Fms-like tyrosine kinase 3 (FLT-3, CD135), c-Kit (CD117), CSF1R (CD115), frizzled 1-10, Her2/neu, HLA-DR, IGFR, IL-2 receptor, IL3R, MCSP (Melanoma-associated cell surface chondroitin sulphate proteoglycane), Muc-1, Prostate-specific membrane antigen (PSMA), Prostate stem cell antigen (PSCA), Prostate specific antigen (PSA), TAG-72, Tenascin, Tem1-8, Tie2 and VEGFR2 (KDR/FLK1), VEGFR3 (FLT4, CD309), PDGFR-α (CD140a), PDGFR-β (CD140b), and the second binding site binds a T cell specific receptor molecule and/or a natural killer (NK) cell specific receptor molecule. In some embodiments the first binding site of the antibody molecule binds a tumour associated surface antigen and the second binding site binds one of CD3, the T cell receptor (TCR), CD28, CD16, NKG2D, Ox40, 4-1 BB, CD2, CD5 and CD95.

In some embodiments the first and/or third binding site of the antibody molecule binds a T cell specific receptor molecule and/or a natural killer (NK) cell specific receptor molecule and the second binding site binds a tumour associated surface antigen. In some embodiments the first binding site of the antibody binds a T cell specific receptor molecule and/or a natural killer (NK) cell specific receptor molecule and the second binding site binds one of A33, CAMPATH-1 (CDw52), Carcinoembryonic antigen (CEA), Carboanhydrase IX (MN/CA IX), CD10, CD19, CD20, CD21, CD22, CD25, CD30, CD33, CD34, CD37, CD44v6, CD45, CD133, CDCP1, Her3, chondroitin sulfate proteoglycan 4 (CSPG4, melanoma-associated chondroitin sulfate proteoglycan), CLEC14, Derlin1, Epidermal growth factor receptor (EGFR), de2-7 EGFR, EGFRvIII, EpCAM, Endoglin, Ep-CAM, Fibroblast activation protein (FAP), Folate-binding protein, G250, Fms-like tyrosine kinase 3 (FLT-3, CD135), frizzled 1-10, Her2/neu, HLA-DR, IGFR, IL-2 receptor, IL3R, MCSP (Melanoma-associated cell surface chondroitin sulphate proteoglycane), Muc-1, Prostate-specific membrane antigen (PSMA), Prostate specific antigen (PSA), TAG-72, Tenascin, Tem1-8, Tie2 and VEGFR. In some embodiments the first binding site of the antibody binds one of CD3, the T cell receptor (TCR), CD28, CD16, NKG2D, Ox40, 4-1 BB, CD2, CD5 and CD95, and the second binding site binds a tumour associated surface antigen.

The term "glycosylation" means the attachment of oligosaccharides (carbohydrates containing two or more simple sugars linked together e.g. from two to about twelve simple sugars linked together) to a glycoprotein. The oligosaccharide side chains are typically linked to the backbone of the glycoprotein through either N- or O-linkages. The oligosaccharides of antibodies disclosed herein occur generally are attached to a CH2 domain of an Fc region as N-linked oligosaccharides. "N-linked glycosylation" refers to the attachment of the carbohydrate moiety to an asparagine residue in a glycoprotein chain. The skilled artisan will recognize that, for example, each of murine IgG1, IgG2a, IgG2b and IgG3 as well as human IgG1, IgG2, IgG3, IgG4, IgA and IgD CH2 domains have a single site for N-linked glycosylation at residue 297.

Sequences of domains or regions included in an antibody molecule according to the invention may be sequences of any desired species. Depending on the subsequent use of the antibody molecule it may, nevertheless, be desirable in some embodiments, to introduce alterations that prevent undesired side effects caused by the antibody. The use of intact non-human antibodies in the treatment of human diseases or disorders carries with it the potential for the now well established problems of immunogenicity, which means that the immune system of the patient may recognise the non-human intact antibody as non-self and mount a neutralising response. This is particularly evident upon multiple administration of the non-human antibody to a human patient. Various techniques have been developed over the years to overcome these problems and generally involve reducing the composition of non-human amino acid sequences in the intact antibody whilst retaining the relative ease in obtaining non-human antibodies from an immunised animal e.g. mouse, rat or rabbit. Broadly two approaches have been used to achieve this. The first are chimeric antibodies, which generally have a non-human (e.g. rodent such as mouse) variable domain fused to a human constant region. Because the antigen-binding site of an antibody is defined by residues within the variable domains the chimeric antibody retains its binding affinity for the antigen but acquires the effector functions of the human constant region and are therefore able to perform effector functions such as described supra. Chimeric antibodies are typically produced using recombinant DNA methods. DNA encoding the antibodies (e.g. cDNA) is isolated and sequenced using conventional procedures (e.g. by using oligonucleotide probes that are capable of binding specifically to genes encoding the H and L chains of the antibody of the invention. Hybridoma cells serve as a typical source of such DNA. Once isolated, the DNA is placed into expression vectors which are then transfected into host cells such as *E. coli,* COS cells, CHO cells or myeloma cells that do not otherwise produce immunoglobulin protein to obtain synthesis of the antibody. The DNA may be modified by substituting the coding sequence for human L and H chains for the corresponding non-human, e.g. murine, H and L constant regions (see e.g. Morrison; PNAS [1984] 81, 6851).

The second approach involves the generation of humanised antibodies wherein the non-human content of the antibody is reduced by humanizing the variable domains. Two techniques for humanisation have gained popularity. The first is humanisation by CDR grafting. CDRs define loops (supra) and antigen-binding specificity of an antibody is mainly defined by the topography and by the chemical characteristics of its CDR surface. These features are in turn determined by the conformation of the individual CDRs, by the relative disposition of the CDRs, and by the nature and disposition of the side chains of the residues including the CDRs. A large decrease in immunogenicity can be achieved by grafting only the CDRs of a non-human, e.g. murine, antibodies ("donor" antibodies) onto human framework ("acceptor framework") and constant regions (see Jones et al (1986) Nature 321, 522-525 and Verhoeyen M et al (1988) Science 239, 1534-1536). However, CDR grafting per se may not result in the complete retention of antigen-binding properties and it is frequently found that some framework residues (sometimes referred to as "back mutations") of the donor antibody need to be preserved in the humanised molecule if significant antigen-binding affinity is to be recovered (see Queen C et al (1989) PNAS 86, 10,029-10,033, Co, M et al (1991) Nature 351, 501-502). In this case, human variable domains showing the greatest sequence homology to the non-human donor antibody are chosen from a database in order to provide the human framework (FR). The selection of human FRs can be made either from human consensus or individual human antibodies. Where necessary key residues from the donor antibody are substituted into the human acceptor framework to preserve CDR conformations, computer modelling of the antibody may be used to help identify such structurally important residues. See WO99/48523, for example.

Alternatively, humanisation maybe achieved by a process of "veneering". A statistical analysis of unique human and murine immunoglobulin heavy and light chain variable domains revealed that the precise patterns of exposed residues are different in human and murine antibodies, and most individual surface positions have a strong preference for a small number of different residues (see Padlan E. A. et al; (1991) Mol. Immunol. 28, 489-498 and Pedersen J. T. et al (1994) J. Mol. Biol. 235; 959-973). Therefore it is possible to reduce the immunogenicity of a non-human Fv by replacing exposed residues in its framework regions that differ from those usually found in human antibodies. Because protein antigenicity may be correlated with surface accessibility, replacement of the surface residues may be sufficient to render the mouse variable domain "invisible" to the human immune system (see also Mark G. E. et al (1994) in Handbook of Experimental Pharmacology vol. 113: The pharmacology of monoclonal Antibodies, Springer-Verlag, pp 105-134). This procedure of humanisation is referred to as "veneering" because only the surface of the antibody is altered, the supporting residues remain undisturbed.

An antibody molecule of the invention may be produced using any known and well-established expression system and recombinant cell culturing technology, for example, by expression in bacterial hosts (prokaryotic systems), or eukaryotic systems such as yeasts, fungi, insect cells or mammalian cells. An antibody molecule of the present invention may be produced in transgenic organisms such as a goat, a plant or a XENOMOUSE transgenic mouse, an engineered mouse strain that has large fragments of the human immunoglobulin loci and is deficient in mouse antibody production. An antibody may also be produced by chemical synthesis.

For recombinant production of an antibody molecule of the invention typically a polynucleotide encoding the antibody is isolated and inserted into a replicable vector such as a plasmid for further cloning (amplification) or expression. An illustrative example of a suitable expression system is a glutamate synthetase system (such as sold by Lonza Biologics), with the host cell being for instance CHO or NS0. A polynucleotide encoding the antibody is readily isolated and sequenced using conventional procedures. Vectors that may be used include plasmid, virus, phage, transposons, minichromsomes of which plasmids are a typical embodiment. Generally such vectors further include a signal sequence, origin of replication, one or more marker genes, an enhancer element, a promoter and transcription termination sequences operably linked to the light and/or heavy chain polynucleotide so as to facilitate expression. Polynucleotides encoding the light and heavy chains may be inserted into separate vectors and transfected into the same host cell or, if desired both the heavy chain and light chain can be inserted into the same vector for transfection into the host cell. Both chains can, for example, be arranged, under the control of a dicistronic operon and expressed to result in the functional and correctly folded antibody molecule as described in Skerra, A. (1994) Use of the tetracycline promoter for the tightly regulated production of a murine antibody fragment in Escherichia coli, Gene 151, 131-135, or Skerra, A. (1994) A general vector, pASK84, for cloning, bacterial production, and single-step purification of antibody Fab fragments, Gene 141, 79-8. Thus according to one aspect of the present invention there is provided a process of constructing a vector encoding the light and/or heavy chains of an antibody or antigen binding fragment thereof of the invention, which method includes inserting into a vector, a polynucleotide encoding either a light chain and/or heavy chain of an antibody molecule of the invention.

When using recombinant techniques, the antibody molecule can be produced intracellularly, in the periplasmic space, or directly secreted into the medium (cf. also Skerra 1994, supra). If the antibody is produced intracellularly, as a first step, the particulate debris, either host cells or lysed fragments, are removed, for example, by centrifugation or ultrafiltration. Carter et al., Bio/Technology 10: 163-167 (1992) describe a procedure for isolating antibodies which are secreted to the periplasmic space of *E coli.* The antibody can also be produced in any oxidizing environment. Such an oxidizing environment may be provided by the periplasm of Gram-negative bacteria such as E. coli, in the extracellular milieu of Gram-positive bacteria or in the lumen of the endoplasmatic reticulum of eukaryotic cells (including animal cells such as insect or mammalian cells) and usually favors the formation of structural disulfide bonds. It is, however, also possible to produce an antibody molecule of the invention in the cytosol of a host cell such as E. coli. In this case, the polypeptide can either be directly obtained in a soluble and folded state or recovered in form of inclusion bodies, followed by renaturation in vitro. A further option is the use of specific host strains having an oxidizing intracellular milieu, which may thus allow the formation of disulfide bonds in the cytosol (Venturi M, Seifert C, Hunte C. (2002) "High level production of functional antibody Fab fragments in an oxidizing bacterial cytoplasm." J. Mol. Biol. 315, 1-8).

The antibody molecule produced by the cells can be purified using any conventional purification technology, for example, hydroxylapatite chromatography, gel electrophoresis, dialysis, and affinity chromatography, with affinity chromatography being one preferred purification technique. Antibody molecules may be purified via affinity purification with proteins/ligands that specifically and reversibly bind constant domains such as the CH1 or the CL domains. Examples of such proteins are immunoglobulin-binding bacterial proteins such as Protein A, Protein G, Protein A/G or Protein L, wherein Protein L binding is restricted to antibody molecules that contain kappa light chains. An alternative method for purification of antibodies with κ-light chains is the use of bead coupled anti kappa antibodies (KappaSelect). The suitability of protein A as an affinity ligand depends on the species and isotype of any immunoglobulin Fc domain that is present in the antibody. Protein A can be used to purify antibodies (Lindmark et al., J. Immunol. Meth. 62: 1-13 (1983)). Protein G is recommended for all mouse isotypes and for human gamma3 (Guss et al., EMBO J. 5: 15671575 (1986)). The choice of the purification method that is used for a particular antibody molecule of the invention is within the knowledge of the person of average skill in the art.

It is also possible to equip one of the chains of the antibody molecule of the invention with an affinity tag. Affinity tags such as the Strep-tag® or Strep-tag@ II (Schmidt, T.G.M. et al. (1996) J. Mol. Biol. 255, 753-766), the myc-tag, the FLAG™-tag, the His6-tag or the HA-tag allow easy detection and also simple purification of the recombinant antibody molecule.

Thus, a method of producing an antibody molecule of the present invention comprises expressing a nucleic acid encoding the antibody molecule under conditions allowing expression of the nucleic acid, preferably the antibody molecule is expressed in a host cell or a cell-free system.

The terms "mutated", "mutant" and "mutation" in reference to a nucleic acid or a polypeptide refers to the exchange, deletion, or insertion of one or more nucleotides or amino acids, respectively, compared to the naturally occurring nucleic acid or polypeptide, i.e. to a reference sequence that can be taken to define the wild-type.

It is understood in this regard that the term "position", when used in accordance with the present invention, means the position of an amino acid within an amino acid sequence depicted herein. This position may be indicated relative to a resembling native sequence, e.g. a sequence of a naturally occurring IgG domain or chain. The term "corresponding" as used herein also includes that a position is not necessarily, or not only, determined by the number of the preceding nucleotides/amino acids. Thus, the position of a given amino acid in accordance with the present invention which may be substituted may vary due to deletion or addition of amino acids elsewhere in the antibody chain.

Thus, under a "corresponding position" in accordance with the present invention it is to be understood that amino acids may differ in the indicated number but may still have similar neighbouring amino acids. Said amino acids which may be exchanged, deleted or added are also encompassed by the term "corresponding position". In order to determine whether an amino acid residue in a given amino acid sequence corresponds to a certain position in the amino acid sequence of a naturally occurring immunoglobuline domain or chain, the skilled person can use means and methods well-known in the art, e.g., alignments, either manually or by using computer programs such as BLAST2.0, which stands for Basic Local Alignment Search Tool or ClustalW or any other suitable program which is suitable to generate sequence alignments.

In some embodiments a substitution (or replacement) is a conservative substitution. Conservative substitutions are generally the following substitutions, listed according to the amino acid to be mutated, each followed by one or more replacement(s) that can be taken to be conservative: Ala → Gly, Ser, Val; Arg → Lys; Asn → Gln, His; Asp → Glu; Cys → Ser; Gin → Asn; Glu → Asp; Gly → Ala; His → Arg, Asn, Gin; Ile → Leu, Val; Leu → Ile, Val; Lys → Arg, Gin, Glu; Met → Leu, Tyr, Ile; Phe → Met, Leu, Tyr; Ser → Thr; Thr → Ser; Trp → Tyr; Tyr → Trp, Phe; Val → Ile, Leu. Other substitutions are also permissible and can be determined empirically or in accord with other known conservative or non-conservative substitutions. As a further orientation, the following eight groups each contain amino acids that can typically be taken to define conservative substitutions for one another:
1) Alanine (Ala), Glycine (Gly);
2) Aspartic acid (Asp), Glutamic acid (Glu);
3) Asparagine (Asn), Glutamine (Gin);
4) Arginine (Arg), Lysine (Lys);
5) Isoleucine (Ile), Leucine (Leu), Methionine (Met), Valine (Val);
6) Phenylalanine (Phe), Tyrosine (Tyr), Tryptophan (Trp);
7) Serine (Ser), Threonine (Thr); and
8) Cysteine (Cys), Methionine (Met)

If such substitutions result in a change in biological activity, then more substantial changes, such as the following, or as further described below in reference to amino acid classes, may be introduced and the products screened for a desired characteristic. Examples of such more substantial changes are: Ala → Leu, Ile; Arg → Gln; Asn → Asp, Lys, Arg, His; Asp → Asn; Cys → Ala; Gin → Glu; Glu → Gln; His → Lys; Ile → Met, Ala, Phe; Leu → Ala, Met, Norleucine; Lys → Asn; Met → Phe; Phe → Val, Ile, Ala; Trp → Phe; Tyr → Thr, Ser; Val → Met, Phe, Ala.

In some embodiments an antibody molecule according to the invention includes one or more amino acid residues, including two, three, four, five, six, seven, eight, nine, ten, eleven, twelve, thirteen, fourteen, fifteen, sixteen, seventeen or eighteen amino acid residues, that are mutated or lacking to prevent dimerization via cystein residues or to modulate Fc-function. In some of these embodiments one or more amino acid residue(s) of the CH2 domain and/or of the hinge region that is able to mediate binding to Fc receptors are mutated or lacking. If present, the one or more amino acid residue(s) able to mediate binding to Fc receptors may be an amino acid residue that is able to activate antibody dependent cellular cytotoxicity (ADCC) or complement-mediated cytotoxicity (CDC). In some embodiments a respective amino acid residue capable of mediating binding to Fc receptors is substituted by another amino acid, generally when comparing the sequence to the sequence of a corresponding naturally occurring domain in an immunoglobulin, such as an IgG. In some embodiments such an amino acid residue capable of mediating binding to Fc receptors is deleted, generally relative to the sequence of a corresponding naturally occurring domain in an immunoglobulin, such as an IgG. However, in other embodiments of the invention that relate to a bispecific or tri-specific antibody molecule consisting of a Fab fragment, a single VH domain or VL domain and an immunoglobulin CH2 domain, it is within the scope of the invention to introduce mutations in the CH2 domain of human γ1, for example, that optimize antibody dependent cytotoxicity (ADCC). Such mutations are described in the international patent applications WO2011/076922, WO2011/089211 and WO 2013/092001, for example.

In some embodiments at least one amino acid residue of the CH2 domain that is able to mediate binding to Fc receptors is lacking or mutated e.g. substituted or deleted. Such amino acid residue(s) can be an amino acid located at one of the positions 226, 228, 229, 230, 231, 232, 233, 234, 235, 236, 237, 238, 265, 297, 327, and 330. In other embodiments, the at least one amino acid residue of the CH2 domain that is able to mediate binding to Fc receptors and that is lacking or mutated is/ are the amino acid residues selected from the group consisting of sequence position 230, 231, 232, 233, 234, 235, 236, 237, 238, 265, 297, 327, and 330 (numbering of sequence positions according to the EU-index). Further one or more amino acid residues of sequence positions 226, 228 and 229, can also be lacking or mutated.

Again, the numbering of amino acids used corresponds to the sequence positions according to the Kabat numbering [EU-Index]. A corresponding deletion of an amino acid may for example be a deletion of amino acid 228, generally a proline in IgG, a deletion of amino acid 229, generally a cysteine in IgG, a deletion of amino acid 230, generally a proline in IgG, a deletion of amino acid 231, generally an alanine in IgG, a deletion of amino acid 232, generally a proline in IgG, a deletion of amino acid 233, generally a glutamic acid in IgG, a deletion of amino acid 234, generally a leucine in IgG, a deletion of amino acid 235, generally a leucine in IgG, a deletion of amino acid 236, generally a glycine in IgG, a deletion of amino acid 237, generally a glycine in IgG, a deletion of amino acid 238, generally a proline in IgG and a deletion of amino acid 265, generally an aspartic acid in IgG. A corresponding substitution of an amino acid may for example be a substitution of amino acid 226, generally a cysteine in IgG, a substitution of amino acid 228, generally a proline in IgG, a substitution of amino acid 229, generally a cysteine in IgG, a substitution of amino acid 230, generally a proline in IgG, a substitution of amino acid 231, generally an alanine in IgG, a substitution of amino acid 232, generally a proline in IgG, a substitution of amino acid 233, generally a glutamic acid in IgG, a substitution of amino acid 234, generally a leucine in IgG, a substitution of amino acid 235, generally a leucine in IgG, a substitution of amino acid 265, generally an aspartic acid in IgG, a substitution of amino acid 297, generally an asparagine in IgG, a substitution of amino acid 327, generally an alanine in IgG, and a substitution of amino acid 330, generally an alanine in IgG. A respective substitution may be one of substitution Cys226→Ser, substitution Cys229→Ser, substitution Glu233→Pro, substitution Leu234→Val, substitution Leu235→Ala, substitution Asp265→Gly, substitution Asn297→Gln, substitution Ala327→Gln, substitution Ala327→Gly, and substitution Ala330→Ser. As can be taken from the above, in some embodiments one or two of the cysteine residues at positions 226 and 229 in the hinge region are being substituted for another amino acid, for instance substituted for a serine residue. Thereby the formation of a disulphide bond with another main chain can be prevented. Further, and as also explained below, deleting and/ or substituting (mutating) selected amino acid residues in the CH2 domain that is able to mediate binding to Fc-receptors can cause an antibody molecule of the invention to have less or no activity in terms of antibody-dependent cell-mediated cytotoxicity and fixation of complement.

Another type of amino acid variant of an antibody alters the original glycosylation pattern (if any) of the antibody molecule. By altering is meant deleting one or more carbohydrate moieties found in the antibody, and/or adding one or more glycosylation sites that are not present in the antibody. Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

In the context of the present invention in some embodiments the portion of the main chain of the antibody molecule of the invention, which represents the Fc region of an immunoglobulin, is typically inert, or at least essentially of low influence, with regard to binding to Fc receptors. As said, this is achieved by deleting and/or substituting (mutating) at least one of selected amino acid residues in the CH2 domain that are able to mediate binding to an Fc-receptor. Such molecules are also referred to herein as "Fc-attenuated" antibody molecules or "Fc^{ko}" antibody molecules. Fc-knockout variants are desirable because binding of the Fabv-molecules to FcR expressing cells and formation of a functional CD3 binding site on the surface of these cells should be prevented. The portion of an antibody chain according to the invention that can be taken to represent a portion of an Fc fragment, i.e. the CH2 domain, thus might define a "scaffold" without providing a particular biological function such as an effector function, for example. However, it has been found in the present invention, that this scaffold may provide significant advantages in terms of purification, production efficiency and/or stability of the antibody molecules of the invention compared to known antibody molecules (cf. the Examples).

In some embodiments the recognition, and accordingly binding, of this Fc-corresponding portion to a given Fc receptor is of about 2-fold, about 5-fold, about 8-fold, about 10-fold, about 12-fold, about 15-fold, about 20-fold or lower than the Fc region of a naturally occurring immunoglobulin. In some embodiments this Fc-corresponding portion is entirely void of its ability of binding to Fc receptors. The binding of an antibody to Fc receptors, including determining a dissociation constant, can easily be determined by the skilled artisan using standard techniques such as surface plasmon resonance, e.g. using a Biacore™ measurement. Any other method of measuring biomolecular binding may likewise be used, which may for instance rely on spectroscopical, photochemical, photometric or radiological means. Examples for the corresponding detection methods are fluorescence correlation spectroscopy, photochemical cross-linking and the use of photoactive or radioactive labels respectively. Some of these methods may include additional separation techniques such as electrophoresis or HPLC.

Where required, a substitution or deletion of amino acid residues, as explained above, may be carried out to this effect. Suitable mutations can be taken from Armour et al. (Eur. J. Immunol. [1999] 29, 2613-2624), for example. Further suitable positions for mutations to a sequence of an antibody chain can be taken from the crystal structure data published on the complex between FcγRIII and the human IgG1 Fc fragment (Sondermann et al., Nature [2000] 406, 267-273). In addition to measuring the binding affinity as described above in order to assess the level of "Fc attenuation" or loss of binding affinity, it is also possible to functionally assess the (lack of the) ability to mediate binding to an Fc-receptor. In the case of antibody molecules which bind CD3 as one target, it is for example possible to assess the binding through the mitogenity of such CD3 binding antibody molecules on cells. The mitogenity is mediated by binding of CD3 antibodies to the Fc-receptors on accessory cells, such as monocytes. If an antibody molecule of the invention that has one binding site for CD3 does not show any mitogenic effect whereas the parent monoclonal anti-CD3 antibody that has a functional Fc part induces strong mitosis in T cells, it is clear that, due to the lack of mitosis, the antibody molecule of the invention lacks the ability for Fc binding and can thus be considered as a "Fc knock-out" molecule. Illustrative examples of a method of assessing anti-CD3 mediated mitogenity have been described by Davis, Vida & Lipsky (J.Immunol (1986) 137, 3758), and by Ceuppens, JL, & van Vaeck, F, (see J.Immunol. (1987) 139, 4067, or Cell. Immunol. (1989) 118, 136). Further illustrative suitable examples of an assay for assessing mitogenity of an antibody have been described by Rosenthal-Allieri et al. (Rosenthal-Allieri MA, Ticcioni M, Deckert M, Breittmeyer JP, Rochet N, Rouleaux M, and Senik A, Bernerd A, Cell Immunol. 1995 163(1):88-95) and Grosse-Hovest et al. (Grosse-Hovest L, Hartlapp I, Marwan W, Brem G, Rammensee H-G, and Jung G, Eur J Immunol. [2003] May;33(5):1334-1340). In addition, the lack of Fc binding can be assessed by the ability of an antibody molecule of the invention to mediate one or more of the well-known effector functions of the Fc part.

As noted above, substitutions or deletions of cysteine residues may be carried out in order to introduce or to remove one or more disulphide bonds, including removing a potential or a previously existing disulphide bond. Thereby linkage between a main chain and a chain of lower weight/shorter length of an antibody molecule according to the invention may be controlled including established, strengthened or abolished. By removing one or more cysteine residues a disulphide bridge may be removed. One such disulphide bond is typically defined by a cysteine in the main chain of a first antibody molecule and a cysteine in the hinge region of a second antibody molecule. In this regard, in some embodiments an antibody according to the invention may include an amino acid substitution of a native cysteine residue at positions 226 and/or 229, relative to the sequence of a human IgG immunoglobulin according to the Kabat numbering [EU-Index], by another amino acid residue.

Substitutions or deletions of amino acid residues such as arginine, asparagine, serine, threonine or tyrosine residues may also be carried out to modify the glycosylation pattern of an antibody. As an illustrative example, an IgG molecule has a single N-linked biantennary carbohydrate at Asn297 of the CH2 domain. For IgG from either serum or produced ex vivo in hybridomas or engineered cells, the IgG are heterogeneous with respect to the Asn297 linked carbohydrate. For human IgG, the core oligosaccharide typically consists of GlcNAc₂Man₃GlcNAc, with differing numbers of outer residues.

As indicated, besides binding of antigens/epitopes, an immunoglobulin is known to have further "effector functions", biological activities attributable to the Fc region (a native sequence Fc region or amino acid sequence variant Fc region) of an immunoglobulin, and vary with the immunoglobulin isotype. Examples of antibody effector functions include: Clq binding and complement dependent cytotoxicity (CDC); Fc receptor binding; antibody-dependent cell-mediated cytotoxicity (ADCC); phagocytosis; down regulation of cell surface receptors (e.g., B cell receptors); and B cell activation. Exerting effector functions of an antibody generally involves recruiting effector cells. Several immunoglobulin effector functions are mediated by Fc receptors (FcRs), which bind the Fc region of an antibody. FcRs are defined by their specificity for immunoglobulin isotypes; Fc receptors for IgG antibodies are referred to as FcγR, for IgE as FcεR, for IgA as FcαR and so on. Any of these effector functions (or the loss of such effector functions) such a CDC or ADCC can be used in order to evaluate whether an antibody molecule of the invention lacks the ability of Fc binding.

In this context, it is noted that the term "Fc receptor" or "FcR" defines a receptor, generally a protein that is capable of binding to the Fc region of an antibody. Fc receptors are found on the surface of certain cells of the immune system of an organism, for example natural killer cells, macrophages, neutrophils, and mast cells. *In vivo* Fc receptors bind to immunoglobulins that are immobilized on infected cells or present on invading pathogens. Their activity stimulates phagocytic or cytotoxic cells to destroy microbes, or infected cells by antibody-mediated phagocytosis or antibody-dependent cell-mediated cytotoxicity. Some viruses such as flaviviruses use Fc receptors to help them infect cells, by a mechanism known as antibody-dependent enhancement of infection. FcRs have been reviewed in Ravetch and Kinet, Annu. Rev. Immunol. 9: 457-92 (1991); Capel et al., Immunomethods 4: 25-34 (1994); and de Haas et al., J. Lab. Clin. Med. 126: 330-41 (1995).

"Complement dependent cytotoxicity" or "CDC" refers to the lysis of a target cell in the presence of complement. Activation of the classical complement pathway is initiated by the binding of the first component of the complement system (Clq) to antibodies (of the appropriate subclass) which are bound to their cognate antigen. To assess complement activation, a CDC assay, e.g., as described in Gazzano-Santoro et al., J. Immunol. Methods 202: 163 (1997) may be performed.

The term "complement system" is used in the art to refer a number of small proteins - called complement factors - found in blood, generally circulating as inactive precursors (pro-proteins). The term refers to the ability of this inalterable and not adaptable system to "complement" the capability of antibodies and phagocytic cells to clear pathogens such as bacteria, as well as antigen-antibody complexes, from an organism. An example of complement factors is the complex C1, which includes C1q and two serine protases, C1r and C1s. The complex C1 is a component of the CDC pathway. C1q is a hexavalent molecule with a molecular weight of approximately 460,000 and a structure likened to a bouquet of tulips in which six collagenous "stalks" are connected to six globular head regions. To activate the complement cascade, C1q has to bind to at least two molecules of IgG1, IgG2 or IgG3.

"Antibody-dependent cell-mediated cytotoxicity" or ADCC refers to a form of cytotoxicity in which secreted Ig bound onto Fc receptors (FcRs) present on certain cytotoxic cells - such as natural killer (NK) cells, neutrophils and macrophages - enable these cytotoxic effector cells to bind specifically to an antigen-bearing target cell and subsequently kill the target cell with cytotoxins. The antibodies "arm" the cytotoxic cells and are required for killing of the target cell by this mechanism. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9: 457-92 (1991). To assess ADCC activity of a molecule of interest, an *in vitro* ADCC assay, such as described in US Patent Nos. 5,500,362 or 5,821,337 may be carried out. Useful effector cells for such assays include, but are not limited to, peripheral blood mononuclear cells (PBMC) and natural killer (NK) cells. In some embodiments ADCC activity of the molecule of interest may be assessed *in vivo,* e.g., in an animal model such as disclosed in Clynes et al., PNAS USA 95: 652-656 (1998).

Several antibody effector functions are mediated by Fc receptors (FcRs), which bind the Fc region of an antibody. FcRs are defined by their specificity for immunoglobulin isotypes; Fc receptors for IgG antibodies are referred to as FcγR, for IgE as FcεR, for IgA as FcαR and so on. Three subclasses of FcγR have been identified: FcγRI (CD64), FcγRII (CD32) and FcγRIII (CD16).

Turning now to nucleic acids of the invention, a nucleic acid molecule encoding one or more chains of an antibody according to the invention may be any nucleic acid in any possible configuration, such as single stranded, double stranded or a combination thereof. Nucleic acids include for instance DNA molecules, RNA molecules, analogues of the DNA or RNA generated using nucleotide analogues or using nucleic acid chemistry, locked nucleic acid molecules (LNA), and protein nucleic acids molecules (PNA). DNA or RNA may be of genomic or synthetic origin and may be single or double stranded. Such nucleic acid can be e.g. mRNA, cRNA, synthetic RNA, genomic DNA, cDNA synthetic DNA, a copolymer of DNA and RNA, oligonucleotides, etc. A respective nucleic acid may furthermore contain non-natural nucleotide analogues and/or be linked to an affinity tag or a label.

In some embodiments a nucleic acid sequence encoding a chain, such as a main chain and/or a smaller chain of an antibody according to the invention is included in a vector such as a plasmid. Where a substitution or deletion is to be included in an antibody chain, when compared to a naturally occurring domain or region of an antibody, the coding sequence of the respective native domain/region, e.g. included in the sequence of an immunoglobulin, can be used as a starting point for the mutagenesis. For the mutagenesis of selected amino acid positions, the person skilled in the art has at his disposal the various established standard methods for site-directed mutagenesis. A commonly used technique is the introduction of mutations by means of PCR (polymerase chain reaction) using mixtures of synthetic oligonucleotides, which bear a degenerate base composition at the desired sequence positions. For example, use of the codon NNK or NNS (wherein N = adenine, guanine or cytosine or thymine; K = guanine or thymine; S = adenine or cytosine) allows incorporation of all 20 amino acids plus the amber stop codon during mutagenesis, whereas the codon VVS limits the number of possibly incorporated amino acids to 12, since it excludes the amino acids Cys, Ile, Leu, Met, Phe, Trp, Tyr, Val from being incorporated into the selected position of the polypeptide sequence; use of the codon NMS (wherein M = adenine or cytosine), for example, restricts the number of possible amino acids to 11 at a selected sequence position since it excludes the amino acids Arg, Cys, Gly, Ile, Leu, Met, Phe, Trp, Val from being incorporated at a selected sequence position. In this respect it is noted that codons for other amino acids (than the regular 20 naturally occurring amino acids) such as selenocystein or pyrrolysine can also be incorporated into a nucleic acid of a antibody molecule. It is also possible, as described by Wang, L., et al. (2001) Science 292, 498-500, or Wang, L., and Schultz, P.G. (2002) Chem. Comm. 1, 1-11, to use "artificial" codons such as UAG which are usually recognized as stop codons in order to insert other unusual amino acids, for example o-methyl-L-tyrosine or p-aminophenylalanine.

The use of nucleotide building blocks with reduced base pair specificity, as for example inosine, 8-oxo-2'deoxyguanosine or 6(2-deoxy-β-D-ribofuranosyl)-3,4-dihydro-8H-pyrimin-do-1,2-oxazine-7-one (Zaccolo et al. (1996) J. Mol. Biol. 255, 589-603), is another option for the introduction of mutations into a chosen sequence segment. A further possibility is the so-called triplet-mutagenesis. This method uses mixtures of different nucleotide triplets, each of which codes for one amino acid, for incorporation into the coding sequence (Virnekäs B, et al., 1994 Nucleic Acids Res 22, 5600-5607).

A nucleic acid molecule encoding a chain, such as a main chain and/or a smaller chain of an antibody according to the invention can be expressed using any suitable expression system, for example in a suitable host cell or in a cell-free system. The obtained antibody molecule is enriched by means of selection and/ or isolation. Thus, in one embodiment, the nucleic acid molecule of the present invention can be comprised in a vector. Similarly, the nucleic acid molecule of the present invention may be comprised in a host cell or the vector comprising the nucleic acid molecule of the present invention may be comprised in a host cell.

As explained above, an antibody molecule according to the invention may be directed against any desired target epitopes/antigens. Depending on the selected epitopes/antigens the antibody may be suitable in the treatment or prevention of disease. Accordingly, in some embodiments an antibody according to the invention may be used in a method of treating and/or preventing a medical condition such as a disorder or disease. Similarly, the antibody molecules of the present invention as well as the hetero-dimeric antibody molecules can be used in the treatment of a disease. In embodiments where one of the antibodies incorporated in a bispecific or tri-specific molecule is capable of activating immune cells in an FcR-dependent manner it may be particularly useful to select an antibody molecule that has an Fc-corresponding portion that shows reduced binding to Fc-receptors. By this means an undesired immune activation mediated by FcR binding is prevented. In some embodiments a disease to be treated or prevented may be a proliferatory disease. Examples of a proliferative disease include, but are not limited to, hemopoetic malignancies, such as acute and chronic myeloic and lymphatic leukemias, as well as lymphomas, or solid tumors. Examples of solid tumors include, but are not limited to, tumors of the gastrointestinal tract, bone, lung, kidney, prostate, breast, brain, ovary, uterus, testis, mesenchymal tumors and skin, such as melanoma.

The present invention further relates to a use of an antibody molecule of the present invention for the treatment of a disease, wherein the antibody molecule forms a hetero-dimer only in vivo on a target cell, thereby reducing "off target activation".

"Off target activation" could be any activiation of cells, which is not due to the cells to be targeted by the used antibody molecules. For example, an off target activation could be a target cell independent T cell activation, which even may become exaggerated in the presence of endothelial cells. Also encompassed is the so-called cytokine storm. This is an immune reaction consisting of a positive feedback loop between cytokines and immune cells, with highly elevated levels of various cytokines. Thus, in one embodiment, the antibody molecule provides for target cell restricted T cell-activation. In another embodiment, the disease to be treated is a proliferatory disease.

The invention also provides a pharmaceutical composition that includes an antibody molecule of the invention and, optionally a pharmaceutically acceptable excipient.

The antibody molecule according to the invention can be administered via any parenteral or non-parenteral (enteral) route that is therapeutically effective for proteinaceous drugs. Parenteral application methods include, for example, intracutaneous, subcutaneous, intramuscular, intratracheal, intranasal, intravitreal or intravenous injection and infusion techniques, e.g. in the form of injection solutions, infusion solutions or tinctures, as well as aerosol installation and inhalation, e.g. in the form of aerosol mixtures, sprays or powders. An overview about pulmonary drug delivery, i.e. either via inhalation of aerosols (which can also be used in intranasal administration) or intracheal instillation is given by J.S. Patton et al. The lungs as a portal of entry for systemic drug delivery. Proc. Amer. Thoracic Soc. 2004 Vol. 1 pages 338-344, for example). Non-parenteral delivery modes are, for instance, orally, e.g. in the form of pills, tablets, capsules, solutions or suspensions, or rectally, e.g. in the form of suppositories. Antibody molecules of the invention can be administered systemically or topically in formulations containing conventional non-toxic pharmaceutically acceptable excipients or carriers, additives and vehicles as desired.

In one embodiment of the present invention the pharmaceutical is administered parenterally to a mammal, and in particular to humans. Corresponding administration methods include, but are not limited to, for example, intracutaneous, subcutaneous, intramuscular, intratracheal or intravenous injection and infusion techniques, e.g. in the form of injection solutions, infusion solutions or tinctures as well as aerosol installation and inhalation, e.g. in the form of aerosol mixtures, sprays or powders. A combination of intravenous and subcutaneous infusion and /or injection might be most convenient in case of compounds with a relatively short serum half life. The pharmaceutical composition may be an aqueous solution, an oil-in water emulsion or a water-in-oil emulsion.

In this regard it is noted that transdermal delivery technologies, e.g. iontophoresis, sonophoresis or microneedle-enhanced delivery, as described in Meidan VM and Michniak BB 2004 Am. J. Ther. 11 (4): 312-316, can also be used for transdermal delivery of an antibody molecule described herein. Non-parenteral delivery modes are, for instance, oral, e.g. in the form of pills, tablets, capsules, solutions or suspensions, or rectal administration, e.g. in the form of suppositories. The antibody molecules of the invention can be administered systemically or topically in formulations containing a variety of conventional non-toxic pharmaceutically acceptable excipients or carriers, additives, and vehicles.

The dosage of the antibody molecule applied may vary within wide limits to achieve the desired preventive effect or therapeutic response. It will, for instance, depend on the affinity of the antibody molecule for a chosen target as well as on the half-life of the complex between the antibody molecule and the ligand in vivo. Further, the optimal dosage will depend on the biodistribution of the antibody molecule or a conjugate thereof, the mode of administration, the severity of the disease/disorder being treated as well as the medical condition of the patient. For example, when used in an ointment for topical applications, a high concentration of the antibody molecule can be used. However, if wanted, the antibody molecule may also be given in a sustained release formulation, for example liposomal dispersions or hydrogel-based polymer microspheres, like PolyActiveTM or OctoDEXTM (cf. Bos et al., Business Briefing: Pharmatech 2003: 1-6). Other sustained release formulations available are for example PLGA based polymers (PR pharmaceuticals), PLA-PEG based hydrogels (Medincell) and PEA based polymers (Medivas).

Accordingly, the antibody molecules of the present invention can be formulated into compositions using pharmaceutically acceptable ingredients as well as established methods of preparation (Gennaro, A.L. and Gennaro, A.R. (2000) Remington: The Science and Practice of Pharmacy, 20th Ed., Lippincott Williams & Wilkins, Philadelphia, PA). To prepare the pharmaceutical compositions, pharmaceutically inert inorganic or organic excipients can be used. To prepare e.g. pills, powders, gelatine capsules or suppositories, for example, lactose, talc, stearic acid and its salts, fats, waxes, solid or liquid polyols, natural and hardened oils can be used. Suitable excipients for the production of solutions, suspensions, emulsions, aerosol mixtures or powders for reconstitution into solutions or aerosol mixtures prior to use include water, alcohols, glycerol, polyols, and suitable mixtures thereof as well as vegetable oils.

The pharmaceutical composition may also contain additives, such as, for example, fillers, binders, wetting agents, glidants, stabilizers, preservatives, emulsifiers, and furthermore solvents or solubilizers or agents for achieving a depot effect. The latter is that fusion proteins may be incorporated into slow or sustained release or targeted delivery systems, such as liposomes and microcapsules.

The formulations can be sterilized by numerous means, including filtration through a bacteria-retaining filter, or by incorporating sterilizing agents in the form of sterile solid compositions which can be dissolved or dispersed in sterile water or other sterile medium just prior to use.

Numerous possible applications for the inventive antibody molecule exist in medicine. In addition to their use in in vitro diagnostics or drug delivery, an antibody molecule of the invention, which binds, for example, tissue- or tumor-specific cellular surface molecules can be generated.

The present invention is further characterized by the following items:
1. A recombinant antibody molecule consisting of a Fab fragment comprising a first binding site for a first antigen, a variable domain of either the light chain or the heavy chain of a second binding site for a second antigen and an immunoglobulin CH2 domain, wherein the Fab fragment and the variable domain are linked via the CH2 domain, wherein in the immunoglobulin CH2 domain at least one cysteine residue that is able to form a disulfide bridge for dimerisation is lacking or mutated.
2. The antibody molecule of item 1, wherein the at least one cysteine residue is selected from the sequence positions 226, 228 and 229 of the CH2 domain, wherein preferably a cysteine at one or both of positions 226 and 229 is replaced by a different amino acid.
3. The antibody molecule of item 1 or 2, comprising the light chain of the variable domain of the second binding site for the second antigen.
4. The antibody molecule of any of items 1 or 2, comprising the heavy chain of the variable domain of the second binding site for the second antigen.
5. The antibody molecule of any of items 1 to 4, wherein either the first binding site or the second binding site binds a tumor associated antigen.
6. The antibody molecule of item 5, wherein the tumor associated antigen is located on the vasculature of a tumor.
7. The antibody molecule of item 5 or 6, wherein the tumor associated antigen is a surface antigen or an antigen of the extracellular matrix.
8. The antibody molecule of any one of items 5 to 7, wherein the tumor associated antigen is selected from the group consisting of CD10, CD19, CD20, CD21, CD22, CD25, CD30, CD33, CD34, CD37, CD44v6, CD45, CDw52, Fms-like tyrosine kinase 3 (FLT-3, CD135), c-Kit (CD117), CSF1 R, (CD115), CD133, PDGFR-α (CD140a), PDGFR-β (CD140b), chondroitin sulfate proteoglycan 4 (CSPG4, melanoma-associated chondroitin sulfate proteoglycan), Muc-1, EGFR, de2-7-EGFR, EGFRvIII, Folate binding protein, Her2neu, Her3, PSMA, PSCA, PSA, TAG-72, HLA-DR, IGFR, CD133, IL3R, fibroblast activating protein (FAP), Carboanhydrase IX (MN/CA IX), Carcinoembryonic antigen (CEA), EpCAM, CDCP1, Derlin1, Tenascin, frizzled 1-10, the vascular antigens VEGFR2 (KDR/FLK1), VEGFR3 (FLT4, CD309), Endoglin, CLEC14, Tem1-8, and Tie2.
9. The antibody molecule of any of items 1 to 8, wherein either the first binding site or the second binding site binds a T-cell- or NK (natural killer) cell specific receptor molecule.
10. The antibody molecule of item 9, wherein the second binding site binds a T-cell- or NK (natural killer) cell specific receptor molecule.
11. The antibody molecule of item 9 or item 10, wherein the T-cell- or NK cell specific receptor molecule is one of CD3, the T cell receptor (TCR), CD28, CD16, NKG2D, Ox40, 4-1 BB, CD2, CD5 and CD95.
12. The antibody molecule of item 11, wherein the TCR is TCR (alpha/beta) or TCR (gamma/delta).
13. The antibody molecule of any of the preceding items, wherein the Fab fragment is linked to the CH2 domain via the heavy chain CH1 and VH domains of the Fab fragment or via the CL and VL light chain domains of the Fab fragment.
14. The antibody molecule of item 13, wherein the heavy chain domains of the Fab fragment or the light chain domains of the Fab fragment are arranged at the N-terminus of the polypeptide chain.
15. The antibody molecule of any of items 1 to 12, wherein the Fab fragment that comprises the first binding site for the first antigen consists of the VL domain fused to the CH1 domain and the VH domain fused to the CL domain.
16. The antibody molecule of item 15, wherein the CH1 domain of the Fab fragment is fused to the CH2 domain.
17. The antibody molecule of item 15 or item 16, wherein the VL-CH1 chain of the Fab fragment is arranged at the N-terminus of the polypeptide chain.
18. The antibody molecule of any of the preceding items, wherein the Fab fragment comprises a hinge region.
19. The antibody molecule of any of the preceding items, wherein the first binding site binds a tumor associated surface antigen and the second binding site binds one of CD3, the T cell receptor (TCR), CD28, CD16, NKG2D, Ox40, 4-1 BB, CD2, CD5 and CD95.
20. The antibody molecule of any of the preceding items, wherein the light chain of the variable domain of the second binding site has a sequence identity of at least 80%, or at least 85 %, or at least 90 %, or at least 95 %, or at least 98 %, or at least 99 % or 100 % to the variable domain of the light chain of UCHT-1 as shown to SEQ ID NO. 3.
21. The antibody molecule of any of the preceding items, wherein the heavy chain of the variable domain of the second binding site has a sequence identity of at least 80% or at least 85 %, or at least 90 %, or at least 95 %, or at least 98 %, or at least 99 % or 100 % to the variable domain of the heavy chain of UCHT-1 as shown to SEQ ID NO. 4.
22. The antibody molecule of any of the preceding items, wherein at least one amino acid residue of the CH2 domain that is able to mediate binding to Fc receptors is lacking or mutated.
23. The antibody molecule of item 22, wherein the amino acid residues are selected from the group consisting of sequence position 230, 231, 232, 233, 234, 235, 236, 237, 238, 265, 297, 327, and 330 (numbering of sequence positions according to the EU-index).
24. The antibody molecule of item 22 or 23, comprising at least one mutation selected from the group consisting of a deletion of amino acid 228, a deletion of amino acid 229, a deletion of amino acid 230, a deletion of amino acid 231, a deletion of amino acid 232, a deletion of amino acid 233, a substitution Glu233→Pro, a substitution Leu234→Val, a deletion of amino acid 234, a substitution Leu235→Ala, a deletion of amino acid 235, a deletion of amino acid 236, a deletion of amino acid 237, a deletion of amino acid 238, a substitution Asp265→Gly, a substitution Asn297→Gln, a substitution Ala327→Gln, and a substitution Ala330→Ser.
25. A bispecific heterodimeric antibody molecule comprising a hetero-dimer of recombinant antibody molecules (monomers),
   wherein the first monomer of the hetero-dimer consists of a Fab fragment comprising a first binding site for a first antigen, a variable domain of the light chain of a second binding site for a second antigen and an immunoglobulin CH2 domain, wherein the Fab fragment and the variable domain of the light chain are linked via the CH2 domain, and
   wherein the second monomer of the hetero-dimer consists of a Fab fragment comprising a first binding site for the first antigen, a variable domain of the heavy chain of the second binding site for the second antigen and an immunoglobulin CH2 domain, wherein the Fab fragment and the variable domain of the heavy chain are linked via the CH2 domain,
   wherein the variable domain of the light chain of the second binding site of the first monomer and the variable domain of the heavy chain of the second binding site of the second monomer associate thereby forming, the second binding site and dimerizing the hetero-dimer, and
   wherein in at least one of the immunoglobulin CH2 domain of the first monomer or the second monomer at least one cysteine residue that is able to form a disulfide bridge for dimerisation is lacking or mutated, thereby preventing that a CH2 mediated disulfide bridge is formed between the two monomers.
26. A tri-specific heterodimeric antibody molecule comprising a hetero-dimer of recombinant antibody molecules (monomers),
   wherein the first monomer of the hetero-dimer consists of a Fab fragment comprising a first binding site for a first antigen, a variable domain of the light chain of a second binding site for a second antigen and an immunoglobulin CH2 domain, wherein the Fab fragment and the variable domain of the light chain are linked via the CH2 domain, and
   wherein the second monomer of the hetero-dimer consists of a Fab fragment comprising a third binding site for a third antigen, wherein the third antigen is different from the first antigen, a variable domain of the heavy chain of the second binding site for the second antigen and an immunoglobulin CH2 domain, wherein the Fab fragment and the variable domain of the heavy chain are linked via the CH2 domain,
   wherein the variable domain of the light chain of the second binding site of the first monomer and the variable domain of the heavy chain of the second binding site of the second monomer associate thereby forming, the second binding site and dimerizing the hetero-dimer, and
   wherein in at least one of the immunoglobulin CH2 domain of the first monomer or the second monomer at least one cysteine residue that is able to form a disulfide bridge for dimerisation is lacking or mutated, thereby preventing that a CH2 mediated disulfide bridge is formed between the two monomers.
27. The antibody molecule of item 25 or 26, wherein in at least one of the immunoglobulin CH2 domain of the first monomer or the second monomer the at least one cysteine residue is selected from the sequence positions 226, 228 and 229 of the CH2 domain is lacking or mutated.
28. The antibody molecule of item 27, wherein in at least one of the immunoglobulin CH2 domain of the first monomer or the second monomer the cysteine at one or both of positions 226 and 229 (numbering of sequence positions according to the EU-index) is lacking or replaced by a different amino acid.
29. The antibody molecule of any of items 25-28, wherein in at least one of the immunoglobulin CH2 domain of the first monomer or the second monomer an amino acid residue of the CH2 domain that is able to mediate binding to an Fc-receptor is lacking or mutated.
30. The antibody molecule of any of items 25 to 29, wherein in at least one of the immunoglobulin CH2 domain of the first monomer or the second monomer the at least one amino acid residue of the CH2 domain that is able to mediate binding to an Fc-receptor is lacking of mutated is selected from the group consisting of sequence positions 228, 230, 231, 232, 233, 234, 235, 236, 237, 238, 265, 297, 327 and 330 (numbering of sequence positions according to the EU-index).
31. The antibody molecule of item 30, comprising at least one mutation selected from the group consisting of a deletion of amino acid 228, a deletion of amino acid 230, a deletion of amino acid 231, a deletion of amino acid 232, a deletion of amino acid 233, a substitution Glu233→Pro, a deletion of amino acid 234, a substitution of amino acid Leu234→Val, a deletion of amino acid 235, a substitution Leu235→Ala, a deletion of amino acid 236, a deletion of amino acid 237, a deletion of amino acid 238, a substitution Asp265→Gly, a substitution Asn297→Gln, a substitution Ala327→Gln, and a substitution Ala330→Ser.
32. The antibody molecule of any one of items 25 to 31, wherein the first and/or the third binding site binds a tumor associated surface antigen.
33. The antibody molecule of item 32, wherein the tumor associated surface antigen is selected from the group consisting of CD10, CD19, CD20, CD21, CD22, CD25, CD30, CD33, CD34, CD37, CD44v6, CD45, CDw52, Fms-like tyrosine kinase 3 (FLT-3, CD135), c-Kit (CD117), CSF1 R (CD115), CD133, PDGFR-α (CD140a), PDGFR-β (CD140b), chondroitin sulfate proteoglycan 4 (CSPG4, melanoma-associated chondroitin sulfate proteoglycan), Muc-1, EGFR, de2-7-EGFR, EGFRvIII, Folate binding protein, Her2neu, Her3, PSMA, PSCA, PSA, TAG-72, HLA-DR, IGFR, CD133, IL3R, fibroblast activating protein (FAP), Carboanhydrase IX (MN/CA IX), Carcinoembryonic antigen (CEA), EpCAM, CDCP1, Derlin1, Tenascin, frizzled 1-10, the vascular antigens VEGFR2 (KDR/FLK1), VEGFR3 (FLT4, CD309), Endoglin, CLEC14, Tem1-8, and Tie2.
34. The antibody molecule of any one of items 25 to 33, wherein the second binding site binds a T-cell- or NK cell associated receptor molecule.
35. The antibody molecule of item 34, wherein the T-cell- or NK cell associated receptor molecule is one of CD3, the T cell receptor (TCR), CD28, CD16, NKG2D,Ox40, 4-1 BB, CD2, CD5 and CD95.
36. The antibody molecule of item 38, wherein the TCR is TCR (alpha/beta) or TCR (gamma/delta).
37. The antibody molecule of any one of items 25 to 36, wherein the first or the third binding site binds a tumor associated surface antigen or binds to a T-cell- or NK cell associated receptor molecule.
38. A pharmaceutical composition comprising an antibody molecule as defined in any of the preceding items.
39. An antibody molecule as defined in any of items 1 to 37 for use in the treatment or diagnosis of a disease.
40. The antibody molecule of item 39, where the disease is a proliferatory disease.
41. The antibody molecule of item 40, wherein the proliferatory disease is selected from the group consisting of hemopoetic malignancies, such as acute and chronic myeloic and lymphatic leukemias, as well as lymphomas, solid tumors such as tumors of the gastrointestinal tract, lung, kidney, prostate, breast, brain, ovary, uterus, mesenchymal tumors and melanoma.
42. The use of an antibody molecule as defined in any of items 1 to 24for the treatment of a disease, wherein the antibody molecule forms a hetero-dimer only in vivo on a target cell, thereby reducing "off target activation".
43. The use of item 42, wherein the antibody molecule provides for target cell restricted T cell-activation.
44. The use of item 42 or 43, wherein the disease is a proliferatory disease.
45. A nucleic acid molecule encoding an antibody molecule as defined in any of items 1 to 24.
46. A nucleic acid molecule of item 45 comprised in a vector.
47. A host cell comprising a nucleic acid molecule of item 46 or a vector of item 46.
48. A method of producing an antibody molecule of any one of items 1 to 24, comprising expressing a nucleic acid encoding the antibody molecule under conditions allowing expression of the nucleic acid.
49. The method of item 49 wherein the antibody molecule is expressed in a host cell or a cell-free system.

The invention is further illustrated by the following non-limiting Examples.

### EXAMPLE I

Fc-attenuated antibody molecules, also designated to be of the Fabv-^{ko} format, with tumour X CD3 specificity, as schematically depicted in **Fig. E,-E',** were generated. Modifications of amino acids of the hinge region and of the C_{H}2 domain were introduced as shown in the table as depicted in **Fig. 2I.** In particular, the following modifications were introduced in this recombinant Fabv-^{ko} antibody molecule. Each of the cysteine residues at positions 226 and 229 (numbering of sequence positions according to the EU-index) (in the hinge region) of the native sequence at sequence positions 226 to 237 CPPCPAPELLGG (SEQ ID NO: 99, numbering according to the EU-index) was replaced by a serine residue. In addition, the amino acid residues ELLG at positions 233-236 in the CH2 domain were exchanged (the exchanged amino acids were shaded in grey; also compare Fig. 2I) as follows: Glu233 was substituted by Pro, Leu234 was substutited by Val, Leu235 was substituted by Ala, and Gly236 was deleted. Thus, the final sequence obtained between positions 226-237 (numbering of sequence positions according to the EU-index) is equal to SEQ ID NO: 98 (SPPSPAPPVAG in which the Gly is the original Gly residue 237). In addition, replacements at positions 265, 297, 327, 330 (numbering of sequence positions according to the EU-index) were introduced in the Fabv-^{ko} molecule (also compare to Fig. 2I). Namely, at these positions the Fabv^{ko} were modified from the original amino acid D(265), N(297), A(327), A(330) into the following amno acid residues: G(265), Q(297), Q(327), S(330). Generally, the replacements at positions 226 and 229 lead to a loss of cysteins, thereby preventing the formation of disulfide bridges. The amino acid replacements at positions 233-236, 265, 297, 327, 330 lead to an attenuation of Fc function.

Cloning and amplification of plasmids was carried out using *Escherichia coli* DH5α (Invitrogen, Karlsruhe, Germany). The build-up of the respective vectors is depicted in Fig. 7.

Cotransfection of expression vectors encoding main and smaller chains, which can also be referred to as heavy and light chains, of the indicated specificities was done in Sp2/0 plasmocytoma cells, obtained from the American Type Culture Collection (ATCC, Manassas, VA). For the construction of the respective vectors see Fig. 7 and Example II below). Cells were cultured in IMDM media, supplemented with 10% fetal bovine serum (Biochrom AG, Berlin, Germany), 1 % penicillin and streptomycin (Lonza, Basel, Switzerland). Stable transfectants were selected by adding 1 mg/ml G418 (Invitrogen, Karlsruhe, Germany).

Antibody molecules were purified from culture supernatants of stably transfected cells via affinity chromatography using KappaSelect for the Fabv-^{ko} format (chromatography media were obtained from GE Healthcare, Munich, Germany).

### EXAMPLE II

Immunoglobulin V regions were combined with the desired constant C regions in an expression vector. The cloning procedure indicated here allows the introduction of complete Ig V regions and their expression in lymphoid cells without any alterations of their amino acid sequence. To this end, the nucleotide sequence of a VDJ and VJ fragment of a monospecific antibody was used to design primer pairs (C C'; D D'; Table 1). The reamplified DNA fragments of the V segments were digested (VJ directly and VDJ after reamplification with primer pair E E' Table 1) with appropriate restriction nucleases (summarized in Table 1) and then ligated into the expression vectors. Alternatively, the V domains were synthezised as DNA fragments at GeneArt, Regensburg, or at Eurofins, Ebersberg, Germany. This method was used for genes coding for the V regions of the antibody directed to EGFR (clone C225). The vectors (Figure 7) contain human heavy and human light constant region genes. Thus, insertion of the amplified and digested V segments reconstitutes the original genomic organisation of the Ig genes in the vectors without altering any amino acid of the V regions.

The original vector for the heavy chain contains the human γ1 isotype Ig heavy chain (Fig. 7A). Restriction sites were introduced at the required positions in introns in order to exchange the Aatll-Clal fragment with the VDJ fragment of the heavy chain of monoclonal antibodies 4G8 (anti-Flt3), BV10 (anti-FLT3), 4G7 (anti-CD19), C225 (anti-EGFR) and 9.2.27 (anti-CSPG4). The AatII-ClaI fragment can also be exchanged with the VDJ fragment of the heavy chain of any other monoclonal antibody. The region relevant for cloning the VDJ fragment is shown enlarged in Figure 7B. The fragment to be exchanged contains parts of the first intron with an AatII restriction site, the second exon of the leader sequence, the VDJ region and part of the heavy chain intron with the restriction site Clal. For the substitution of all exons of the constant region of the human γ1 heavy chain, restriction sites were introduced at the required position in the heavy chain intron (Mlul) and in the 5'-UTR heavy chain polyA-region (pA-region; Spel), as shown in Figure 7A and7C.

Furthermore, with the expression vectors constructed, it is possible to exchange the entire constant region of the human Igγ1 isotype (Mlul-Spel fragment; see Figure 7A) either against constant regions of all other antibody isotypes or against Fc parts with enhanced or reduced effector function. In the case of antibodies optimized for triggering ADCC (antibody dependent cellular cytotoxicity) amino acid substitutions were introduced in the CH2 domain of human γ1 constant region as shown in International patent applications WO2011/076922 and WO2011/089211. In order to generate bispecific antibody molecules as depicted in Figs. 2A-H, A'-H' Mlul and Spel flanked DNA fragments containing either exons coding for wildtype or modified constant domains of the Ig heavy chain can be inserted. The Mlul-Spel fragment to be exchanged is shown enlarged in Figure 7C.

To add a single variable domain either VH or VL are included via the restriction enzyme sites BspEI and Spel, as also shown in Figure 7A. The region relevant for cloning of a single variable domain fragment VL or VH is shown enlarged in Figure 7C. Single variable domain fragments (VH or VL) with the specifity for CD3 (clone humanized UCHT1 and murine OKT3), CD28 (clone 9.3), TCRα/β (clone BMA031) were generated by PCR with oligonucleotides F and F' listed in Table 2. Alternatively, they were synthesized as DNA-fragments at Eurofins Genomics, Ebersberg, Germany. The DNA fragment of the single variable domain segment VH and VL, respectively, was digested with the appropriate restriction nucleases (summarized in Table 2) and was then ligated into the expression vector.

The original vector for the light chain contains the VJ region of the light chain and the C region of human κ gene (Figure 7D). Restriction sites were introduced at the required locations (Xhol and Spel) in order to substitute the light chain Xhol-Spel fragment with the appropriate VJ fragment of the light chain of monoclonal antibodies 4G8 (anti-FLT3), BV10 (anti-FLT3), 4G7 (anti-CD19), C225 (anti-EGFR) or 9.2.27 (anti-CSPG4) or any other monoclonal antibody. The region adjacent to the fragment to be exchanged is shown in Figure 7E. This region contains parts of the second exon of the leader sequence, a suitable restriction site (Xhol) for in frame fusion, the VJ region and parts of the kappa chain intron with restriction site Spel. In order to replace the constant domain of the light chain (CL) restriction sites were introduced at the required locations (Pmll and BsmBI). The region adjacent to the fragment to be exchanged is shown enlarged in Figure 7F. This region contains parts of the kappa chain intron, a suitable restriction site (Pmll), the CL region and parts of the 3'-UTR region kappa chain polyA-region (pA-region) with restriction site (BsmBI).

**Table 1: Oligonucleotides used for amplification of VDJ and VJ segments for the insertion into expression vectors**

| Oligonucleotides used for the heavy chain VDJ segment | | |
|---|---|---|
| C | 4G7-H-for | 5'-ctc ttc aca ggt gtc ctc tct gag gtc cag ctg cag cag tct gga cct g-3' (SEQ ID NO: 64) |
| C' | 4G7-H-rev | 5'-ggg aga agg tag gac tca cct gag gag act gtg aga gtg gtg cct tgg ccc cag tag tc-3' (SEQ ID NO: 65) |
| C | 9.2.27-H-for | 5'-tct tca cag gtg tcc tct ccc agg tga agc tgc agc aat ctg gac ctg agc-3' (SEQ ID NO: 66) |
| C' | 9.2.27-H-rev | 5'-aat ggg aga agg tag gac tca cct gag gag acg gtg acc gtg gtc cct tgg-3' (SEQ ID NO: 67) |
| C | 4G8-H-for | 5'-tct ctt cac agg tgt cct ctc tca ggt cca act gca gca gcc tgg ggc tga gc-3' (SEQ ID NO: 68) |
| C' | 4G8-H-rev | 5'-gag aag gta gga ctc acc tga gga gac tgt gag agt ggt gcc ttg gcc cca g-3' (SEQ ID NO: 69) |
| C | BV10-H-for | 5'-aga cgt cca ctc tgt ctt tct ctt cac agg tgt cct ctc cca ggt gca gct gaa gca gtc-3'(SEQ ID NO: 70) |
| C' | BV10-H-rev | 5'-gag aag gta gga ctc acc tga gga gac ggt gac tga ggt tcc ttg acc c-3' (SEQ ID NO: 71) |
| E | universal for (AatII) | 5'-aga cgt cca ctc tgt ctt tct ctt cac agg tgt cct ctc c-3' (SEQ ID NO: 72) |
| E' | universal rev (Clal) | 5'-tat cga ttt aga atg gga gaa ggt agg act cac-3' (SEQ ID NO: 73) |

| Oligonucleotides used for the light chain VJ segment | | |
|---|---|---|
| D | 4G7-L-for (Xhol) | 5'-a**ct cga g**ga gat att gtg atg act cag gct gca ccc tct ata c-3' (SEQ ID NO: 74) |
| D' | 4G7-L-rev (Spel) | 5'-a**ac tag t**ac tta cgt ttc agc tcc agc ttg gtc cca gca ccg aac gtg-3' (SEQ ID NO: 75) |
| D | 9.2.27-L-for (Xhol) | 5'-t**ct cga g**ga gac atc gag ctc act cag tct cca gct tct ttg-3' (SEQ ID NO: 76) |
| D' | 9.2.27-L-rev (Spel) | 5'-a**ac tag t**ac tta cgt ttg atc tcc agc ttg gtg ccc cct cca aag g-3' (SEQ ID NO: 77) |
| D | 4G8-L-for (Xhol) | 5'-a**ct cga g**ga gat att gtg cta act cag tct cca gcc acc ctg-3' (SEQ ID NO: 78) |
| D' | 4G8-L-rev (Spel) | 5'-t**ac tag t**ac tta cgt ttt att tcc agc ttg gtc ccc cct cc-3' (SEQ ID NO: 79) |
| D | BV10-L-for (Xhol) | 5'-a**ct cga g**ga gac att gtg atg aca cag tct cca tcc tcc c-3' (SEQ ID NO: 80) |
| D' | BV10-L-rev (Spel) | 5'-**act agt** act tac gtt tca gct cca gct tgg tcc cag cac cga acg tg-3' (SEQ ID NO: 81) |

| | | |
|---|---|---|
| Restriction sites are shown in bold and indicated by letters in parentheses. | | |

**Table 2: Oligonucleotides used for amplification of single variable domain segments for the insertion into expression vectors**

| Oligonucleotides used for the single variable domain segment | | |
|---|---|---|
| F | UCHT1-H-for (BspEI) | 5'- t**tc cgg a**ga ggt tca gct ggt gga gtc tgg cgg tgg cct ggt gca gc-3' (SEQ ID NO: 82) |
| F' | UCHT1-H-rev (Spel) | 5'- t**ac tag t**ta tca cga gga gac ggt gac cag ggt tcc ttg acc cca gac -3'(SEQ ID NO:83) |
| F | UCHT1-L-for (BspEI) | 5'-t**tc cgg** aga tat cca gat gac cca gtc ccc gag ctc cct gtc-3' (SEQ ID NO:84) |
| F' | UCHT1-L-rev (Spel) | 5'- t**ac tag t**ta tca ttt gat ctc cac ctt ggt gcc ctg tcc gaa cgt cca c-3' (SEQ ID NO: 85) |
| F | BMA031-H-for (BspEI) | 5'- t**tc cgg** aga agt gca gct gca gca gtc-3' (SEQ ID NO: 86) |
| F' | BMA031-H-rev Spel | 5'-t**ac ta**g **t**ta tca gct aga cac ggt gac cag agt gc-3'(SEQ ID NO: 87) |
| F | BMA031-L-for (BspEI) | 5'-t**tc cgg a**ca gat cgt gct gac cca gtc-3' (SEQ ID NO:88) |
| F' | BMA031-L-rev (Spel) | 5'-t**ac tag t**ta tca ctt cag ttc cag c-3' (SEQ ID NO: 89) |
| F | OKT3-H-for (BspEI) | 5'-t**tc cgg a**ca ggt gca gct gca gca gt-3' (SEQ ID NO:90) |
| F' | OKT3-H-rev (Spel) | 5't**ac ta**g **t**ta tca tga gga gac ggt gag cgt ggt cc-3' (SEQ ID NO: 91) |
| F | OKT3-L-for (BspEI) | 5'-t**tc cgg a**ga cat tgt gct cac cca g-3' (SEQ ID NO: 92) |
| F' | OKT3-L-rev (Spel) | 5'- t**ac tag t**ta tca gtt tat ttc caa ctt tgt cc-3' (SEQ ID NO: 93) |
| F | 9.3-for (BspEI) | 5'-t**tc cgg a**ca ggt gaa gct gca gca gtc tgg ccc tgg cct ggt gac cc -3' (SEQ ID NO: 94) |
| F' | 9.3-H-rev (Spel) | 5'-t**ac tag t**ta tca aga gct cac agt cac tgt ggt gcc ctg gcc cca gta-3'(SEQ ID NO:95) |
| F | 9.3-L-for (BspEI) | 5'-t**tc cgg a**ga cat tgt gct gac cca gtc ccc tgc ctc cct gg-3'(SEQ ID NO: 96) |
| F' | 9.3-L-rev (Spel) | 5'- t**ac tag t**ta tca cct ctt gat ctc cag ctt ggt gcc ccc tcc aaa ggt g -3' (SEQ ID NO: 97) |

| | | |
|---|---|---|
| Restriction sites are shown in bold and indicated by letters in parentheses. | | |

Thus, exemplary 1½-specific antibody molecules (Fabv-^{ko}) with FLT3xCD3-VH and -VL (4G8xUCHT1-VH, -VL, 4G8xOKT3-VH, -VL BV10xUCHT1-VH, -VL), FLT3xTCRα/β-VH and -VL (4G8xBMA031-VH and -VL, BV10xBMA031-VH and -VL), FLT3xCD28-VH and -VL (4G8x9.3-VH and -VL, BV10x9.3-VH and -VL), FLT3xCD16-VH and -VL (4G8x3G8-VH and -VL, BV10x3G8-VH and -VL), CD19xCD3-VH and -VL (4G7xUCHT1-VH and -VL), CD19xTCRα/β-VH and -VL (4G7xBMA031-VH and -VL), CD19xCD28-VH and - VL (4G7x9.3-VH and -VL), CD19xCD16-VH and -VL (4G7x3G8-VH and -VL), CSPG4xCD3-VH and -VL (9.2.27xUCHT1-VH and -VL), CSPG4xTCRα/β-VH and -VL (9.2.27xBMA031-VH and -VL), CSPG4xCD28-VH and -VL (9.2.27x9.3-VH and -VL), CSPG4xCD16-VH and -VL (9.2.27x3G8-VH and -VL), EGFRxCD3-VH and -VL (C225xUCHT1-VH and -VL), EGFRxTCRα/β-VH and -VL (C225xBMA031-VH and -VL), EGFRxCD28-VH and -VL (C225x9.3-VH and -VL), EGFRxCD16-VH and -VL (C225x3G8-VH and -VL) can have the sequences as depicted in Fig. 6C. Sequences of the corresponding chains are depicted as SEQ ID NO: 22 to SEQ ID NO: 61 in Fig. 6C.

Cotransfection of the expression vectors encoding the chimeric heavy and light chain (IgG1/κ) or modified heavy chains into the non-Ig-producing myeloma cell line Sp2/0 yielded stable transfectomas secreting monoclonal antibody molecules which are able to bind specifically to the desired antigen. [

Cotransfection of expression vectors encoding main and smaller chains, which can also be referred to as heavy and light chains, of indicated specificities was done in Sp2/0 plasmocytoma cells, obtained from the American Type Culture Collection (ATCC, Manassas, VA). For the build-up of the respective vectors reference is made to Fig. 7 (see also Example II -). Cells were cultured in IMDM media, supplemented with 10% fetal calf serum (PAN-Biotech, Aidenbach, Germany), 1 % penicillin and streptomycin (Lonza, Basel, Switzerland). Stable transfectants were selected by adding 1 mg/ml G418 (Invitrogen, Karlsruhe, Germany).

Recombinant antibody molecules were purified from supernatants of cultures of stably transfected cells via affinity chromatography using KappaSelect for the Fabv-^{ko} format (chromatography media were obtained from GE Healthcare, Munich, Germany, KappaSelect has as affinity ligand a recombinant protein (Mr 13 000), produced in S. cerevisiae, with affinity for the constant domain of the immunoglobulin kappa light chain).

### EXAMPLE III

An improved, Fab based bifunctional and combinatorial antibody format (Fabv-^{ko} format)

In the Fabv-^{ko}format the targeting moiety consists of an N-terminal Fab and a C-terminal VH- or VL-domain linked by a CH2-domain. To prevent binding to Fc receptors and homodimerization via disulfide bonds several amino acid modifications have been introduced into this domain (Fig. 1 and 2A-E, A'-E', Fig. 6). As indicated above the principal advantages of this format, compared to an antibody in the tdsc-format consisting solely of three variable domains are (1) superior production rates, (2) improved serum half life, (3) preserved binding affinity of the targeting part and (4) decreased multimerization/aggregation tendency. Decreased multimerization/aggregation is particularly important, if the C-terminal single chain antibody is directed to the TCR/CD3 complex to induce target cell restricted T cell activation. In this case even small amounts of aggregates may lead to off-target T cell activation.

### EXAMPLE IV

Defining an optimal TCR/CD3 antibody for use within the Fabv-format.

Fig. 3 shows gel filtration analysis of Fabv-^{ko} antibody molecules with two target specificities, FLT3 and CD19, and VH and VL regions derived from the CD3 antibodies UCHT1 and OKT3 and the TCR antibody BMA031 (see Fig. 6 for the polypeptide sequences). Fabv-^{ko} antibody molecules containing VH and VL regions of OKT3 and BMA031 formed some multimers and aggregates (>60% of the material).

The Fabv-^{ko} -molecules containing the variable domains of the OKT3 or BMA031 antibodies formed some homoaggregates whereas Fabv-^{ko} -molecules with V-regions derived from UCHT1 did not form multimers (Fig. 3). Thus, it appears that UCHT1 shows the best characteristics of the used antibodies in terms of formation of multimers and aggregates.

Fig. 4 demonstrates that a combination of FLT3 X UCHT1-VH and CD19 X UCHT1-VL Fabv-^{ko} antibodies, but neither reagent alone is capable of activating human T cells in the presence of NALM16 target cells that express both, the FLT3- and the CD19 antigen. Likewise, a combination of Fabv-^{ko} - antibody molecules targeting FLT3, but neither antibody alone, is capable of mediating the killing of NALM16 cells by activated cytolytic T cells (Fig. 5). This indicates that a functional CD3 binding site has been formed by the formation of heterodimers of the kind described in this invention.

One skilled in the art would readily appreciate that the present invention is well adapted to carry out the objects and obtain the ends and advantages mentioned, as well as those inherent therein. Further, it will be readily apparent to one skilled in the art that varying substitutions and modifications may be made to the invention disclosed herein without departing from the scope and spirit of the invention. The compositions, methods, procedures, treatments, molecules and specific compounds described herein are presently representative of certain embodiments are exemplary and are not intended as limitations on the scope of the invention. Changes therein and other uses will occur to those skilled in the art which are encompassed within the spirit of the invention are defined by the scope of the claims. The listing or discussion of a previously published document in this specification should not necessarily be taken as an acknowledgement that the document is part of the state of the art or is common general knowledge.

The invention illustratively described herein may suitably be practiced in the absence of any element or elements, limitation or limitations, not specifically disclosed herein. Thus, for example, the terms "comprising", "including," containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by exemplary embodiments and optional features, modification and variation of the inventions embodied therein may be resorted to by those skilled in the art, and that such modifications and variations are considered to be within the scope of this invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

### Reference List

1) Staerz UD, Kanagawa O, Bevan MJ. Hybrid antibodies can target sites for attack by T cells. Nature 1985; 314:628-631
2) Perez P, Hoffman RW, Shaw S, Bluestone JA, Segal DM. Specific targeting of cytotoxic T-cells by anti-T3 linked to anti-target cell antibody. Nature 1985; 316:354-356
3) Jung G, Honsik CJ, Reisfeld RA and Müller-Eberhard HJ. Activation of human peripheral blood mononuclear cells by anti-T3: Killing of tumor target cells coated with anti-target X anti-T3-conjugates. Proc Natl Acad Sci USA 1986; 83:4479-4483
4) Jung G and Müller-Eberhard HJ. An in vitro model for tumor immunotherapy with antibody-heteroconjugates. Immunol Today 1988; 9:257-260
5) Jung G, Freimann U, v.Marschall Z, Reisfeld RA and Wilmanns W. Target cell induced T cell activation with bi- and trispecific antibody fragments. Eur J Immunol 1991; 21:2431-2435
6) Bargou R, Leo E, Zugmaier G et al. Tumor regression in cancer patients by very low doses of a T-cell-engaging antibody. Science 2008; 321:974-977
7) Topp MS, Kufer P, Gokbuget N et al. Targeted therapy with the T-cell-engaging antibody blinatumomab of chemotherapy-refractory minimal residual disease in B-lineage acute lymphoblastic leukemia patients results in high response rate and prolonged leukemia-free survival. J Clin Oncol 2011; 29:2493-2498.
8) Kroesen BJ, Buter J, Sleijfer DT et al. Phase I study of intravenously applied bispecific antibody in renal cell cancer patients receiving subcutaneous interleukin 2. Br J Cancer 1994; 70:652-661.
9) Tibben JG, Boerman OC, Massuger LF et al. Pharmacokinetics, biodistribution and biological effects of intravenously administered bispecific monoclonal antibody OC/TR F(ab')2 in ovarian carcinoma patients. Int J Cancer 1996; 66:477-483.
10) Molema G, Tervaert JW, Kroesen BJ, Helfrich W, Meijer DK, de Leij LF. CD3 directed bispecific antibodies induce increased lymphocyte-endothelial cell interactions in vitro. Br J Cancer 2000; 82:472-479.

## Claims

1. A recombinant antibody molecule consisting of a Fab fragment comprising a first binding site for a first antigen, a variable domain of either the light chain or the heavy chain of a second binding site for a second antigen and an immunoglobulin CH2 domain, wherein the Fab fragment and the variable domain are linked via the CH2 domain, wherein in the immunoglobulin CH2 domain at least one cysteine residue that is able to form a disulfide bridge for dimerisation is lacking or mutated.

2. The antibody molecule of claim 1, wherein the at least one cysteine residue is selected from the sequence positions 226, 228 and 229 of the CH2 domain, wherein preferably a cysteine at one or both of positions 226 and 229 is replaced by a different amino acid.

3. The antibody molecule of claim 1 or 2, comprising the light chain of the variable domain of the second binding site for the second antigen.

4. The antibody molecule of any of claims 1 or 2, comprising the heavy chain of the variable domain of the second binding site for the second antigen.

5. The antibody molecule of any of claims 1 to 4, wherein either the first binding site or the second binding site binds a tumor associated antigen.

6. The antibody molecule of any of claims 1 to 5, wherein either the first binding site or the second binding site binds a T-cell- or NK (natural killer) cell specific receptor molecule.

7. The antibody molecule of any of the preceding claims, wherein the light chain of the variable domain of the second binding site has a sequence identity of at least 80%, or at least 85 %, or at least 90 %, or at least 95 %, or at least 98 %, or at least 99 % or 100 % to the variable domain of the light chain of UCHT-1 as shown in SEQ ID NO. 3 or
wherein the heavy chain of the variable domain of the second binding site has a sequence identity of at least 80% or at least 85 %, or at least 90 %, or at least 95 %, or at least 98 %, or at least 99 % or 100 % to the variable domain of the heavy chain of UCHT-1 as shown to SEQ ID NO. 4.

8. The antibody molecule of any of the preceding claims, wherein at least one amino acid residue of the CH2 domain that is able to mediate binding to Fc receptors is lacking or mutated.

9. The antibody molecule of claim 8, wherein the amino acid residues are selected from the group consisting of sequence position 230, 231, 232, 233, 234, 235, 236, 237, 238, 265, 297, 327, and 330 (numbering of sequence positions according to the EU-index), wherein the least one mutation is preferably selected from the group consisting of a deletion of amino acid 230, a deletion of amino acid 231, a deletion of amino acid 232, a deletion of amino acid 233, a substitution Glu233→Pro, a substitution Leu234→Val, a deletion of amino acid 234, a substitution Leu235→Ala, a deletion of amino acid 235, a deletion of amino acid 236, a deletion of amino acid 237, a deletion of amino acid 238, a substitution Asp265→Gly, a substitution Asn297→Gln, a substitution Ala327→Gln, and a substitution Ala330→Ser.

10. A bispecific heterodimeric antibody molecule comprising a hetero-dimer of recombinant antibody molecules (monomers),
wherein the first monomer of the hetero-dimer consists of a Fab fragment comprising a first binding site for a first antigen, a variable domain of the light chain of a second binding site for a second antigen and an immunoglobulin CH2 domain, wherein the Fab fragment and the variable domain of the light chain are linked via the CH2 domain, and
wherein the second monomer of the hetero-dimer consists of a Fab fragment comprising a first binding site for the first antigen, a variable domain of the heavy chain of the second binding site for the second antigen and an immunoglobulin CH2 domain, wherein the Fab fragment and the variable domain of the heavy chain are linked via the CH2 domain,
wherein the variable domain of the light chain of the second binding site of the first monomer and the variable domain of the heavy chain of the second binding site of the second monomer associate thereby forming, the second binding site and dimerizing the hetero-dimer, and
wherein in at least one of the immunoglobulin CH2 domain of the first monomer or the second monomer at least one cysteine residue that is able to form a disulfide bridge for dimerisation is lacking or mutated, thereby preventing that a CH2 domain mediated disulfide bridge is formed between the two monomers.

11. A tri-specific heterodimeric antibody molecule comprising a hetero-dimer of recombinant antibody molecules (monomers),
wherein the first monomer of the hetero-dimer consists of a Fab fragment comprising a first binding site for a first antigen, a variable domain of the light chain of a second binding site for a second antigen and an immunoglobulin CH2 domain, wherein the Fab fragment and the variable domain of the light chain are linked via the CH2 domain, and
wherein the second monomer of the hetero-dimer consists of a Fab fragment comprising a third binding site for a third antigen, wherein the third antigen is different from the first antigen, a variable domain of the heavy chain of the second binding site for the second antigen and an immunoglobulin CH2 domain, wherein the Fab fragment and the variable domain of the heavy chain are linked via the CH2 domain,
wherein the variable domain of the light chain of the second binding site of the first monomer and the variable domain of the heavy chain of the second binding site of the second monomer associate thereby forming, the second binding site and dimerizing the hetero-dimer,
wherein in at least one of the immunoglobulin CH2 domain of the first monomer or the second monomer at least one cysteine residue that is able to form a disulfide bridge for dimerisation is lacking or mutated, thereby preventing that a CH2 domain mediated disulfide bridge is formed between the two monomers.

12. A pharmaceutical composition comprising an antibody molecule as defined in any of the preceding claims.

13. An antibody molecule as defined in any of claims 1 to 9 for use in the treatment or diagnosis of a disease.

14. The use of an antibody molecule as defined in any of claims 1 to 9 for the treatment of a disease, wherein the antibody molecule forms a hetero-dimer only in vivo on a target cell, thereby reducing "off target activation".

15. The use of claim 14, wherein the antibody molecule provides for target cell restricted T cell-activation.wherein the disease preferably is a proliferatory disease.


SEQUENCE LISTING

<110>  Eberhard Karls Universitaet Tuebingen
       Universitaet Wuerzburg
       Deutsches Krebsforschungszentrum

<120>  RECOMBINANT ANTIBODY MOLECULES FOR TARGET CELL RESTRICTED T CELL
       ACTIVATION

<130>  DKF15031EP

<160>  127

<170>  PatentIn version 3.5

<210>  1
<211>  107
<212>  PRT
<213>  Artificial Sequence

<220>
<223>  anti-FLT3 chimeric single variable domain  light chain (clone
       4G8) (VL)

<400>  1

Asp Ile Val Leu Thr Gln Ser Pro Ala Thr Leu Ser Val Thr Pro Gly
1               5                   10                  15

Asp Ser Val Ser Leu Ser Cys Arg Ala Ser Gln Ser Ile Ser Asn Asn
            20                  25                  30

Leu His Trp Tyr Gln Gln Lys Ser His Glu Ser Pro Arg Leu Leu Ile
        35                  40                  45

Lys Tyr Ala Ser Gln Ser Ile Ser Gly Ile Pro Ser Arg Phe Ser Gly
    50                  55                  60

Ser Gly Ser Gly Thr Asp Phe Thr Leu Ser Ile Asn Ser Val Glu Thr
65                  70                  75                  80

Glu Asp Phe Gly Val Tyr Phe Cys Gln Gln Ser Asn Thr Trp Pro Tyr
                85                  90                  95

Thr Phe Gly Gly Gly Thr Lys Leu Glu Ile Lys
            100                 105

<210>  2
<211>  118
<212>  PRT
<213>  Artificial Sequence

<220>
<223>  anti-FLT3 chimeric single variable domain heavy chain (clone 4G8)
       (VH)

<400>  2

Gln Val Gln Leu Gln Gln Pro Gly Ala Glu Leu Val Lys Pro Gly Ala
1               5                   10                  15

Ser Leu Lys Leu Ser Cys Lys Ser Ser Gly Tyr Thr Phe Thr Ser Tyr
            20                  25                  30

Trp Met His Trp Val Arg Gln Arg Pro Gly His Gly Leu Glu Trp Ile
        35                  40                  45

Gly Glu Ile Asp Pro Ser Asp Ser Tyr Lys Asp Tyr Asn Gln Lys Phe
    50                  55                  60

Lys Asp Lys Ala Thr Leu Thr Val Asp Arg Ser Ser Asn Thr Ala Tyr
65                  70                  75                  80

Met His Leu Ser Ser Leu Thr Ser Asp Asp Ser Ala Val Tyr Tyr Cys
                85                  90                  95

Ala Arg Ala Ile Thr Thr Thr Pro Phe Asp Phe Trp Gly Gln Gly Thr
            100                 105                 110

Thr Leu Thr Val Ser Ser
        115

<210>  3
<211>  107
<212>  PRT
<213>  Artificial Sequence

<220>
<223>  CD3 single variable domain (clone UCHT1) VL

<400>  3

<210>  4
<211>  122
<212>  PRT
<213>  Artificial Sequence

<220>
<223>  CD3 single variable domain (clone UCHT1) VH

<400>  4

<210>  5
<211>  113
<212>  PRT
<213>  Artificial Sequence

<220>
<223>  anti-FLT3 single variable domain (clone BV10), VL

<400>  5

Asp Ile Val Met Thr Gln Ser Pro Ser Ser Leu Ser Val Ser Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Met Ala Trp Tyr Gln Gln Lys Pro Gly Gln
        35                  40                  45

Pro Pro Lys Leu Leu Ile Tyr Gly Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp His Ser Tyr Pro Leu Thr Phe Gly Ala Gly Thr Lys Leu Glu Leu
            100                 105                 110

Lys

<210> 6
<211> 123
<212> PRT
<213> Artificial Sequence

<220>
<223> anti-FLT3 single variable domain VH (clone BV10)

<400> 6

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Leu His Trp Val Arg Gln Ser Pro Gly Lys Gly Leu Glu Trp Leu
        35                  40                  45

Gly Val Ile Trp Ser Gly Gly Ser Thr Asp Tyr Asn Ala Ala Phe Ile
    50                  55                  60

Ser Arg Leu Ser Ile Ser Lys Asp Asn Ser Lys Ser Gln Val Phe Phe
65                  70                  75                  80

Lys Met Asn Ser Leu Gln Ala Asp Asp Thr Ala Ile Tyr Tyr Cys Ala
                85                  90                  95

Arg Lys Gly Gly Ile Tyr Tyr Ala Asn His Tyr Tyr Ala Met Asp Tyr
            100                 105                 110

Trp Gly Gln Gly Thr Ser Val Thr Val Ser Ser
        115                 120

<210>  7
<211>  106
<212>  PRT
<213>  Artificial Sequence

<220>
<223>  C-terminal TCR /  single variable domain (clone BMA031) VL

<400>  7

Gln Ile Val Leu Thr Gln Ser Pro Ala Ile Met Ser Ala Ser Pro Gly
1               5                   10                  15

Glu Lys Val Thr Met Thr Cys Ser Ala Thr Ser Ser Val Ser Tyr Met
            20                  25                  30

His Trp Tyr Gln Gln Lys Ser Gly Thr Ser Pro Lys Arg Trp Ile Tyr
        35                  40                  45

Asp Thr Ser Lys Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys Gln Gln Trp Ser Ser Asn Pro Leu Thr
                85                  90                  95

Phe Gly Ala Gly Thr Lys Leu Glu Leu Lys
            100                 105

<210>  8
<211>  119
<212>  PRT
<213>  Artificial Sequence

<220>
<223>  C-terminal TCR /  single variable domain (clone BMA031 VH

<400>  8

Glu Val Gln Leu Gln Gln Ser Gly Pro Glu Leu Val Lys Pro Gly Ala
1               5                   10                  15

Ser Val Lys Met Ser Cys Lys Ala Ser Gly Tyr Lys Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Val Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val His Tyr Cys
                85                  90                  95

Ala Arg Gly Ser Tyr Tyr Asp Tyr Asp Gly Phe Val Tyr Gly Gln Gly
            100                 105                 110

Thr Leu Val Thr Val Ser Ser
        115

<210>  9
<211>  112
<212>  PRT
<213>  Artificial Sequence

<220>
<223>  Anti-CD19 single variable domain VL (clone 4G7)

<400>  9

Asp Ile Val Met Thr Gln Ala Ala Pro Ser Ile Pro Val Thr Pro Gly
1               5                   10                  15

Glu Ser Val Ser Ile Ser Cys Arg Ser Ser Lys Ser Leu Leu Asn Ser
            20                  25                  30

Asn Gly Asn Thr Tyr Leu Tyr Trp Phe Leu Gln Arg Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Gly Ser Gly Ser Gly Thr Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Phe Thr Phe Gly Ala Gly Thr Lys Leu Glu Leu Lys
            100                 105                 110

<210>  10
<211>  121
<212>  PRT
<213>  Artificial Sequence

<220>
<223>  Anti-CD19 single variable domain VH (clone 4G7)

<400>  10

Glu Val Gln Leu Gln Gln Ser Gly Pro Glu Leu Ile Lys Pro Gly Ala
1               5                   10                  15

Ser Val Lys Met Ser Cys Lys Ala Ser Gly Tyr Thr Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Gly Thr Tyr Tyr Tyr Gly Ser Arg Val Phe Asp Tyr Trp Gly
            100                 105                 110

Gln Gly Thr Thr Leu Thr Val Ser Ser
        115                 120


<210>  11
<211>  112
<212>  PRT
<213>  Artificial Sequence

<220>
<223>  Anti- CD28 single variable domain (clone 9.3) VL

<400>  11

Asp Ile Glu Leu Thr Gln Ser Pro Ala Ser Leu Ala Val Ser Leu Gly
1               5                   10                  15

Gln Arg Ala Thr Ile Ser Cys Arg Ala Ser Glu Ser Val Glu Tyr Tyr
            20                  25                  30

Val Thr Ser Leu Met Gln Trp Tyr Gln Gln Lys Pro Gly Gln Pro Pro
        35                  40                  45

Lys Leu Leu Ile Phe Ala Ala Ser Asn Val Glu Ser Gly Val Pro Ala
    50                  55                  60

Arg Phe Ser Gly Ser Gly Ser Gly Thr Asn Phe Ser Leu Asn Ile His
65                  70                  75                  80

Pro Val Asp Glu Asp Asp Val Ala Met Tyr Phe Cys Gln Gln Ser Arg
                85                  90                  95

Lys Val Pro Tyr Thr Phe Gly Gly Gly Thr Lys Leu Glu Ile Lys Arg
            100                 105                 110

<210>  12
<211>  120
<212>  PRT
<213>  Artificial Sequence

<220>
<223>  Anti- CD28 single variable domain (clone 9.3) VH

<400>  12

Gln Val Lys Leu Gln Gln Ser Gly Pro Gly Leu Val Thr Pro Ser Gln
1               5                   10                  15

Ser Leu Ser Ile Thr Cys Thr Val Ser Gly Phe Ser Leu Ser Asp Tyr
            20                  25                  30

Gly Val His Trp Val Arg Gln Ser Pro Gly Gln Gly Leu Glu Trp Leu
        35                  40                  45

Gly Val Ile Trp Ala Gly Gly Gly Thr Asn Tyr Asn Ser Ala Leu Met
    50                  55                  60

Ser Arg Lys Ser Ile Ser Lys Asp Asn Ser Lys Ser Gln Val Phe Leu
65                  70                  75                  80

Lys Met Asn Ser Leu Gln Ala Asp Asp Thr Ala Val Tyr Tyr Cys Ala
                85                  90                  95

Arg Asp Lys Gly Tyr Ser Tyr Tyr Tyr Ser Met Asp Tyr Trp Gly Gln
            100                 105                 110

Gly Thr Thr Val Thr Val Ser Ser
        115                 120

<210>  13
<211>  111
<212>  PRT

<213>  Artificial Sequence

<220>
<223>  Anti-CD16 single variable domain (clone 3G8) VL

<400>  13

Asp Ile Val Leu Thr Gln Ser Pro Ala Ser Leu Ala Val Ser Leu Gly
1               5                   10                  15

Gln Arg Ala Thr Ile Ser Cys Lys Ala Ser Gln Ser Val Asp Phe Asp
            20                  25                  30

Gly Asp Ser Phe Met Asn Trp Tyr Gln Gln Lys Pro Gly Gln Pro Pro
        35                  40                  45

Lys Leu Leu Ile Tyr Thr Thr Ser Asn Leu Glu Ser Gly Ile Pro Ala
    50                  55                  60

Arg Phe Ser Ala Ser Gly Ser Gly Thr Asp Phe Thr Leu Asn Ile His
65                  70                  75                  80

Pro Val Glu Glu Glu Asp Thr Ala Thr Tyr Tyr Cys Gln Gln Ser Asn
                85                  90                  95

Glu Asp Pro Tyr Thr Phe Gly Gly Gly Thr Lys Leu Glu Ile Lys
            100                 105                 110

<210>  14
<211>  118
<212>  PRT
<213>  Artificial Sequence

<220>
<223>  Anti-CD16 single variable domain (clone 3G8) VH

<400>  14

Gln Val Thr Leu Lys Glu Ser Gly Pro Gly Ile Leu Gln Pro Ser Gln
1               5                   10                  15

Thr Leu Ser Leu Thr Cys Ser Phe Ser Gly Phe Ser Leu Arg Thr Ser
            20                  25                  30

Gly Met Gly Val Gly Trp Ile Arg Gln Pro Ser Gly Lys Gly Leu Glu
        35                  40                  45

Trp Leu Ala His Ile Trp Trp Asp Asp Asp Lys Arg Tyr Asn Pro Ala
    50                  55                  60

Leu Lys Ser Arg Leu Thr Ile Ser Lys Asp Thr Ser Ser Asn Gln Val
65                  70                  75                  80

Phe Leu Lys Ile Ala Ser Val Asp Thr Ala Asp Thr Ala Thr Tyr Tyr
                85                  90                  95

Cys Ala Gln Ile Asn Pro Ala Trp Phe Ala Tyr Trp Gly Gln Gly Thr
            100                 105                 110

Leu Val Thr Val Ser Ser
        115

<210>  15
<211>  111
<212>  PRT
<213>  Artificial Sequence

<220>
<223>  Anti-CSPG4 chimeric single variable domain  VL (clone 9.2.27)

<400>  15

Asp Ile Glu Leu Thr Gln Ser Pro Ala Ser Leu Ala Val Ser Leu Gly
1               5                   10                  15

Gln Arg Ala Thr Ile Ser Cys Arg Ala Ser Glu Ser Val Asp Ser Tyr
            20                  25                  30

Gly Asn Ser Phe Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Arg Thr Asp Phe Thr Leu Thr Ile Asp
65                  70                  75                  80

Pro Val Glu Ala Asp Asp Ala Ala Thr Tyr Tyr Cys Gln Gln Asn Asn
                85                  90                  95

Glu Asp Pro Leu Thr Phe Gly Gly Gly Thr Lys Leu Glu Ile Lys
            100                 105                 110

<210>  16
<211>  121
<212>  PRT
<213>  Artificial Sequence

<220>
<223>  anti-CSPG4 single variable domain  VH (clone 9.2.27)

<400>  16

Gln Val Lys Leu Gln Gln Ser Gly Pro Glu Leu Val Lys Pro Gly Ala

1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Thr Val Thr Val Ser Ser
        115                 120

<210>  17
<211>  107
<212>  PRT
<213>  Artificial Sequence

<220>
<223>  Anti-EGFR single variable domain  VL(clone C225)

<400>  17

Asp Ile Leu Leu Thr Gln Ser Pro Val Ile Leu Ser Val Ser Pro Gly
1               5                   10                  15

Glu Arg Val Ser Phe Ser Cys Arg Ala Ser Gln Ser Ile Gly Thr Asn
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Asn Asn Asn Trp Pro Thr
                85                  90                  95

Thr Phe Gly Ala Gly Thr Lys Leu Glu Leu Lys
            100                 105

<210>  18
<211>  121
<212>  PRT
<213>  Artificial Sequence

<220>
<223>  anti-CSPG4 single variable domain  VH (clone 9.2.27)

<400>  18

Gln Val Lys Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Thr Val Thr Val Ser Ser
        115                 120

<210>  19
<211>  98
<212>  PRT
<213>  Artificial Sequence

<220>
<223>  CH1 domain

<400>  19

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr

                20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val

<210>  20
<211>  124
<212>  PRT
<213>  Artificial Sequence

<220>
<223>  Hinge and CH2  region

<400>  20

Glu Pro Lys Ser Cys Asp Lys Thr His Thr Ser Pro Pro Ser Pro Ala
1               5                   10                  15

Pro Pro Val Ala Gly Pro Ser Val Phe Leu Phe Pro Pro Lys Pro Lys
            20                  25                  30

Asp Thr Leu Met Ile Ser Arg Thr Pro Glu Val Thr Cys Val Val Val
        35                  40                  45

Gly Val Ser His Glu Asp Pro Glu Val Lys Phe Asn Trp Tyr Val Asp
    50                  55                  60

Gly Val Glu Val His Asn Ala Lys Thr Lys Pro Arg Glu Glu Gln Tyr
65                  70                  75                  80

Gln Ser Thr Tyr Arg Val Val Ser Val Leu Thr Val Leu His Gln Asp
                85                  90                  95

Trp Leu Asn Gly Lys Glu Tyr Lys Cys Lys Val Ser Asn Lys Gln Leu
            100                 105                 110

Pro Ser Pro Ile Glu Lys Thr Ile Ser Lys Ala Lys
        115                 120

<210>  21
<211>  107
<212>  PRT
<213>  Artificial Sequence

<220>
<223>  C-terminal constant domain

<400>  21


<210>  22
<211>  452
<212>  PRT
<213>  Artificial Sequence

<220>
<223>  FLT3 x CD3;  Fabv

<400>  22

Gln Val Gln Leu Gln Gln Pro Gly Ala Glu Leu Val Lys Pro Gly Ala
1               5                   10                  15

Ser Leu Lys Leu Ser Cys Lys Ser Ser Gly Tyr Thr Phe Thr Ser Tyr
            20                  25                  30

Trp Met His Trp Val Arg Gln Arg Pro Gly His Gly Leu Glu Trp Ile
        35                  40                  45

Gly Glu Ile Asp Pro Ser Asp Ser Tyr Lys Asp Tyr Asn Gln Lys Phe

50                  55                  60

Lys Asp Lys Ala Thr Leu Thr Val Asp Arg Ser Ser Asn Thr Ala Tyr
65                  70                  75                  80

Met His Leu Ser Ser Leu Thr Ser Asp Asp Ser Ala Val Tyr Tyr Cys
                85                  90                  95

Ala Arg Ala Ile Thr Thr Thr Pro Phe Asp Phe Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Ser Pro Pro Ser Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Gly Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Gln Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gln Leu Pro Ser Pro Ile Glu Lys Thr Ile
                325                 330                 335

Ser Lys Ala Lys Gly Gln Pro Ser Gly Asp Ile Gln Met Thr Gln Ser
            340                 345                 350

Pro Ser Ser Leu Ser Ala Ser Val Gly Asp Arg Val Thr Ile Thr Cys
        355                 360                 365

Arg Ala Ser Gln Asp Ile Arg Asn Tyr Leu Asn Trp Tyr Gln Gln Lys
    370                 375                 380

Pro Gly Lys Ala Pro Lys Leu Leu Ile Tyr Tyr Thr Ser Arg Leu Glu
385                 390                 395                 400

Ser Gly Val Pro Ser Arg Phe Ser Gly Ser Gly Ser Gly Thr Asp Tyr
                405                 410                 415

Thr Leu Thr Ile Ser Ser Leu Gln Pro Glu Asp Phe Ala Thr Tyr Tyr
            420                 425                 430

Cys Gln Gln Gly Asn Thr Leu Pro Trp Thr Phe Gly Gln Gly Thr Lys
        435                 440                 445

Val Glu Ile Lys
    450

<210>  23
<211>  467
<212>  PRT
<213>  Artificial Sequence

<220>
<223>  FLT3 x CD3;  Fabv

<400>  23

Gln Val Gln Leu Gln Gln Pro Gly Ala Glu Leu Val Lys Pro Gly Ala
1               5                   10                  15

Ser Leu Lys Leu Ser Cys Lys Ser Ser Gly Tyr Thr Phe Thr Ser Tyr
            20                  25                  30

Trp Met His Trp Val Arg Gln Arg Pro Gly His Gly Leu Glu Trp Ile
        35                  40                  45

Gly Glu Ile Asp Pro Ser Asp Ser Tyr Lys Asp Tyr Asn Gln Lys Phe

50                  55                  60

Lys Asp Lys Ala Thr Leu Thr Val Asp Arg Ser Ser Asn Thr Ala Tyr
65                  70                  75                  80

Met His Leu Ser Ser Leu Thr Ser Asp Asp Ser Ala Val Tyr Tyr Cys
                85                  90                  95

Ala Arg Ala Ile Thr Thr Thr Pro Phe Asp Phe Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Ser Pro Pro Ser Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Gly Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Gln Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gln Leu Pro Ser Pro Ile Glu Lys Thr Ile
                325                 330                 335

Ser Lys Ala Lys Gly Gln Pro Ser Gly Glu Val Gln Leu Val Glu Ser
            340                 345                 350

Gly Gly Gly Leu Val Gln Pro Gly Gly Ser Leu Arg Leu Ser Cys Ala
        355                 360                 365

Ala Ser Gly Tyr Ser Phe Thr Gly Tyr Thr Met Asn Trp Val Arg Gln
    370                 375                 380

Ala Pro Gly Lys Gly Leu Glu Trp Val Ala Leu Ile Asn Pro Tyr Lys
385                 390                 395                 400

Gly Val Ser Thr Tyr Asn Gln Lys Phe Lys Asp Arg Phe Thr Ile Ser
                405                 410                 415

Val Asp Lys Ser Lys Asn Thr Ala Tyr Leu Gln Met Asn Ser Leu Arg
            420                 425                 430

Ala Glu Asp Thr Ala Val Tyr Tyr Cys Ala Arg Ser Gly Tyr Tyr Gly
        435                 440                 445

Asp Ser Asp Trp Tyr Phe Asp Val Trp Gly Gln Gly Thr Leu Val Thr
    450                 455                 460

Val Ser Ser
465

<210>  24
<211>  457
<212>  PRT
<213>  Artificial Sequence

<220>
<223>  FLT3 x CD3 Fabv

<400>  24

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Leu His Trp Val Arg Gln Ser Pro Gly Lys Gly Leu Glu Trp Leu

35                  40                  45

Gly Val Ile Trp Ser Gly Gly Ser Thr Asp Tyr Asn Ala Ala Phe Ile
    50                  55                  60

Ser Arg Leu Ser Ile Ser Lys Asp Asn Ser Lys Ser Gln Val Phe Phe
65                  70                  75                  80

Lys Met Asn Ser Leu Gln Ala Asp Asp Thr Ala Ile Tyr Tyr Cys Ala
                85                  90                  95

Arg Lys Gly Gly Ile Tyr Tyr Ala Asn His Tyr Tyr Ala Met Asp Tyr
            100                 105                 110

Trp Gly Gln Gly Thr Ser Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Ser Pro Pro Ser Pro Ala Pro Pro Val
225                 230                 235                 240

Ala Gly Pro Ser Val Phe Leu Phe Pro Pro Lys Pro Lys Asp Thr Leu
                245                 250                 255

Met Ile Ser Arg Thr Pro Glu Val Thr Cys Val Val Val Gly Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Gln Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Gln Leu Pro Ser Pro
                325                 330                 335

Ile Glu Lys Thr Ile Ser Lys Ala Lys Gly Gln Pro Ser Gly Asp Ile
            340                 345                 350

Gln Met Thr Gln Ser Pro Ser Ser Leu Ser Ala Ser Val Gly Asp Arg
        355                 360                 365

Val Thr Ile Thr Cys Arg Ala Ser Gln Asp Ile Arg Asn Tyr Leu Asn
    370                 375                 380

Trp Tyr Gln Gln Lys Pro Gly Lys Ala Pro Lys Leu Leu Ile Tyr Tyr
385                 390                 395                 400

Thr Ser Arg Leu Glu Ser Gly Val Pro Ser Arg Phe Ser Gly Ser Gly
                405                 410                 415

Ser Gly Thr Asp Tyr Thr Leu Thr Ile Ser Ser Leu Gln Pro Glu Asp
            420                 425                 430

Phe Ala Thr Tyr Tyr Cys Gln Gln Gly Asn Thr Leu Pro Trp Thr Phe
        435                 440                 445

Gly Gln Gly Thr Lys Val Glu Ile Lys
    450                 455

<210>  25
<211>  472
<212>  PRT
<213>  Artificial Sequence

<220>
<223>  FLT3 x CD3 Fabv

<400>  25

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Leu His Trp Val Arg Gln Ser Pro Gly Lys Gly Leu Glu Trp Leu

35                  40                  45

Gly Val Ile Trp Ser Gly Gly Ser Thr Asp Tyr Asn Ala Ala Phe Ile
    50                  55                  60

Ser Arg Leu Ser Ile Ser Lys Asp Asn Ser Lys Ser Gln Val Phe Phe
65                  70                  75                  80

Lys Met Asn Ser Leu Gln Ala Asp Asp Thr Ala Ile Tyr Tyr Cys Ala
                85                  90                  95

Arg Lys Gly Gly Ile Tyr Tyr Ala Asn His Tyr Tyr Ala Met Asp Tyr
            100                 105                 110

Trp Gly Gln Gly Thr Ser Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Ser Pro Pro Ser Pro Ala Pro Pro Val
225                 230                 235                 240

Ala Gly Pro Ser Val Phe Leu Phe Pro Pro Lys Pro Lys Asp Thr Leu
                245                 250                 255

Met Ile Ser Arg Thr Pro Glu Val Thr Cys Val Val Val Gly Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Gln Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Gln Leu Pro Ser Pro
                325                 330                 335

Ile Glu Lys Thr Ile Ser Lys Ala Lys Gly Gln Pro Ser Gly Glu Val
            340                 345                 350

Gln Leu Val Glu Ser Gly Gly Gly Leu Val Gln Pro Gly Gly Ser Leu
        355                 360                 365

Arg Leu Ser Cys Ala Ala Ser Gly Tyr Ser Phe Thr Gly Tyr Thr Met
    370                 375                 380

Asn Trp Val Arg Gln Ala Pro Gly Lys Gly Leu Glu Trp Val Ala Leu
385                 390                 395                 400

Ile Asn Pro Tyr Lys Gly Val Ser Thr Tyr Asn Gln Lys Phe Lys Asp
                405                 410                 415

Arg Phe Thr Ile Ser Val Asp Lys Ser Lys Asn Thr Ala Tyr Leu Gln
            420                 425                 430

Met Asn Ser Leu Arg Ala Glu Asp Thr Ala Val Tyr Tyr Cys Ala Arg
        435                 440                 445

Ser Gly Tyr Tyr Gly Asp Ser Asp Trp Tyr Phe Asp Val Trp Gly Gln
    450                 455                 460

Gly Thr Leu Val Thr Val Ser Ser
465                 470

<210>  26
<211>  464
<212>  PRT
<213>  Artificial Sequence

<220>
<223>  FLT3 x TCR Fabv

<400>  26

Gln Val Gln Leu Gln Gln Pro Gly Ala Glu Leu Val Lys Pro Gly Ala
1               5                   10                  15

Ser Leu Lys Leu Ser Cys Lys Ser Ser Gly Tyr Thr Phe Thr Ser Tyr

20                  25                  30

Trp Met His Trp Val Arg Gln Arg Pro Gly His Gly Leu Glu Trp Ile
        35                  40                  45

Gly Glu Ile Asp Pro Ser Asp Ser Tyr Lys Asp Tyr Asn Gln Lys Phe
    50                  55                  60

Lys Asp Lys Ala Thr Leu Thr Val Asp Arg Ser Ser Asn Thr Ala Tyr
65                  70                  75                  80

Met His Leu Ser Ser Leu Thr Ser Asp Asp Ser Ala Val Tyr Tyr Cys
                85                  90                  95

Ala Arg Ala Ile Thr Thr Thr Pro Phe Asp Phe Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Ser Pro Pro Ser Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Gly Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Gln Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gln Leu Pro Ser Pro Ile Glu Lys Thr Ile
                325                 330                 335

Ser Lys Ala Lys Gly Gln Pro Ser Gly Glu Val Gln Leu Gln Gln Ser
            340                 345                 350

Gly Pro Glu Leu Val Lys Pro Gly Ala Ser Val Lys Met Ser Cys Lys
        355                 360                 365

Ala Ser Gly Tyr Lys Phe Thr Ser Tyr Val Met His Trp Val Lys Gln
    370                 375                 380

Lys Pro Gly Gln Gly Leu Glu Trp Ile Gly Tyr Ile Asn Pro Tyr Asn
385                 390                 395                 400

Asp Val Thr Lys Tyr Asn Glu Lys Phe Lys Gly Lys Ala Thr Leu Thr
                405                 410                 415

Ser Asp Lys Ser Ser Ser Thr Ala Tyr Met Glu Leu Ser Ser Leu Thr
            420                 425                 430

Ser Glu Asp Ser Ala Val His Tyr Cys Ala Arg Gly Ser Tyr Tyr Asp
        435                 440                 445

Tyr Asp Gly Phe Val Tyr Gly Gln Gly Thr Leu Val Thr Val Ser Ser
    450                 455                 460

<210>  27
<211>  451
<212>  PRT
<213>  Artificial Sequence

<220>
<223>  FLT3 x TCR Fabv

<400>  27

Gln Val Gln Leu Gln Gln Pro Gly Ala Glu Leu Val Lys Pro Gly Ala
1               5                   10                  15

Ser Leu Lys Leu Ser Cys Lys Ser Ser Gly Tyr Thr Phe Thr Ser Tyr

20                  25                  30

Trp Met His Trp Val Arg Gln Arg Pro Gly His Gly Leu Glu Trp Ile
        35                  40                  45

Gly Glu Ile Asp Pro Ser Asp Ser Tyr Lys Asp Tyr Asn Gln Lys Phe
    50                  55                  60

Lys Asp Lys Ala Thr Leu Thr Val Asp Arg Ser Ser Asn Thr Ala Tyr
65                  70                  75                  80

Met His Leu Ser Ser Leu Thr Ser Asp Asp Ser Ala Val Tyr Tyr Cys
                85                  90                  95

Ala Arg Ala Ile Thr Thr Thr Pro Phe Asp Phe Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Ser Pro Pro Ser Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Gly Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Gln Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gln Leu Pro Ser Pro Ile Glu Lys Thr Ile
                325                 330                 335

Ser Lys Ala Lys Gly Gln Pro Ser Gly Gln Ile Val Leu Thr Gln Ser
            340                 345                 350

Pro Ala Ile Met Ser Ala Ser Pro Gly Glu Lys Val Thr Met Thr Cys
        355                 360                 365

Ser Ala Thr Ser Ser Val Ser Tyr Met His Trp Tyr Gln Gln Lys Ser
    370                 375                 380

Gly Thr Ser Pro Lys Arg Trp Ile Tyr Asp Thr Ser Lys Leu Ala Ser
385                 390                 395                 400

Gly Val Pro Ala Arg Phe Ser Gly Ser Gly Ser Gly Thr Ser Tyr Ser
                405                 410                 415

Leu Thr Ile Ser Ser Met Glu Ala Glu Asp Ala Ala Thr Tyr Tyr Cys
            420                 425                 430

Gln Gln Trp Ser Ser Asn Pro Leu Thr Phe Gly Ala Gly Thr Lys Leu
        435                 440                 445

Glu Leu Lys
    450

<210>  28
<211>  469
<212>  PRT
<213>  Artificial Sequence

<220>
<223>  FLT3 x TCR Fabv

<400>  28

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr

20                  25                  30

Gly Leu His Trp Val Arg Gln Ser Pro Gly Lys Gly Leu Glu Trp Leu
        35                  40                  45

Gly Val Ile Trp Ser Gly Gly Ser Thr Asp Tyr Asn Ala Ala Phe Ile
    50                  55                  60

Ser Arg Leu Ser Ile Ser Lys Asp Asn Ser Lys Ser Gln Val Phe Phe
65                  70                  75                  80

Lys Met Asn Ser Leu Gln Ala Asp Asp Thr Ala Ile Tyr Tyr Cys Ala
                85                  90                  95

Arg Lys Gly Gly Ile Tyr Tyr Ala Asn His Tyr Tyr Ala Met Asp Tyr
            100                 105                 110

Trp Gly Gln Gly Thr Ser Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Ser Pro Pro Ser Pro Ala Pro Pro Val
225                 230                 235                 240

Ala Gly Pro Ser Val Phe Leu Phe Pro Pro Lys Pro Lys Asp Thr Leu
                245                 250                 255

Met Ile Ser Arg Thr Pro Glu Val Thr Cys Val Val Val Gly Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Gln Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Gln Leu Pro Ser Pro
                325                 330                 335

Ile Glu Lys Thr Ile Ser Lys Ala Lys Gly Gln Pro Ser Gly Glu Val
            340                 345                 350

Gln Leu Gln Gln Ser Gly Pro Glu Leu Val Lys Pro Gly Ala Ser Val
        355                 360                 365

Lys Met Ser Cys Lys Ala Ser Gly Tyr Lys Phe Thr Ser Tyr Val Met
    370                 375                 380

His Trp Val Lys Gln Lys Pro Gly Gln Gly Leu Glu Trp Ile Gly Tyr
385                 390                 395                 400

Ile Asn Pro Tyr Asn Asp Val Thr Lys Tyr Asn Glu Lys Phe Lys Gly
                405                 410                 415

Lys Ala Thr Leu Thr Ser Asp Lys Ser Ser Ser Thr Ala Tyr Met Glu
            420                 425                 430

Leu Ser Ser Leu Thr Ser Glu Asp Ser Ala Val His Tyr Cys Ala Arg
        435                 440                 445

Gly Ser Tyr Tyr Asp Tyr Asp Gly Phe Val Tyr Gly Gln Gly Thr Leu
    450                 455                 460

Val Thr Val Ser Ser
465

<210>  29
<211>  456
<212>  PRT
<213>  Artificial Sequence

<220>
<223>  FLT3 x TCR Fabv

<400>  29

Gln Val Gln Leu Lys Gln Ser Gly Pro Gly Leu Val Gln Pro Ser Gln

1               5                   10                  15

Ser Leu Ser Ile Thr Cys Thr Val Ser Gly Phe Ser Leu Thr Asn Tyr
            20                  25                  30

Gly Leu His Trp Val Arg Gln Ser Pro Gly Lys Gly Leu Glu Trp Leu
        35                  40                  45

Gly Val Ile Trp Ser Gly Gly Ser Thr Asp Tyr Asn Ala Ala Phe Ile
    50                  55                  60

Ser Arg Leu Ser Ile Ser Lys Asp Asn Ser Lys Ser Gln Val Phe Phe
65                  70                  75                  80

Lys Met Asn Ser Leu Gln Ala Asp Asp Thr Ala Ile Tyr Tyr Cys Ala
                85                  90                  95

Arg Lys Gly Gly Ile Tyr Tyr Ala Asn His Tyr Tyr Ala Met Asp Tyr
            100                 105                 110

Trp Gly Gln Gly Thr Ser Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Ser Pro Pro Ser Pro Ala Pro Pro Val
225                 230                 235                 240

Ala Gly Pro Ser Val Phe Leu Phe Pro Pro Lys Pro Lys Asp Thr Leu
                245                 250                 255

Met Ile Ser Arg Thr Pro Glu Val Thr Cys Val Val Val Gly Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Gln Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Gln Leu Pro Ser Pro
                325                 330                 335

Ile Glu Lys Thr Ile Ser Lys Ala Lys Gly Gln Pro Ser Gly Gln Ile
            340                 345                 350

Val Leu Thr Gln Ser Pro Ala Ile Met Ser Ala Ser Pro Gly Glu Lys
        355                 360                 365

Val Thr Met Thr Cys Ser Ala Thr Ser Ser Val Ser Tyr Met His Trp
    370                 375                 380

Tyr Gln Gln Lys Ser Gly Thr Ser Pro Lys Arg Trp Ile Tyr Asp Thr
385                 390                 395                 400

Ser Lys Leu Ala Ser Gly Val Pro Ala Arg Phe Ser Gly Ser Gly Ser
                405                 410                 415

Gly Thr Ser Tyr Ser Leu Thr Ile Ser Ser Met Glu Ala Glu Asp Ala
            420                 425                 430

Ala Thr Tyr Tyr Cys Gln Gln Trp Ser Ser Asn Pro Leu Thr Phe Gly
        435                 440                 445

Ala Gly Thr Lys Leu Glu Leu Lys
    450                 455

<210>  30
<211>  457
<212>  PRT
<213>  Artificial Sequence

<220>
<223>  FLT3 x CD28 Fabv

<400>  30

Gln Val Gln Leu Gln Gln Pro Gly Ala Glu Leu Val Lys Pro Gly Ala

1               5                   10                  15

Ser Leu Lys Leu Ser Cys Lys Ser Ser Gly Tyr Thr Phe Thr Ser Tyr
            20                  25                  30

Trp Met His Trp Val Arg Gln Arg Pro Gly His Gly Leu Glu Trp Ile
        35                  40                  45

Gly Glu Ile Asp Pro Ser Asp Ser Tyr Lys Asp Tyr Asn Gln Lys Phe
    50                  55                  60

Lys Asp Lys Ala Thr Leu Thr Val Asp Arg Ser Ser Asn Thr Ala Tyr
65                  70                  75                  80

Met His Leu Ser Ser Leu Thr Ser Asp Asp Ser Ala Val Tyr Tyr Cys
                85                  90                  95

Ala Arg Ala Ile Thr Thr Thr Pro Phe Asp Phe Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Ser Pro Pro Ser Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Gly Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Gln Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gln Leu Pro Ser Pro Ile Glu Lys Thr Ile
                325                 330                 335

Ser Lys Ala Lys Gly Gln Pro Ser Gly Asp Ile Glu Leu Thr Gln Ser
            340                 345                 350

Pro Ala Ser Leu Ala Val Ser Leu Gly Gln Arg Ala Thr Ile Ser Cys
        355                 360                 365

Arg Ala Ser Glu Ser Val Glu Tyr Tyr Val Thr Ser Leu Met Gln Trp
    370                 375                 380

Tyr Gln Gln Lys Pro Gly Gln Pro Pro Lys Leu Leu Ile Phe Ala Ala
385                 390                 395                 400

Ser Asn Val Glu Ser Gly Val Pro Ala Arg Phe Ser Gly Ser Gly Ser
                405                 410                 415

Gly Thr Asn Phe Ser Leu Asn Ile His Pro Val Asp Glu Asp Asp Val
            420                 425                 430

Ala Met Tyr Phe Cys Gln Gln Ser Arg Lys Val Pro Tyr Thr Phe Gly
        435                 440                 445

Gly Gly Thr Lys Leu Glu Ile Lys Arg
    450                 455

<210>  31
<211>  465
<212>  PRT
<213>  Artificial Sequence

<220>
<223>  FLT3 x CD28 Fabv

<400>  31

Gln Val Gln Leu Gln Gln Pro Gly Ala Glu Leu Val Lys Pro Gly Ala

1               5                   10                  15

Ser Leu Lys Leu Ser Cys Lys Ser Ser Gly Tyr Thr Phe Thr Ser Tyr
            20                  25                  30

Trp Met His Trp Val Arg Gln Arg Pro Gly His Gly Leu Glu Trp Ile
        35                  40                  45

Gly Glu Ile Asp Pro Ser Asp Ser Tyr Lys Asp Tyr Asn Gln Lys Phe
    50                  55                  60

Lys Asp Lys Ala Thr Leu Thr Val Asp Arg Ser Ser Asn Thr Ala Tyr
65                  70                  75                  80

Met His Leu Ser Ser Leu Thr Ser Asp Asp Ser Ala Val Tyr Tyr Cys
                85                  90                  95

Ala Arg Ala Ile Thr Thr Thr Pro Phe Asp Phe Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Ser Pro Pro Ser Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Gly Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Gln Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gln Leu Pro Ser Pro Ile Glu Lys Thr Ile
                325                 330                 335

Ser Lys Ala Lys Gly Gln Pro Ser Gly Gln Val Lys Leu Gln Gln Ser
            340                 345                 350

Gly Pro Gly Leu Val Thr Pro Ser Gln Ser Leu Ser Ile Thr Cys Thr
        355                 360                 365

Val Ser Gly Phe Ser Leu Ser Asp Tyr Gly Val His Trp Val Arg Gln
    370                 375                 380

Ser Pro Gly Gln Gly Leu Glu Trp Leu Gly Val Ile Trp Ala Gly Gly
385                 390                 395                 400

Gly Thr Asn Tyr Asn Ser Ala Leu Met Ser Arg Lys Ser Ile Ser Lys
                405                 410                 415

Asp Asn Ser Lys Ser Gln Val Phe Leu Lys Met Asn Ser Leu Gln Ala
            420                 425                 430

Asp Asp Thr Ala Val Tyr Tyr Cys Ala Arg Asp Lys Gly Tyr Ser Tyr
        435                 440                 445

Tyr Tyr Ser Met Asp Tyr Trp Gly Gln Gly Thr Thr Val Thr Val Ser
    450                 455                 460

Ser
465

<210>  32
<211>  462
<212>  PRT
<213>  Artificial Sequence

<220>
<223>  FLT3 x CD28 Fabv

<400>  32

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Leu His Trp Val Arg Gln Ser Pro Gly Lys Gly Leu Glu Trp Leu
        35                  40                  45

Gly Val Ile Trp Ser Gly Gly Ser Thr Asp Tyr Asn Ala Ala Phe Ile
    50                  55                  60

Ser Arg Leu Ser Ile Ser Lys Asp Asn Ser Lys Ser Gln Val Phe Phe
65                  70                  75                  80

Lys Met Asn Ser Leu Gln Ala Asp Asp Thr Ala Ile Tyr Tyr Cys Ala
                85                  90                  95

Arg Lys Gly Gly Ile Tyr Tyr Ala Asn His Tyr Tyr Ala Met Asp Tyr
            100                 105                 110

Trp Gly Gln Gly Thr Ser Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Ser Pro Pro Ser Pro Ala Pro Pro Val
225                 230                 235                 240

Ala Gly Pro Ser Val Phe Leu Phe Pro Pro Lys Pro Lys Asp Thr Leu
                245                 250                 255

Met Ile Ser Arg Thr Pro Glu Val Thr Cys Val Val Val Gly Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Gln Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Gln Leu Pro Ser Pro
                325                 330                 335

Ile Glu Lys Thr Ile Ser Lys Ala Lys Gly Gln Pro Ser Gly Asp Ile
            340                 345                 350

Glu Leu Thr Gln Ser Pro Ala Ser Leu Ala Val Ser Leu Gly Gln Arg
        355                 360                 365

Ala Thr Ile Ser Cys Arg Ala Ser Glu Ser Val Glu Tyr Tyr Val Thr
    370                 375                 380

Ser Leu Met Gln Trp Tyr Gln Gln Lys Pro Gly Gln Pro Pro Lys Leu
385                 390                 395                 400

Leu Ile Phe Ala Ala Ser Asn Val Glu Ser Gly Val Pro Ala Arg Phe
                405                 410                 415

Ser Gly Ser Gly Ser Gly Thr Asn Phe Ser Leu Asn Ile His Pro Val
            420                 425                 430

Asp Glu Asp Asp Val Ala Met Tyr Phe Cys Gln Gln Ser Arg Lys Val
        435                 440                 445

Pro Tyr Thr Phe Gly Gly Gly Thr Lys Leu Glu Ile Lys Arg
    450                 455                 460

<210>  33
<211>  470
<212>  PRT
<213>  Artificial Sequence

<220>
<223>  FLT3 x CD28 Fabv

<400>  33

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Leu His Trp Val Arg Gln Ser Pro Gly Lys Gly Leu Glu Trp Leu
        35                  40                  45

Gly Val Ile Trp Ser Gly Gly Ser Thr Asp Tyr Asn Ala Ala Phe Ile
    50                  55                  60

Ser Arg Leu Ser Ile Ser Lys Asp Asn Ser Lys Ser Gln Val Phe Phe
65                  70                  75                  80

Lys Met Asn Ser Leu Gln Ala Asp Asp Thr Ala Ile Tyr Tyr Cys Ala
                85                  90                  95

Arg Lys Gly Gly Ile Tyr Tyr Ala Asn His Tyr Tyr Ala Met Asp Tyr
            100                 105                 110

Trp Gly Gln Gly Thr Ser Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Ser Pro Pro Ser Pro Ala Pro Pro Val
225                 230                 235                 240

Ala Gly Pro Ser Val Phe Leu Phe Pro Pro Lys Pro Lys Asp Thr Leu
                245                 250                 255

Met Ile Ser Arg Thr Pro Glu Val Thr Cys Val Val Val Gly Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Gln Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Gln Leu Pro Ser Pro
                325                 330                 335

Ile Glu Lys Thr Ile Ser Lys Ala Lys Gly Gln Pro Ser Gly Gln Val
            340                 345                 350

Lys Leu Gln Gln Ser Gly Pro Gly Leu Val Thr Pro Ser Gln Ser Leu
        355                 360                 365

Ser Ile Thr Cys Thr Val Ser Gly Phe Ser Leu Ser Asp Tyr Gly Val
    370                 375                 380

His Trp Val Arg Gln Ser Pro Gly Gln Gly Leu Glu Trp Leu Gly Val
385                 390                 395                 400

Ile Trp Ala Gly Gly Gly Thr Asn Tyr Asn Ser Ala Leu Met Ser Arg
                405                 410                 415

Lys Ser Ile Ser Lys Asp Asn Ser Lys Ser Gln Val Phe Leu Lys Met
            420                 425                 430

Asn Ser Leu Gln Ala Asp Asp Thr Ala Val Tyr Tyr Cys Ala Arg Asp
        435                 440                 445

Lys Gly Tyr Ser Tyr Tyr Tyr Ser Met Asp Tyr Trp Gly Gln Gly Thr
    450                 455                 460

Thr Val Thr Val Ser Ser
465                 470

<210>  34
<211>  456
<212>  PRT

<213>  Artificial Sequence

<220>
<223>  FLT3 x CD16 Fabv

<400>  34

Gln Val Gln Leu Gln Gln Pro Gly Ala Glu Leu Val Lys Pro Gly Ala
1               5                   10                  15

Ser Leu Lys Leu Ser Cys Lys Ser Ser Gly Tyr Thr Phe Thr Ser Tyr
            20                  25                  30

Trp Met His Trp Val Arg Gln Arg Pro Gly His Gly Leu Glu Trp Ile
        35                  40                  45

Gly Glu Ile Asp Pro Ser Asp Ser Tyr Lys Asp Tyr Asn Gln Lys Phe
    50                  55                  60

Lys Asp Lys Ala Thr Leu Thr Val Asp Arg Ser Ser Asn Thr Ala Tyr
65                  70                  75                  80

Met His Leu Ser Ser Leu Thr Ser Asp Asp Ser Ala Val Tyr Tyr Cys
                85                  90                  95

Ala Arg Ala Ile Thr Thr Thr Pro Phe Asp Phe Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Ser Pro Pro Ser Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Gly Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Gln Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gln Leu Pro Ser Pro Ile Glu Lys Thr Ile
                325                 330                 335

Ser Lys Ala Lys Gly Gln Pro Ser Gly Asp Ile Val Leu Thr Gln Ser
            340                 345                 350

Pro Ala Ser Leu Ala Val Ser Leu Gly Gln Arg Ala Thr Ile Ser Cys
        355                 360                 365

Lys Ala Ser Gln Ser Val Asp Phe Asp Gly Asp Ser Phe Met Asn Trp
    370                 375                 380

Tyr Gln Gln Lys Pro Gly Gln Pro Pro Lys Leu Leu Ile Tyr Thr Thr
385                 390                 395                 400

Ser Asn Leu Glu Ser Gly Ile Pro Ala Arg Phe Ser Ala Ser Gly Ser
                405                 410                 415

Gly Thr Asp Phe Thr Leu Asn Ile His Pro Val Glu Glu Glu Asp Thr
            420                 425                 430

Ala Thr Tyr Tyr Cys Gln Gln Ser Asn Glu Asp Pro Tyr Thr Phe Gly
        435                 440                 445

Gly Gly Thr Lys Leu Glu Ile Lys
    450                 455

<210>  35
<211>  463
<212>  PRT

<213>  Artificial Sequence

<220>
<223>  FLT3 x CD16 Fabv

<400>  35

Gln Val Gln Leu Gln Gln Pro Gly Ala Glu Leu Val Lys Pro Gly Ala
1               5                   10                  15

Ser Leu Lys Leu Ser Cys Lys Ser Ser Gly Tyr Thr Phe Thr Ser Tyr
            20                  25                  30

Trp Met His Trp Val Arg Gln Arg Pro Gly His Gly Leu Glu Trp Ile
        35                  40                  45

Gly Glu Ile Asp Pro Ser Asp Ser Tyr Lys Asp Tyr Asn Gln Lys Phe
    50                  55                  60

Lys Asp Lys Ala Thr Leu Thr Val Asp Arg Ser Ser Asn Thr Ala Tyr
65                  70                  75                  80

Met His Leu Ser Ser Leu Thr Ser Asp Asp Ser Ala Val Tyr Tyr Cys
                85                  90                  95

Ala Arg Ala Ile Thr Thr Thr Pro Phe Asp Phe Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Ser Pro Pro Ser Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Gly Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Gln Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gln Leu Pro Ser Pro Ile Glu Lys Thr Ile
                325                 330                 335

Ser Lys Ala Lys Gly Gln Pro Ser Gly Gln Val Thr Leu Lys Glu Ser
            340                 345                 350

Gly Pro Gly Ile Leu Gln Pro Ser Gln Thr Leu Ser Leu Thr Cys Ser
        355                 360                 365

Phe Ser Gly Phe Ser Leu Arg Thr Ser Gly Met Gly Val Gly Trp Ile
    370                 375                 380

Arg Gln Pro Ser Gly Lys Gly Leu Glu Trp Leu Ala His Ile Trp Trp
385                 390                 395                 400

Asp Asp Asp Lys Arg Tyr Asn Pro Ala Leu Lys Ser Arg Leu Thr Ile
                405                 410                 415

Ser Lys Asp Thr Ser Ser Asn Gln Val Phe Leu Lys Ile Ala Ser Val
            420                 425                 430

Asp Thr Ala Asp Thr Ala Thr Tyr Tyr Cys Ala Gln Ile Asn Pro Ala
        435                 440                 445

Trp Phe Ala Tyr Trp Gly Gln Gly Thr Leu Val Thr Val Ser Ser
    450                 455                 460

<210>  36
<211>  461
<212>  PRT

<213>  Artificial Sequence

<220>
<223>  FLT3 x CD16 Fabv

<400>  36

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Leu His Trp Val Arg Gln Ser Pro Gly Lys Gly Leu Glu Trp Leu
        35                  40                  45

Gly Val Ile Trp Ser Gly Gly Ser Thr Asp Tyr Asn Ala Ala Phe Ile
    50                  55                  60

Ser Arg Leu Ser Ile Ser Lys Asp Asn Ser Lys Ser Gln Val Phe Phe
65                  70                  75                  80

Lys Met Asn Ser Leu Gln Ala Asp Asp Thr Ala Ile Tyr Tyr Cys Ala
                85                  90                  95

Arg Lys Gly Gly Ile Tyr Tyr Ala Asn His Tyr Tyr Ala Met Asp Tyr
            100                 105                 110

Trp Gly Gln Gly Thr Ser Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Ser Pro Pro Ser Pro Ala Pro Pro Val
225                 230                 235                 240

Ala Gly Pro Ser Val Phe Leu Phe Pro Pro Lys Pro Lys Asp Thr Leu
                245                 250                 255

Met Ile Ser Arg Thr Pro Glu Val Thr Cys Val Val Val Gly Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Gln Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Gln Leu Pro Ser Pro
                325                 330                 335

Ile Glu Lys Thr Ile Ser Lys Ala Lys Gly Gln Pro Ser Gly Asp Ile
            340                 345                 350

Val Leu Thr Gln Ser Pro Ala Ser Leu Ala Val Ser Leu Gly Gln Arg
        355                 360                 365

Ala Thr Ile Ser Cys Lys Ala Ser Gln Ser Val Asp Phe Asp Gly Asp
    370                 375                 380

Ser Phe Met Asn Trp Tyr Gln Gln Lys Pro Gly Gln Pro Pro Lys Leu
385                 390                 395                 400

Leu Ile Tyr Thr Thr Ser Asn Leu Glu Ser Gly Ile Pro Ala Arg Phe
                405                 410                 415

Ser Ala Ser Gly Ser Gly Thr Asp Phe Thr Leu Asn Ile His Pro Val
            420                 425                 430

Glu Glu Glu Asp Thr Ala Thr Tyr Tyr Cys Gln Gln Ser Asn Glu Asp
        435                 440                 445

Pro Tyr Thr Phe Gly Gly Gly Thr Lys Leu Glu Ile Lys
    450                 455                 460

<210>  37
<211>  468
<212>  PRT

<213>  Artificial Sequence

<220>
<223>  FLT3 x CD16 Fabv

<400>  37

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Leu His Trp Val Arg Gln Ser Pro Gly Lys Gly Leu Glu Trp Leu
        35                  40                  45

Gly Val Ile Trp Ser Gly Gly Ser Thr Asp Tyr Asn Ala Ala Phe Ile
    50                  55                  60

Ser Arg Leu Ser Ile Ser Lys Asp Asn Ser Lys Ser Gln Val Phe Phe
65                  70                  75                  80

Lys Met Asn Ser Leu Gln Ala Asp Asp Thr Ala Ile Tyr Tyr Cys Ala
                85                  90                  95

Arg Lys Gly Gly Ile Tyr Tyr Ala Asn His Tyr Tyr Ala Met Asp Tyr
            100                 105                 110

Trp Gly Gln Gly Thr Ser Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Ser Pro Pro Ser Pro Ala Pro Pro Val
225                 230                 235                 240

Ala Gly Pro Ser Val Phe Leu Phe Pro Pro Lys Pro Lys Asp Thr Leu
                245                 250                 255

Met Ile Ser Arg Thr Pro Glu Val Thr Cys Val Val Val Gly Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Gln Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Gln Leu Pro Ser Pro
                325                 330                 335

Ile Glu Lys Thr Ile Ser Lys Ala Lys Gly Gln Pro Ser Gly Gln Val
            340                 345                 350

Thr Leu Lys Glu Ser Gly Pro Gly Ile Leu Gln Pro Ser Gln Thr Leu
        355                 360                 365

Ser Leu Thr Cys Ser Phe Ser Gly Phe Ser Leu Arg Thr Ser Gly Met
    370                 375                 380

Gly Val Gly Trp Ile Arg Gln Pro Ser Gly Lys Gly Leu Glu Trp Leu
385                 390                 395                 400

Ala His Ile Trp Trp Asp Asp Asp Lys Arg Tyr Asn Pro Ala Leu Lys
                405                 410                 415

Ser Arg Leu Thr Ile Ser Lys Asp Thr Ser Ser Asn Gln Val Phe Leu
            420                 425                 430

Lys Ile Ala Ser Val Asp Thr Ala Asp Thr Ala Thr Tyr Tyr Cys Ala
        435                 440                 445

Gln Ile Asn Pro Ala Trp Phe Ala Tyr Trp Gly Gln Gly Thr Leu Val
    450                 455                 460

Thr Val Ser Ser
465

<210>  38
<211>  455
<212>  PRT
<213>  Artificial Sequence

<220>
<223>  CD19 x CD3 Fabv

<400>  38

Glu Val Gln Leu Gln Gln Ser Gly Pro Glu Leu Ile Lys Pro Gly Ala
1               5                   10                  15

Ser Val Lys Met Ser Cys Lys Ala Ser Gly Tyr Thr Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Gly Thr Tyr Tyr Tyr Gly Ser Arg Val Phe Asp Tyr Trp Gly
            100                 105                 110

Gln Gly Thr Thr Leu Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Asp Ile Gln Met
            340                 345                 350

Thr Gln Ser Pro Ser Ser Leu Ser Ala Ser Val Gly Asp Arg Val Thr
        355                 360                 365

Ile Thr Cys Arg Ala Ser Gln Asp Ile Arg Asn Tyr Leu Asn Trp Tyr
    370                 375                 380

Gln Gln Lys Pro Gly Lys Ala Pro Lys Leu Leu Ile Tyr Tyr Thr Ser
385                 390                 395                 400

Arg Leu Glu Ser Gly Val Pro Ser Arg Phe Ser Gly Ser Gly Ser Gly
                405                 410                 415

Thr Asp Tyr Thr Leu Thr Ile Ser Ser Leu Gln Pro Glu Asp Phe Ala
            420                 425                 430

Thr Tyr Tyr Cys Gln Gln Gly Asn Thr Leu Pro Trp Thr Phe Gly Gln
        435                 440                 445

Gly Thr Lys Val Glu Ile Lys
    450                 455

<210>  39
<211>  470
<212>  PRT
<213>  Artificial Sequence

<220>
<223>  CD19 x CD3 Fabv

<400>  39

Glu Val Gln Leu Gln Gln Ser Gly Pro Glu Leu Ile Lys Pro Gly Ala
1               5                   10                  15

Ser Val Lys Met Ser Cys Lys Ala Ser Gly Tyr Thr Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Gly Thr Tyr Tyr Tyr Gly Ser Arg Val Phe Asp Tyr Trp Gly
            100                 105                 110

Gln Gly Thr Thr Leu Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Glu Val Gln Leu
            340                 345                 350

Val Glu Ser Gly Gly Gly Leu Val Gln Pro Gly Gly Ser Leu Arg Leu
        355                 360                 365

Ser Cys Ala Ala Ser Gly Tyr Ser Phe Thr Gly Tyr Thr Met Asn Trp
    370                 375                 380

Val Arg Gln Ala Pro Gly Lys Gly Leu Glu Trp Val Ala Leu Ile Asn
385                 390                 395                 400

Pro Tyr Lys Gly Val Ser Thr Tyr Asn Gln Lys Phe Lys Asp Arg Phe
                405                 410                 415

Thr Ile Ser Val Asp Lys Ser Lys Asn Thr Ala Tyr Leu Gln Met Asn
            420                 425                 430

Ser Leu Arg Ala Glu Asp Thr Ala Val Tyr Tyr Cys Ala Arg Ser Gly
        435                 440                 445

Tyr Tyr Gly Asp Ser Asp Trp Tyr Phe Asp Val Trp Gly Gln Gly Thr
    450                 455                 460

Leu Val Thr Val Ser Ser
465                 470

<210>  40
<211>  467
<212>  PRT
<213>  Artificial Sequence

<220>
<223>  CD19 x TCR Fabv

<400>  40

Glu Val Gln Leu Gln Gln Ser Gly Pro Glu Leu Ile Lys Pro Gly Ala
1               5                   10                  15

Ser Val Lys Met Ser Cys Lys Ala Ser Gly Tyr Thr Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Gly Thr Tyr Tyr Tyr Gly Ser Arg Val Phe Asp Tyr Trp Gly
            100                 105                 110

Gln Gly Thr Thr Leu Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Glu Val Gln Leu
            340                 345                 350

Gln Gln Ser Gly Pro Glu Leu Val Lys Pro Gly Ala Ser Val Lys Met
        355                 360                 365

Ser Cys Lys Ala Ser Gly Tyr Lys Phe Thr Ser Tyr Val Met His Trp
    370                 375                 380

Val Lys Gln Lys Pro Gly Gln Gly Leu Glu Trp Ile Gly Tyr Ile Asn
385                 390                 395                 400

Pro Tyr Asn Asp Val Thr Lys Tyr Asn Glu Lys Phe Lys Gly Lys Ala
                405                 410                 415

Thr Leu Thr Ser Asp Lys Ser Ser Ser Thr Ala Tyr Met Glu Leu Ser
            420                 425                 430

Ser Leu Thr Ser Glu Asp Ser Ala Val His Tyr Cys Ala Arg Gly Ser
        435                 440                 445

Tyr Tyr Asp Tyr Asp Gly Phe Val Tyr Gly Gln Gly Thr Leu Val Thr
450                 455                 460

Val Ser Ser
465

<210> 41
<211> 454
<212> PRT
<213> Artificial Sequence

<220>
<223> CD19 x TCR Fabv

<400> 41

Glu Val Gln Leu Gln Gln Ser Gly Pro Glu Leu Ile Lys Pro Gly Ala
1               5                   10                  15

Ser Val Lys Met Ser Cys Lys Ala Ser Gly Tyr Thr Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Gly Thr Tyr Tyr Tyr Gly Ser Arg Val Phe Asp Tyr Trp Gly
            100                 105                 110

Gln Gly Thr Thr Leu Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Gln Ile Val Leu
            340                 345                 350

Thr Gln Ser Pro Ala Ile Met Ser Ala Ser Pro Gly Glu Lys Val Thr
        355                 360                 365

Met Thr Cys Ser Ala Thr Ser Ser Val Ser Tyr Met His Trp Tyr Gln
    370                 375                 380

Gln Lys Ser Gly Thr Ser Pro Lys Arg Trp Ile Tyr Asp Thr Ser Lys
385                 390                 395                 400

Leu Ala Ser Gly Val Pro Ala Arg Phe Ser Gly Ser Gly Ser Gly Thr
                405                 410                 415

Ser Tyr Ser Leu Thr Ile Ser Ser Met Glu Ala Glu Asp Ala Ala Thr
            420                 425                 430

Tyr Tyr Cys Gln Gln Trp Ser Ser Asn Pro Leu Thr Phe Gly Ala Gly
        435                 440                 445

Thr Lys Leu Glu Leu Lys
    450

<210>  42
<211>  460
<212>  PRT
<213>  Artificial Sequence

<220>
<223>  CD19 x CD28 Fabv

<400>  42

Glu Val Gln Leu Gln Gln Ser Gly Pro Glu Leu Ile Lys Pro Gly Ala
1               5                   10                  15

Ser Val Lys Met Ser Cys Lys Ala Ser Gly Tyr Thr Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Gly Thr Tyr Tyr Tyr Gly Ser Arg Val Phe Asp Tyr Trp Gly
            100                 105                 110

Gln Gly Thr Thr Leu Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Asp Ile Glu Leu
            340                 345                 350

Thr Gln Ser Pro Ala Ser Leu Ala Val Ser Leu Gly Gln Arg Ala Thr
        355                 360                 365

Ile Ser Cys Arg Ala Ser Glu Ser Val Glu Tyr Tyr Val Thr Ser Leu
    370                 375                 380

Met Gln Trp Tyr Gln Gln Lys Pro Gly Gln Pro Pro Lys Leu Leu Ile
385                 390                 395                 400

Phe Ala Ala Ser Asn Val Glu Ser Gly Val Pro Ala Arg Phe Ser Gly
                405                 410                 415

Ser Gly Ser Gly Thr Asn Phe Ser Leu Asn Ile His Pro Val Asp Glu
            420                 425                 430

Asp Asp Val Ala Met Tyr Phe Cys Gln Gln Ser Arg Lys Val Pro Tyr
        435                 440                 445

Thr Phe Gly Gly Gly Thr Lys Leu Glu Ile Lys Arg
    450                 455                 460

<210>  43
<211>  468
<212>  PRT
<213>  Artificial Sequence

<220>
<223>  CD19 x CD28 Fabv

<400>  43

Glu Val Gln Leu Gln Gln Ser Gly Pro Glu Leu Ile Lys Pro Gly Ala
1               5                   10                  15

Ser Val Lys Met Ser Cys Lys Ala Ser Gly Tyr Thr Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Gly Thr Tyr Tyr Tyr Gly Ser Arg Val Phe Asp Tyr Trp Gly
            100                 105                 110

Gln Gly Thr Thr Leu Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Gln Val Lys Leu
            340                 345                 350

Gln Gln Ser Gly Pro Gly Leu Val Thr Pro Ser Gln Ser Leu Ser Ile
        355                 360                 365

Thr Cys Thr Val Ser Gly Phe Ser Leu Ser Asp Tyr Gly Val His Trp
    370                 375                 380

Val Arg Gln Ser Pro Gly Gln Gly Leu Glu Trp Leu Gly Val Ile Trp
385                 390                 395                 400

Ala Gly Gly Gly Thr Asn Tyr Asn Ser Ala Leu Met Ser Arg Lys Ser
                405                 410                 415

Ile Ser Lys Asp Asn Ser Lys Ser Gln Val Phe Leu Lys Met Asn Ser
            420                 425                 430

Leu Gln Ala Asp Asp Thr Ala Val Tyr Tyr Cys Ala Arg Asp Lys Gly
        435                 440                 445

Tyr Ser Tyr Tyr Tyr Ser Met Asp Tyr Trp Gly Gln Gly Thr Thr Val
    450                 455                 460

Thr Val Ser Ser
465

<210>  44
<211>  459
<212>  PRT
<213>  Artificial Sequence

<220>
<223>  CD19 x CD16 Fabv

<400>  44

Glu Val Gln Leu Gln Gln Ser Gly Pro Glu Leu Ile Lys Pro Gly Ala
1               5                   10                  15

Ser Val Lys Met Ser Cys Lys Ala Ser Gly Tyr Thr Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Gly Thr Tyr Tyr Tyr Gly Ser Arg Val Phe Asp Tyr Trp Gly
            100                 105                 110

Gln Gly Thr Thr Leu Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Asp Ile Val Leu
            340                 345                 350

Thr Gln Ser Pro Ala Ser Leu Ala Val Ser Leu Gly Gln Arg Ala Thr
        355                 360                 365

Ile Ser Cys Lys Ala Ser Gln Ser Val Asp Phe Asp Gly Asp Ser Phe
    370                 375                 380

Met Asn Trp Tyr Gln Gln Lys Pro Gly Gln Pro Pro Lys Leu Leu Ile
385                 390                 395                 400

Tyr Thr Thr Ser Asn Leu Glu Ser Gly Ile Pro Ala Arg Phe Ser Ala
                405                 410                 415

Ser Gly Ser Gly Thr Asp Phe Thr Leu Asn Ile His Pro Val Glu Glu
            420                 425                 430

Glu Asp Thr Ala Thr Tyr Tyr Cys Gln Gln Ser Asn Glu Asp Pro Tyr
        435                 440                 445

Thr Phe Gly Gly Gly Thr Lys Leu Glu Ile Lys
    450                 455

<210>  45
<211>  466
<212>  PRT
<213>  Artificial Sequence

<220>
<223>  CD19 x CD16 Fabv

<400>  45

Glu Val Gln Leu Gln Gln Ser Gly Pro Glu Leu Ile Lys Pro Gly Ala
1               5                   10                  15

Ser Val Lys Met Ser Cys Lys Ala Ser Gly Tyr Thr Phe Thr Ser Tyr
            20                  25                  30

Val Met His Trp Val Lys Gln Lys Pro Gly Gln Gly Leu Glu Trp Ile
        35                  40                  45

Gly Tyr Ile Asn Pro Tyr Asn Asp Gly Thr Lys Tyr Asn Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Gly Thr Tyr Tyr Tyr Gly Ser Arg Val Phe Asp Tyr Trp Gly
            100                 105                 110

Gln Gly Thr Thr Leu Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Gln Val Thr Leu
            340                 345                 350

Lys Glu Ser Gly Pro Gly Ile Leu Gln Pro Ser Gln Thr Leu Ser Leu
        355                 360                 365

Thr Cys Ser Phe Ser Gly Phe Ser Leu Arg Thr Ser Gly Met Gly Val
    370                 375                 380

Gly Trp Ile Arg Gln Pro Ser Gly Lys Gly Leu Glu Trp Leu Ala His
385                 390                 395                 400

Ile Trp Trp Asp Asp Asp Lys Arg Tyr Asn Pro Ala Leu Lys Ser Arg
                405                 410                 415

Leu Thr Ile Ser Lys Asp Thr Ser Ser Asn Gln Val Phe Leu Lys Ile
            420                 425                 430

Ala Ser Val Asp Thr Ala Asp Thr Ala Thr Tyr Tyr Cys Ala Gln Ile
        435                 440                 445

Asn Pro Ala Trp Phe Ala Tyr Trp Gly Gln Gly Thr Leu Val Thr Val
    450                 455                 460

Ser Ser
465

<210> 46
<211> 455
<212> PRT
<213> Artificial Sequence

<220>
<223> CSPG4 x CD3 Fabv

<400> 46

Gln Val Lys Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Asp Ile Gln Met
            340                 345                 350

Thr Gln Ser Pro Ser Ser Leu Ser Ala Ser Val Gly Asp Arg Val Thr
        355                 360                 365

Ile Thr Cys Arg Ala Ser Gln Asp Ile Arg Asn Tyr Leu Asn Trp Tyr
    370                 375                 380

Gln Gln Lys Pro Gly Lys Ala Pro Lys Leu Leu Ile Tyr Tyr Thr Ser
385                 390                 395                 400

Arg Leu Glu Ser Gly Val Pro Ser Arg Phe Ser Gly Ser Gly Ser Gly
                405                 410                 415

Thr Asp Tyr Thr Leu Thr Ile Ser Ser Leu Gln Pro Glu Asp Phe Ala
            420                 425                 430

Thr Tyr Tyr Cys Gln Gln Gly Asn Thr Leu Pro Trp Thr Phe Gly Gln
        435                 440                 445

Gly Thr Lys Val Glu Ile Lys
    450                 455

<210>  47
<211>  470
<212>  PRT
<213>  Artificial Sequence

<220>
<223>  CSPG4 x CD3 Fabv

<400>  47

Gln Val Lys Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Glu Val Gln Leu
            340                 345                 350

Val Glu Ser Gly Gly Gly Leu Val Gln Pro Gly Gly Ser Leu Arg Leu
        355                 360                 365

Ser Cys Ala Ala Ser Gly Tyr Ser Phe Thr Gly Tyr Thr Met Asn Trp
    370                 375                 380

Val Arg Gln Ala Pro Gly Lys Gly Leu Glu Trp Val Ala Leu Ile Asn
385                 390                 395                 400

Pro Tyr Lys Gly Val Ser Thr Tyr Asn Gln Lys Phe Lys Asp Arg Phe
                405                 410                 415

Thr Ile Ser Val Asp Lys Ser Lys Asn Thr Ala Tyr Leu Gln Met Asn
            420                 425                 430

Ser Leu Arg Ala Glu Asp Thr Ala Val Tyr Tyr Cys Ala Arg Ser Gly
        435                 440                 445

Tyr Tyr Gly Asp Ser Asp Trp Tyr Phe Asp Val Trp Gly Gln Gly Thr
    450                 455                 460

Leu Val Thr Val Ser Ser
465                 470

<210>  48
<211>  467
<212>  PRT
<213>  Artificial Sequence

<220>
<223>  CSPG4 x TCR Fabv

<400>  48

Gln Val Lys Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Glu Val Gln Leu
            340                 345                 350

Gln Gln Ser Gly Pro Glu Leu Val Lys Pro Gly Ala Ser Val Lys Met
        355                 360                 365

Ser Cys Lys Ala Ser Gly Tyr Lys Phe Thr Ser Tyr Val Met His Trp
    370                 375                 380

Val Lys Gln Lys Pro Gly Gln Gly Leu Glu Trp Ile Gly Tyr Ile Asn
385                 390                 395                 400

Pro Tyr Asn Asp Val Thr Lys Tyr Asn Glu Lys Phe Lys Gly Lys Ala
                405                 410                 415

Thr Leu Thr Ser Asp Lys Ser Ser Ser Thr Ala Tyr Met Glu Leu Ser
            420                 425                 430

Ser Leu Thr Ser Glu Asp Ser Ala Val His Tyr Cys Ala Arg Gly Ser
        435                 440                 445

Tyr Tyr Asp Tyr Asp Gly Phe Val Tyr Gly Gln Gly Thr Leu Val Thr
    450                 455                 460

Val Ser Ser
465

<210>  49
<211>  454
<212>  PRT
<213>  Artificial Sequence

<220>
<223>  CSPG4 x TCR Fabv

<400>  49

Gln Val Lys Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Gln Ile Val Leu
            340                 345                 350

Thr Gln Ser Pro Ala Ile Met Ser Ala Ser Pro Gly Glu Lys Val Thr
        355                 360                 365

Met Thr Cys Ser Ala Thr Ser Ser Val Ser Tyr Met His Trp Tyr Gln
    370                 375                 380

Gln Lys Ser Gly Thr Ser Pro Lys Arg Trp Ile Tyr Asp Thr Ser Lys
385                 390                 395                 400

Leu Ala Ser Gly Val Pro Ala Arg Phe Ser Gly Ser Gly Ser Gly Thr
                405                 410                 415

Ser Tyr Ser Leu Thr Ile Ser Ser Met Glu Ala Glu Asp Ala Ala Thr
            420                 425                 430

Tyr Tyr Cys Gln Gln Trp Ser Ser Asn Pro Leu Thr Phe Gly Ala Gly
        435                 440                 445

Thr Lys Leu Glu Leu Lys
    450

<210>  50
<211>  460
<212>  PRT
<213>  Artificial Sequence

<220>
<223>  CSPG4 x CD28 Fabv

<400>  50

Gln Val Lys Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Asp Ile Glu Leu
            340                 345                 350

Thr Gln Ser Pro Ala Ser Leu Ala Val Ser Leu Gly Gln Arg Ala Thr
        355                 360                 365

Ile Ser Cys Arg Ala Ser Glu Ser Val Glu Tyr Tyr Val Thr Ser Leu
    370                 375                 380

Met Gln Trp Tyr Gln Gln Lys Pro Gly Gln Pro Pro Lys Leu Leu Ile
385                 390                 395                 400

Phe Ala Ala Ser Asn Val Glu Ser Gly Val Pro Ala Arg Phe Ser Gly
                405                 410                 415

Ser Gly Ser Gly Thr Asn Phe Ser Leu Asn Ile His Pro Val Asp Glu
            420                 425                 430

Asp Asp Val Ala Met Tyr Phe Cys Gln Gln Ser Arg Lys Val Pro Tyr
        435                 440                 445

Thr Phe Gly Gly Gly Thr Lys Leu Glu Ile Lys Arg
    450                 455                 460

<210>  51
<211>  468
<212>  PRT
<213>  Artificial Sequence

<220>
<223>  CSPG4 x CD28 Fabv

<400>  51

Gln Val Lys Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Gln Val Lys Leu
            340                 345                 350

Gln Gln Ser Gly Pro Gly Leu Val Thr Pro Ser Gln Ser Leu Ser Ile
        355                 360                 365

Thr Cys Thr Val Ser Gly Phe Ser Leu Ser Asp Tyr Gly Val His Trp
    370                 375                 380

Val Arg Gln Ser Pro Gly Gln Gly Leu Glu Trp Leu Gly Val Ile Trp
385                 390                 395                 400

Ala Gly Gly Gly Thr Asn Tyr Asn Ser Ala Leu Met Ser Arg Lys Ser
                405                 410                 415

Ile Ser Lys Asp Asn Ser Lys Ser Gln Val Phe Leu Lys Met Asn Ser
            420                 425                 430

Leu Gln Ala Asp Asp Thr Ala Val Tyr Tyr Cys Ala Arg Asp Lys Gly
        435                 440                 445

Tyr Ser Tyr Tyr Tyr Ser Met Asp Tyr Trp Gly Gln Gly Thr Thr Val
    450                 455                 460

Thr Val Ser Ser
465

<210>  52
<211>  459
<212>  PRT
<213>  Artificial Sequence

<220>
<223>  CSPG4 x CD16 Fabv

<400>  52

Gln Val Lys Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Asp Ile Val Leu
            340                 345                 350

Thr Gln Ser Pro Ala Ser Leu Ala Val Ser Leu Gly Gln Arg Ala Thr
        355                 360                 365

Ile Ser Cys Lys Ala Ser Gln Ser Val Asp Phe Asp Gly Asp Ser Phe
    370                 375                 380

Met Asn Trp Tyr Gln Gln Lys Pro Gly Gln Pro Pro Lys Leu Leu Ile
385                 390                 395                 400

Tyr Thr Thr Ser Asn Leu Glu Ser Gly Ile Pro Ala Arg Phe Ser Ala
                405                 410                 415

Ser Gly Ser Gly Thr Asp Phe Thr Leu Asn Ile His Pro Val Glu Glu
            420                 425                 430

Glu Asp Thr Ala Thr Tyr Tyr Cys Gln Gln Ser Asn Glu Asp Pro Tyr
        435                 440                 445

Thr Phe Gly Gly Gly Thr Lys Leu Glu Ile Lys
    450                 455

<210>  53
<211>  466
<212>  PRT
<213>  Artificial Sequence

<220>
<223>  CSPG4 x CD16 Fabv

<400>  53

Gln Val Lys Leu Gln Gln Ser Gly Pro Glu Leu Val Lys Pro Gly Ala
1               5                   10                  15

Ser Val Lys Ile Ser Cys Lys Ala Ser Gly Tyr Ala Phe Ser Arg Ser
            20                  25                  30

Trp Met Asn Trp Val Lys Gln Arg Pro Gly Gln Gly Leu Glu Trp Ile
        35                  40                  45

Gly Arg Ile Tyr Pro Gly Asp Gly Asp Thr Asn Tyr Asn Gly Lys Phe
    50                  55                  60

Lys Gly Lys Ala Thr Leu Thr Ala Asp Lys Ser Ser Ser Thr Ala Tyr
65                  70                  75                  80

Met Gln Val Ser Ser Leu Thr Ser Val Asp Ser Ala Val Tyr Phe Cys
                85                  90                  95

Ala Arg Gly Asn Thr Val Val Val Pro Tyr Thr Met Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Ser Pro Pro Ser Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Gly Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Gln Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gln Leu Pro Ser Pro Ile Glu
                325                 330                 335

Lys Thr Ile Ser Lys Ala Lys Gly Gln Pro Ser Gly Gln Val Thr Leu
            340                 345                 350

Lys Glu Ser Gly Pro Gly Ile Leu Gln Pro Ser Gln Thr Leu Ser Leu
        355                 360                 365

Thr Cys Ser Phe Ser Gly Phe Ser Leu Arg Thr Ser Gly Met Gly Val
    370                 375                 380

Gly Trp Ile Arg Gln Pro Ser Gly Lys Gly Leu Glu Trp Leu Ala His
385                 390                 395                 400

Ile Trp Trp Asp Asp Asp Lys Arg Tyr Asn Pro Ala Leu Lys Ser Arg
                405                 410                 415

Leu Thr Ile Ser Lys Asp Thr Ser Ser Asn Gln Val Phe Leu Lys Ile
            420                 425                 430

Ala Ser Val Asp Thr Ala Asp Thr Ala Thr Tyr Tyr Cys Ala Gln Ile
        435                 440                 445

Asn Pro Ala Trp Phe Ala Tyr Trp Gly Gln Gly Thr Leu Val Thr Val
    450                 455                 460

Ser Ser
465

<210>  54
<211>  453
<212>  PRT
<213>  Artificial Sequence

<220>
<223>  EGFR x CD3 Fabv

<400>  54

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Gly Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Gln Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gln Leu Pro Ser Pro Ile Glu Lys Thr
                325                 330                 335

Ile Ser Lys Ala Lys Gly Gln Pro Ser Gly Asp Ile Gln Met Thr Gln
            340                 345                 350

Ser Pro Ser Ser Leu Ser Ala Ser Val Gly Asp Arg Val Thr Ile Thr
        355                 360                 365

Cys Arg Ala Ser Gln Asp Ile Arg Asn Tyr Leu Asn Trp Tyr Gln Gln
    370                 375                 380

Lys Pro Gly Lys Ala Pro Lys Leu Leu Ile Tyr Tyr Thr Ser Arg Leu
385                 390                 395                 400

Glu Ser Gly Val Pro Ser Arg Phe Ser Gly Ser Gly Ser Gly Thr Asp
                405                 410                 415

Tyr Thr Leu Thr Ile Ser Ser Leu Gln Pro Glu Asp Phe Ala Thr Tyr
            420                 425                 430

Tyr Cys Gln Gln Gly Asn Thr Leu Pro Trp Thr Phe Gly Gln Gly Thr
        435                 440                 445

Lys Val Glu Ile Lys
    450

<210>  55
<211>  468
<212>  PRT
<213>  Artificial Sequence

<220>
<223>  EGFR x CD3 Fabv

<400>  55

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Gly Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Gln Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gln Leu Pro Ser Pro Ile Glu Lys Thr
                325                 330                 335

Ile Ser Lys Ala Lys Gly Gln Pro Ser Gly Glu Val Gln Leu Val Glu
            340                 345                 350

Ser Gly Gly Gly Leu Val Gln Pro Gly Gly Ser Leu Arg Leu Ser Cys
        355                 360                 365

Ala Ala Ser Gly Tyr Ser Phe Thr Gly Tyr Thr Met Asn Trp Val Arg
    370                 375                 380

Gln Ala Pro Gly Lys Gly Leu Glu Trp Val Ala Leu Ile Asn Pro Tyr
385                 390                 395                 400

Lys Gly Val Ser Thr Tyr Asn Gln Lys Phe Lys Asp Arg Phe Thr Ile
                405                 410                 415

Ser Val Asp Lys Ser Lys Asn Thr Ala Tyr Leu Gln Met Asn Ser Leu
            420                 425                 430

Arg Ala Glu Asp Thr Ala Val Tyr Tyr Cys Ala Arg Ser Gly Tyr Tyr
        435                 440                 445

Gly Asp Ser Asp Trp Tyr Phe Asp Val Trp Gly Gln Gly Thr Leu Val
    450                 455                 460

Thr Val Ser Ser
465

<210>  56
<211>  465
<212>  PRT
<213>  Artificial Sequence

<220>
<223>  EGFR x TCR Fabv

<400>  56

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Gly Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Gln Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gln Leu Pro Ser Pro Ile Glu Lys Thr
                325                 330                 335

Ile Ser Lys Ala Lys Gly Gln Pro Ser Gly Glu Val Gln Leu Gln Gln
            340                 345                 350

Ser Gly Pro Glu Leu Val Lys Pro Gly Ala Ser Val Lys Met Ser Cys
        355                 360                 365

Lys Ala Ser Gly Tyr Lys Phe Thr Ser Tyr Val Met His Trp Val Lys
    370                 375                 380

Gln Lys Pro Gly Gln Gly Leu Glu Trp Ile Gly Tyr Ile Asn Pro Tyr
385                 390                 395                 400

Asn Asp Val Thr Lys Tyr Asn Glu Lys Phe Lys Gly Lys Ala Thr Leu
                405                 410                 415

Thr Ser Asp Lys Ser Ser Ser Thr Ala Tyr Met Glu Leu Ser Ser Leu
            420                 425                 430

Thr Ser Glu Asp Ser Ala Val His Tyr Cys Ala Arg Gly Ser Tyr Tyr
        435                 440                 445

Asp Tyr Asp Gly Phe Val Tyr Gly Gln Gly Thr Leu Val Thr Val Ser
    450                 455                 460

Ser
465

<210>  57
<211>  452
<212>  PRT
<213>  Artificial Sequence

<220>
<223>  EGFR x TCR Fabv

<400>  57

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Gly Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Gln Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gln Leu Pro Ser Pro Ile Glu Lys Thr
                325                 330                 335

Ile Ser Lys Ala Lys Gly Gln Pro Ser Gly Gln Ile Val Leu Thr Gln
            340                 345                 350

Ser Pro Ala Ile Met Ser Ala Ser Pro Gly Glu Lys Val Thr Met Thr
        355                 360                 365

Cys Ser Ala Thr Ser Ser Val Ser Tyr Met His Trp Tyr Gln Gln Lys
    370                 375                 380

Ser Gly Thr Ser Pro Lys Arg Trp Ile Tyr Asp Thr Ser Lys Leu Ala
385                 390                 395                 400

Ser Gly Val Pro Ala Arg Phe Ser Gly Ser Gly Ser Gly Thr Ser Tyr
                405                 410                 415

Ser Leu Thr Ile Ser Ser Met Glu Ala Glu Asp Ala Ala Thr Tyr Tyr
            420                 425                 430

Cys Gln Gln Trp Ser Ser Asn Pro Leu Thr Phe Gly Ala Gly Thr Lys
        435                 440                 445

Leu Glu Leu Lys
    450

<210>  58
<211>  458
<212>  PRT
<213>  Artificial Sequence

<220>
<223>  EGFR x CD28 Fabv

<400>  58

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Gly Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Gln Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gln Leu Pro Ser Pro Ile Glu Lys Thr
                325                 330                 335

Ile Ser Lys Ala Lys Gly Gln Pro Ser Gly Asp Ile Glu Leu Thr Gln
            340                 345                 350

Ser Pro Ala Ser Leu Ala Val Ser Leu Gly Gln Arg Ala Thr Ile Ser
        355                 360                 365

Cys Arg Ala Ser Glu Ser Val Glu Tyr Tyr Val Thr Ser Leu Met Gln
    370                 375                 380

Trp Tyr Gln Gln Lys Pro Gly Gln Pro Pro Lys Leu Leu Ile Phe Ala
385                 390                 395                 400

Ala Ser Asn Val Glu Ser Gly Val Pro Ala Arg Phe Ser Gly Ser Gly
                405                 410                 415

Ser Gly Thr Asn Phe Ser Leu Asn Ile His Pro Val Asp Glu Asp Asp
            420                 425                 430

Val Ala Met Tyr Phe Cys Gln Gln Ser Arg Lys Val Pro Tyr Thr Phe
        435                 440                 445

Gly Gly Gly Thr Lys Leu Glu Ile Lys Arg
    450                 455

<210>  59
<211>  466
<212>  PRT
<213>  Artificial Sequence

<220>
<223>  EGFR x CD28 Fabv

<400>  59

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Gly Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Gln Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gln Leu Pro Ser Pro Ile Glu Lys Thr
                325                 330                 335

Ile Ser Lys Ala Lys Gly Gln Pro Ser Gly Gln Val Lys Leu Gln Gln
            340                 345                 350

Ser Gly Pro Gly Leu Val Thr Pro Ser Gln Ser Leu Ser Ile Thr Cys
        355                 360                 365

Thr Val Ser Gly Phe Ser Leu Ser Asp Tyr Gly Val His Trp Val Arg
    370                 375                 380

Gln Ser Pro Gly Gln Gly Leu Glu Trp Leu Gly Val Ile Trp Ala Gly
385                 390                 395                 400

Gly Gly Thr Asn Tyr Asn Ser Ala Leu Met Ser Arg Lys Ser Ile Ser
                405                 410                 415

Lys Asp Asn Ser Lys Ser Gln Val Phe Leu Lys Met Asn Ser Leu Gln
            420                 425                 430

Ala Asp Asp Thr Ala Val Tyr Tyr Cys Ala Arg Asp Lys Gly Tyr Ser
        435                 440                 445

Tyr Tyr Tyr Ser Met Asp Tyr Trp Gly Gln Gly Thr Thr Val Thr Val
    450                 455                 460

Ser Ser
465

<210>  60
<211>  457
<212>  PRT
<213>  Artificial Sequence

<220>
<223>  EGFR x CD16 Fabv

<400>  60

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Gly Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Gln Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gln Leu Pro Ser Pro Ile Glu Lys Thr
                325                 330                 335

Ile Ser Lys Ala Lys Gly Gln Pro Ser Gly Asp Ile Val Leu Thr Gln
            340                 345                 350

Ser Pro Ala Ser Leu Ala Val Ser Leu Gly Gln Arg Ala Thr Ile Ser
        355                 360                 365

Cys Lys Ala Ser Gln Ser Val Asp Phe Asp Gly Asp Ser Phe Met Asn
    370                 375                 380

Trp Tyr Gln Gln Lys Pro Gly Gln Pro Pro Lys Leu Leu Ile Tyr Thr
385                 390                 395                 400

Thr Ser Asn Leu Glu Ser Gly Ile Pro Ala Arg Phe Ser Ala Ser Gly
                405                 410                 415

Ser Gly Thr Asp Phe Thr Leu Asn Ile His Pro Val Glu Glu Glu Asp
            420                 425                 430

Thr Ala Thr Tyr Tyr Cys Gln Gln Ser Asn Glu Asp Pro Tyr Thr Phe
        435                 440                 445

Gly Gly Gly Thr Lys Leu Glu Ile Lys
    450                 455

<210>  61
<211>  464
<212>  PRT
<213>  Artificial Sequence

<220>
<223>  EGFR x CD16 Fabv

<400>  61

Gln Val Gln Leu Lys Gln Ser Gly Pro Gly Leu Val Gln Pro Ser Gln
1               5                   10                  15

Ser Leu Ser Ile Thr Cys Thr Val Ser Gly Phe Ser Leu Thr Asn Tyr
            20                  25                  30

Gly Val His Trp Val Arg Gln Ser Pro Gly Lys Gly Leu Glu Trp Leu
        35                  40                  45

Gly Val Ile Trp Ser Gly Gly Asn Thr Asp Tyr Asn Thr Pro Phe Thr
    50                  55                  60

Ser Arg Leu Ser Ile Asn Lys Asp Asn Ser Lys Ser Gln Val Phe Phe
65                  70                  75                  80

Lys Met Asn Ser Leu Gln Ser Asn Asp Thr Ala Ile Tyr Tyr Cys Ala
                85                  90                  95

Arg Ala Leu Thr Tyr Tyr Asp Tyr Glu Phe Ala Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Ser Pro Pro Ser Pro Ala Pro Pro Val Ala Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Gly Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Gln Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gln Leu Pro Ser Pro Ile Glu Lys Thr
                325                 330                 335

Ile Ser Lys Ala Lys Gly Gln Pro Ser Gly Gln Val Thr Leu Lys Glu
            340                 345                 350

Ser Gly Pro Gly Ile Leu Gln Pro Ser Gln Thr Leu Ser Leu Thr Cys
        355                 360                 365

Ser Phe Ser Gly Phe Ser Leu Arg Thr Ser Gly Met Gly Val Gly Trp
    370                 375                 380

Ile Arg Gln Pro Ser Gly Lys Gly Leu Glu Trp Leu Ala His Ile Trp
385                 390                 395                 400

Trp Asp Asp Asp Lys Arg Tyr Asn Pro Ala Leu Lys Ser Arg Leu Thr
                405                 410                 415

Ile Ser Lys Asp Thr Ser Ser Asn Gln Val Phe Leu Lys Ile Ala Ser
            420                 425                 430

Val Asp Thr Ala Asp Thr Ala Thr Tyr Tyr Cys Ala Gln Ile Asn Pro
        435                 440                 445

Ala Trp Phe Ala Tyr Trp Gly Gln Gly Thr Leu Val Thr Val Ser Ser
    450                 455                 460

<210>  62
<211>  106
<212>  PRT
<213>  Artificial Sequence

<220>
<223>  CD3 single variable domain (clone OKT3) VL

<400>  62

Asp Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

Asn Trp Tyr Gln Gln Lys Ser Gly Thr Ser Pro Lys Arg Trp Ile Tyr
        35                  40                  45

Asp Thr Ser Lys Leu Ala Ser Gly Val Pro Ala His Phe Arg Gly Ser
    50                  55                  60

Gly Ser Gly Thr Ser Tyr Ser Leu Thr Ile Ser Gly Met Glu Ala Glu
65                  70                  75                  80

Asp Ala Ala Thr Tyr Tyr Cys Gln Gln Trp Ser Ser Asn Pro Phe Thr
                85                  90                  95

Phe Gly Ser Gly Thr Lys Leu Glu Ile Asn
            100                 105

<210>  63
<211>  119
<212>  PRT
<213>  Artificial Sequence

<220>
<223>  CD3 single variable domain (clone OKT3) VH

<400>  63

<210>  64

<211>  49
<212>  DNA
<213>  Artificial Sequence

<220>
<223>  4G7-H-for

<400>  64
ctcttcacag gtgtcctctc tgaggtccag ctgcagcagt ctggacctg                49


<210>  65
<211>  59
<212>  DNA
<213>  Artificial Sequence

<220>
<223>  4G7-H-rev

<400>  65
gggagaaggt aggactcacc tgaggagact gtgagagtgg tgccttggcc ccagtagtc      59


<210>  66
<211>  51
<212>  DNA
<213>  Artificial Sequence

<220>
<223>  9.2.27-H-for

<400>  66
tcttcacagg tgtcctctcc caggtgaagc tgcagcaatc tggacctgag c              51


<210>  67
<211>  51
<212>  DNA
<213>  Artificial Sequence

<220>
<223>  9.2.27-H-rev

<400>  67
aatgggagaa ggtaggactc acctgaggag acggtgaccg tggtcccttg g              51


<210>  68
<211>  53
<212>  DNA
<213>  Artificial Sequence

<220>
<223>  4G8-H-for

<400>  68
tctcttcaca ggtgtcctct ctcaggtcca actgcagcag cctggggctg agc            53


<210>  69
<211>  52
<212>  DNA
<213>  Artificial Sequence

<220>
<223>  4G8-H-rev

<400>  69
gagaaggtag gactcacctg aggagactgt gagagtggtg ccttggcccc ag          52


<210>  70
<211>  60
<212>  DNA
<213>  Artificial Sequence

<220>
<223>  BV10-H-for

<400>  70
agacgtccac tctgtctttc tcttcacagg tgtcctctcc caggtgcagc tgaagcagtc      60


<210>  71
<211>  49
<212>  DNA
<213>  Artificial Sequence

<220>
<223>  BV10-H-rev

<400>  71
gagaaggtag gactcacctg aggagacggt gactgaggtt ccttgaccc          49


<210>  72
<211>  40
<212>  DNA
<213>  Artificial Sequence

<220>
<223>  universal for (AatII)

<400>  72
agacgtccac tctgtctttc tcttcacagg tgtcctctcc          40


<210>  73
<211>  33
<212>  DNA
<213>  Artificial Sequence

<220>
<223>  universal rev (ClaI)

<400>  73
tatcgattta gaatgggaga aggtaggact cac          33


<210>  74
<211>  43
<212>  DNA
<213>  Artificial Sequence

<220>
<223>  4G7-L-for (XhoI)

<400>  74
actcgaggag atattgtgat gactcaggct gcaccctcta tac                43

<210>  75
<211>  48
<212>  DNA
<213>  Artificial Sequence

<220>
<223>  4G7-L-rev (SpeI)

<400>  75
aactagtact tacgtttcag ctccagcttg gtcccagcac cgaacgtg           48

<210>  76
<211>  42
<212>  DNA
<213>  Artificial Sequence

<220>
<223>  9.2.27-L-for (XhoI)

<400>  76
tctcgaggag acatcgagct cactcagtct ccagcttctt tg                 42

<210>  77
<211>  46
<212>  DNA
<213>  Artificial Sequence

<220>
<223>  9.2.27-L-rev (SpeI)

<400>  77
aactagtact tacgtttgat ctccagcttg gtgccccctc caaagg              46

<210>  78
<211>  42
<212>  DNA
<213>  Artificial Sequence

<220>
<223>  4G8-L-for (XhoI)

<400>  78
actcgaggag atattgtgct aactcagtct ccagccaccc tg                 42

<210>  79
<211>  41
<212>  DNA
<213>  Artificial Sequence

<220>
<223>  4G8-L-rev (SpeI)

<400>  79
tactagtact tacgttttat ttccagcttg gtcccccctc c                  41

<210>  80
<211>  40
<212>  DNA
<213>  Artificial Sequence

<220>
<223>  BV10-L-for (XhoI)

<400>  80
actcgaggag acattgtgat gacacagtct ccatcctccc                              40


<210>  81
<211>  47
<212>  DNA
<213>  Artificial Sequence

<220>
<223>  BV10-L-rev (SpeI)

<400>  81
actagtactt acgtttcagc tccagcttgg tcccagcacc gaacgtg                      47


<210>  82
<211>  47
<212>  DNA
<213>  Artificial Sequence

<220>
<223>  UCHT1-H-for (BspEI)

<400>  82
ttccggagag gttcagctgg tggagtctgg cggtggcctg gtgcagc                      47


<210>  83
<211>  48
<212>  DNA
<213>  Artificial Sequence

<220>
<223>  UCHT1-H-rev (SpeI)

<400>  83
tactagttat cacgaggaga cggtgaccag ggttccttga ccccagac                     48


<210>  84
<211>  42
<212>  DNA
<213>  Artificial Sequence

<220>
<223>  UCHT1-L-for (BspEI)

<400>  84
ttccggagat atccagatga cccagtcccc gagctccctg tc                           42


<210>  85

<211>  49
<212>  DNA
<213>  Artificial Sequence

<220>
<223>  UCHT1-L-rev (SpeI)

<400>  85
tactagttat catttgatct ccaccttggt gccctgtccg aacgtccac                49


<210>  86
<211>  27
<212>  DNA
<213>  Artificial Sequence

<220>
<223>  BMA031-H-for (BspEI)

<400>  86
ttccggagaa gtgcagctgc agcagtc                                        27


<210>  87
<211>  35
<212>  DNA
<213>  Artificial Sequence

<220>
<223>  BMA031-H-rev SpeI

<400>  87
tactagttat cagctagaca cggtgaccag agtgc                               35


<210>  88
<211>  27
<212>  DNA
<213>  Artificial Sequence

<220>
<223>  BMA031-L-for (BspEI)

<400>  88
ttccggacag atcgtgctga cccagtc                                        27


<210>  89
<211>  25
<212>  DNA
<213>  Artificial Sequence

<220>
<223>  BMA031-L-rev (SpeI)

<400>  89
tactagttat cacttcagtt ccagc                                          25


<210>  90
<211>  26
<212>  DNA
<213>  Artificial Sequence

<220>
<223>  OKT3-H-for (BspEI)

<400>  90
ttccggacag gtgcagctgc agcagt                                          26


<210>  91
<211>  35
<212>  DNA
<213>  Artificial Sequence

<220>
<223>  OKT3-H-rev (SpeI)

<400>  91
tactagttat catgaggaga cggtgagcgt ggtcc                                35


<210>  92
<211>  25
<212>  DNA
<213>  Artificial Sequence

<220>
<223>  OKT3-L-for (BspEI)

<400>  92
ttccggagac attgtgctca cccag                                           25


<210>  93
<211>  32
<212>  DNA
<213>  Artificial Sequence

<220>
<223>  OKT3-L-rev (SpeI)

<400>  93
tactagttat cagtttattt ccaactttgt cc                                   32


<210>  94
<211>  47
<212>  DNA
<213>  Artificial Sequence

<220>
<223>  9.3-for (BspEI)

<400>  94
ttccggacag gtgaagctgc agcagtctgg ccctggcctg gtgaccc                   47


<210>  95
<211>  48
<212>  DNA
<213>  Artificial Sequence

<220>
<223>  9.3-H-rev (SpeI)

<400>  95
tactagttat caagagctca cagtcactgt ggtgccctgg ccccagta                    48


<210>  96
<211>  41
<212>  DNA
<213>  Artificial Sequence

<220>
<223>  9.3-L-for (BspEI)

<400>  96
ttccggagac attgtgctga cccagtcccc tgcctccctg g                           41


<210>  97
<211>  49
<212>  DNA
<213>  Artificial Sequence

<220>
<223>  9.3-L-rev (SpeI)

<400>  97
tactagttat cacctcttga tctccagctt ggtgccccct ccaaaggtg                   49


<210>  98
<211>  11
<212>  PRT
<213>  Artificial Sequence

<220>
<223>  parts of the hinge region and CH2 region

<400>  98

Ser Pro Pro Ser Pro Ala Pro Pro Val Ala Gly
1               5                   10


<210>  99
<211>  12
<212>  PRT
<213>  Artificial Sequence

<220>
<223>  Parts of the hinge and CH2 region

<400>  99

Cys Pro Pro Cys Pro Ala Pro Glu Leu Leu Gly Gly
1               5                   10


<210>  100
<211>  451
<212>  PRT
<213>  Artificial Sequence

<220>

<223>  FLT3 x CD3 Fabv

<400>  100

Gln Val Gln Leu Gln Gln Pro Gly Ala Glu Leu Val Lys Pro Gly Ala
1               5                   10                  15

Ser Leu Lys Leu Ser Cys Lys Ser Ser Gly Tyr Thr Phe Thr Ser Tyr
            20                  25                  30

Trp Met His Trp Val Arg Gln Arg Pro Gly His Gly Leu Glu Trp Ile
        35                  40                  45

Gly Glu Ile Asp Pro Ser Asp Ser Tyr Lys Asp Tyr Asn Gln Lys Phe
    50                  55                  60

Lys Asp Lys Ala Thr Leu Thr Val Asp Arg Ser Ser Asn Thr Ala Tyr
65                  70                  75                  80

Met His Leu Ser Ser Leu Thr Ser Asp Asp Ser Ala Val Tyr Tyr Cys
                85                  90                  95

Ala Arg Ala Ile Thr Thr Thr Pro Phe Asp Phe Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Ser Pro Pro Ser Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Gly Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Gln Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gln Leu Pro Ser Pro Ile Glu Lys Thr Ile
                325                 330                 335

Ser Lys Ala Lys Gly Gln Pro Ser Gly Asp Ile Val Leu Thr Gln Ser
            340                 345                 350

Pro Ala Ile Met Ser Ala Ser Pro Gly Glu Lys Val Thr Met Thr Cys
        355                 360                 365

Ser Ala Ser Ser Ser Val Ser Tyr Met Asn Trp Tyr Gln Gln Lys Ser
    370                 375                 380

Gly Thr Ser Pro Lys Arg Trp Ile Tyr Asp Thr Ser Lys Leu Ala Ser
385                 390                 395                 400

Gly Val Pro Ala His Phe Arg Gly Ser Gly Ser Gly Thr Ser Tyr Ser
                405                 410                 415

Leu Thr Ile Ser Gly Met Glu Ala Glu Asp Ala Ala Thr Tyr Tyr Cys
            420                 425                 430

Gln Gln Trp Ser Ser Asn Pro Phe Thr Phe Gly Ser Gly Thr Lys Leu
        435                 440                 445

Glu Ile Asn
    450

<210>  101
<211>  464
<212>  PRT
<213>  Artificial Sequence

<220>

<223>  FLT3 x CD3 Fabv

<400>  101

Gln Val Gln Leu Gln Gln Pro Gly Ala Glu Leu Val Lys Pro Gly Ala
1               5                   10                  15

Ser Leu Lys Leu Ser Cys Lys Ser Ser Gly Tyr Thr Phe Thr Ser Tyr
            20                  25                  30

Trp Met His Trp Val Arg Gln Arg Pro Gly His Gly Leu Glu Trp Ile
        35                  40                  45

Gly Glu Ile Asp Pro Ser Asp Ser Tyr Lys Asp Tyr Asn Gln Lys Phe
    50                  55                  60

Lys Asp Lys Ala Thr Leu Thr Val Asp Arg Ser Ser Asn Thr Ala Tyr
65                  70                  75                  80

Met His Leu Ser Ser Leu Thr Ser Asp Asp Ser Ala Val Tyr Tyr Cys
                85                  90                  95

Ala Arg Ala Ile Thr Thr Thr Pro Phe Asp Phe Trp Gly Gln Gly Thr
            100                 105                 110

Thr Leu Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Ser Pro Pro Ser Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Gly Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Gln Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gln Leu Pro Ser Pro Ile Glu Lys Thr Ile
                325                 330                 335

Ser Lys Ala Lys Gly Gln Pro Ser Gly Gln Val Gln Leu Gln Gln Ser
            340                 345                 350

Gly Ala Glu Leu Ala Arg Pro Gly Ala Ser Val Lys Met Ser Cys Lys
        355                 360                 365

Ala Ser Gly Tyr Thr Phe Thr Arg Tyr Thr Met His Trp Val Lys Gln
    370                 375                 380

Arg Pro Gly Gln Gly Leu Glu Trp Ile Gly Tyr Ile Asn Pro Ser Arg
385                 390                 395                 400

Gly Tyr Thr Asn Tyr Asn Gln Lys Phe Lys Asp Lys Ala Thr Leu Thr
                405                 410                 415

Thr Asp Lys Ser Ser Ser Thr Ala Tyr Met Gln Leu Ser Ser Leu Thr
            420                 425                 430

Ser Glu Asp Ser Ala Val Tyr Tyr Cys Ala Arg Tyr Tyr Asp Asp His
        435                 440                 445

Tyr Cys Leu Asp Tyr Trp Gly Gln Gly Thr Thr Leu Thr Val Ser Ser
    450                 455                 460

<210>  102
<211>  214
<212>  PRT
<213>  Artificial Sequence

<220>

<223>  Anti-FLT3 chimeric light chain (clone 4G8)

<400>  102

Asp Ile Val Leu Thr Gln Ser Pro Ala Thr Leu Ser Val Thr Pro Gly
1               5                   10                  15

Asp Ser Val Ser Leu Ser Cys Arg Ala Ser Gln Ser Ile Ser Asn Asn
            20                  25                  30

Leu His Trp Tyr Gln Gln Lys Ser His Glu Ser Pro Arg Leu Leu Ile
        35                  40                  45

Lys Tyr Ala Ser Gln Ser Ile Ser Gly Ile Pro Ser Arg Phe Ser Gly
    50                  55                  60

Ser Gly Ser Gly Thr Asp Phe Thr Leu Ser Ile Asn Ser Val Glu Thr
65                  70                  75                  80

Glu Asp Phe Gly Val Tyr Phe Cys Gln Gln Ser Asn Thr Trp Pro Tyr
                85                  90                  95

Thr Phe Gly Gly Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  103
<211>  220
<212>  PRT

<213>  Artificial Sequence

<220>
<223>  Anti-FLT3 chimeric light chain (clone BV10)

<400>  103

Asp Ile Val Met Thr Gln Ser Pro Ser Ser Leu Ser Val Ser Ala Gly
1               5                   10                  15

Glu Lys Val Thr Met Ser Cys Lys Ser Ser Gln Ser Leu Leu Asn Ser
            20                  25                  30

Gly Asn Gln Lys Asn Tyr Met Ala Trp Tyr Gln Gln Lys Pro Gly Gln
        35                  40                  45

Pro Pro Lys Leu Leu Ile Tyr Gly Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Gln Ala Glu Asp Leu Ala Val Tyr Tyr Cys Gln Asn
                85                  90                  95

Asp His Ser Tyr Pro Leu Thr Phe Gly Ala Gly Thr Lys Leu Glu Leu
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210>  104
<211>  218
<212>  PRT
<213>  Artificial Sequence

<220>
<223>  Anti-CSPG4 chimeric light chain (clone 9.2.27)

<400>  104

Asp Ile Glu Leu Thr Gln Ser Pro Ala Ser Leu Ala Val Ser Leu Gly
1               5                   10                  15

Gln Arg Ala Thr Ile Ser Cys Arg Ala Ser Glu Ser Val Asp Ser Tyr
            20                  25                  30

Gly Asn Ser Phe Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Ala
    50                  55                  60

Arg Phe Ser Gly Ser Gly Ser Arg Thr Asp Phe Thr Leu Thr Ile Asp
65                  70                  75                  80

Pro Val Glu Ala Asp Asp Ala Ala Thr Tyr Tyr Cys Gln Gln Asn Asn
                85                  90                  95

Glu Asp Pro Leu Thr Phe Gly Gly Gly Thr Lys Leu Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys

210                 215

<210>  105
<211>  219
<212>  PRT
<213>  Artificial Sequence

<220>
<223>  Anti-CD19 chimeric light (clone 4G7)

<400>  105

Asp Ile Val Met Thr Gln Ala Ala Pro Ser Ile Pro Val Thr Pro Gly
1               5                   10                  15

Glu Ser Val Ser Ile Ser Cys Arg Ser Ser Lys Ser Leu Leu Asn Ser
            20                  25                  30

Asn Gly Asn Thr Tyr Leu Tyr Trp Phe Leu Gln Arg Pro Gly Gln Ser
        35                  40                  45

Pro Gln Leu Leu Ile Tyr Arg Met Ser Asn Leu Ala Ser Gly Val Pro
    50                  55                  60

Asp Arg Phe Ser Gly Ser Gly Ser Gly Thr Ala Phe Thr Leu Arg Ile
65                  70                  75                  80

Ser Arg Val Glu Ala Glu Asp Val Gly Val Tyr Tyr Cys Met Gln His
                85                  90                  95

Leu Glu Tyr Pro Phe Thr Phe Gly Ala Gly Thr Lys Leu Glu Leu Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  106
<211>  214
<212>  PRT
<213>  Artificial Sequence

<220>
<223>  Anti-EGFR light chain (clone C225)

<400>  106

Asp Ile Leu Leu Thr Gln Ser Pro Val Ile Leu Ser Val Ser Pro Gly
1               5                   10                  15

Glu Arg Val Ser Phe Ser Cys Arg Ala Ser Gln Ser Ile Gly Thr Asn
            20                  25                  30

Ile His Trp Tyr Gln Gln Arg Thr Asn Gly Ser Pro Arg Leu Leu Ile
        35                  40                  45

Lys Tyr Ala Ser Glu Ser Ile Ser Gly Ile Pro Ser Arg Phe Ser Gly
    50                  55                  60

Ser Gly Ser Gly Thr Asp Phe Thr Leu Ser Ile Asn Ser Val Glu Ser
65                  70                  75                  80

Glu Asp Ile Ala Asp Tyr Tyr Cys Gln Gln Asn Asn Asn Trp Pro Thr
                85                  90                  95

Thr Phe Gly Ala Gly Thr Lys Leu Glu Leu Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  107
<211>  56
<212>  DNA
<213>  Artificial Sequence

<220>
<223>  L1 and further

<400>  107
atgggatgga gctggatctt tctctttctc ctgtcaggaa ctgcaggtaa ggggct          56

<210>  108
<211>  15
<212>  PRT
<213>  Artificial Sequence

<220>
<223>  L1

<400>  108

Met Gly Trp Ser Trp Ile Phe Leu Phe Leu Leu Ser Gly Thr Ala
1               5                   10                  15

<210>  109
<211>  43
<212>  DNA
<213>  Artificial Sequence

<220>
<223>  between L1 and L2-VDJ

<400>  109
tgacgtccac tctgtctttc tcttcacagg tgtcctctcc cag                        43

<210>  110
<211>  4
<212>  PRT
<213>  Artificial Sequence

<220>
<223>  L2-VDJ

<400>  110

Val Leu Ser Gln
1

<210>  111
<211>  36

<212>  DNA
<213>  Artificial Sequence

<220>
<223>  VDJ ClaI

<400>  111
tcaggtgagt cctaccttct cccattctaa atcgat                                36


<210>  112
<211>  35
<212>  DNA
<213>  Artificial Sequence

<220>
<223>  MluI-CH1

<400>  112
atcacatacg cgtttttctt gtagcctcca ccaag                                 35


<210>  113
<211>  28
<212>  DNA
<213>  Artificial Sequence

<220>
<223>  CH1-BspEI

<400>  113
acccacaggg cagccctccg gagatatc                                         28


<210>  114
<211>  6
<212>  PRT
<213>  Artificial Sequence

<220>
<223>  CH1-BspEI-VL

<400>  114

Gln Pro Ser Gly Asp Ile
1               5


<210>  115
<211>  20
<212>  DNA
<213>  Artificial Sequence

<220>
<223>  VL-SpeI

<400>  115
atcaaatgat aactagtccc                                                  20


<210>  116
<211>  35
<212>  DNA

<213>  Artificial Sequence

<220>
<223>  MluI-CH1

<400>  116
atcacatacg cgtttttctt gtagcctcca ccaag                                35


<210>  117
<211>  28
<212>  DNA
<213>  Artificial Sequence

<220>
<223>  CH1-BspEI-VH

<400>  117
acccacaggg cagccctccg gagaggtt                                        28


<210>  118
<211>  6
<212>  PRT
<213>  Artificial Sequence

<220>
<223>  CH1-BspEI-VH

<400>  118

Gln Pro Ser Gly Glu Val
1               5


<210>  119
<211>  20
<212>  DNA
<213>  Artificial Sequence

<220>
<223>  VH-SpeI

<400>  119
tcctcgtgat aactagtccc                                                 20


<210>  120
<211>  49
<212>  DNA
<213>  Artificial Sequence

<220>
<223>  L1

<400>  120
atggttttca cacctcagat acttggactt atgctttttt ggatttcag                 49


<210>  121
<211>  16
<212>  PRT
<213>  Artificial Sequence

<220>
<223>  L1

<400>  121

Met Val Phe Thr Pro Gln Ile Leu Gly Leu Met Leu Phe Trp Ile Ser
1               5                   10                  15


<210>  122
<211>  12
<212>  DNA
<213>  Artificial Sequence

<220>
<223>  between L1 and L2

<400>  122
gtatgactgc tt                                                          12


<210>  123
<211>  20
<212>  DNA
<213>  Artificial Sequence

<220>
<223>  between L1 and L2

<400>  123
tttccaggtg tccgacgaga                                                  20


<210>  124
<211>  4
<212>  PRT
<213>  Artificial Sequence

<220>
<223>  L2

<400>  124

Ala Arg Gly Asp
1


<210>  125
<211>  18
<212>  DNA
<213>  Artificial Sequence

<220>
<223>  VJ-SpeI

<400>  125
aaacgtaagt actagttt                                                    18


<210>  126
<211>  41
<212>  DNA

<213>  Artificial Sequence

<220>
<223>  Pm1I-CL

<400>  126
cataccatcc tcacgtgctt ccttcctcag ggactgtggc t                41


<210>  127
<211>  33
<212>  DNA
<213>  Artificial Sequence

<220>
<223>  CL-BsmBI

<400>  127
ggagagtgtt agagacaaag gtcctgagac gcc                33